# EUROPEAN PATENT SPECIFICATION

(11) **EP 0 876 388 B1**
(45) Date of publication and mention of the grant of the patent: **18.02.2004**
(21) Application number: 96936579.0
(22) Date of filing: 17.10.1996
(51) Int. Cl.: C07H 17/08, A61K 31/70

(54) **TRICYCLIC ERYTHROMYCIN DERIVATIVES**
TRICYCLISCHE ERYTHROMYCINDERIVATE
DERIVES TRICYCLIQUES D'ERYTHROMYCINE

(30) Priority: 08.11.1995 US 555246
(43) Date of publication of application: 11.11.1998
(73) Proprietor: Abbott Laboratories, Abbott Park, Illinois 60064-3500 (US)
(72) Inventor: OR, Yat, Sun, Libertyville, IL 60048 (US); PHAN, Ly, Tam, Park City, IL 60085 (US); CHU, Daniel, T., Santa Clara, CA 95051 (US); SPINA, Kenneth, P., Chicago, IL 60634 (US); HALLAS, Robert, Kenosha, WI 53142 (US); ELLIOTT, Richard, L., Grayslake, IL 60030 (US)
(74) Representative: Modiano, Guido, Dr.-Ing.
(86) International application number: PCT/US1996/016585
(87) International publication number: WO 1997/017356

(56) References cited:
- EP-A- 0 559 896
- EP-A- 0 619 319
- EP-A- 0 638 585

## Description

### Technical Field

The present invention relates to novel semisynthetic macrolides having antibacterial activity and useful in the treatment and prevention of bacterial infections. More particularly, the invention relates to tricyclic erythromycin derivatives, compositions containing such compounds and methods for using the same, as well as processes for making such compounds.

### Background of the Invention

Erythromycins A through D, represented by formula (E),

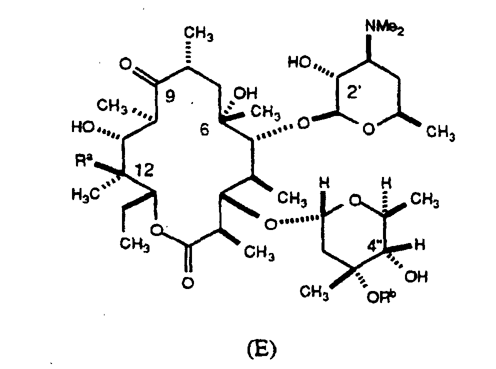

| Erythromycin | R^{a} | R^{b} |
|---|---|---|
| A | -OH | -CH₃ |
| B | -H | -CH₃ |
| C | -OH | -H |
| D | -H | -H |

are well-known and potent antibacterial agents, used widely to treat and prevent bacterial infection. As with other antibacterials, however, bacterial strains having resistance or insufficient susceptibility to erythromycin have been identified. Also, erythromycin A has only weak activity against Gram-negative bacteria. Therefore, there is a continuing need to identify new erythromycin derivative compounds which possess improved antibacterial activity, which have less potential for developing resistance, which possess the desired Gram-negative activity, or which possess unexpected selectivity against target microorganisms. Consequently, numerous investigators have prepared chemical derivatives of erythromycin in an attempt to obtain analogs having modified or improved profiles of antibiotic activity.

Kashimura *et al*. have disclosed 6-O-methylerythromycin derivatives having a tricyclic basic nuclear structure in European Application 559896, published November 11, 1991. Also, Asaka *et al*. have disclosed 5-O-desoaminylerythronolide derivatives containing a tricyclic carbamate structure in PCT Application WO 93/21200, published April 22, 1992.

### Summary of the Invention

The present invention provides a novel class of antibacterial tricyclic erythromycin compounds which possess antibacterial activity.

In one aspect of the present invention are disclosed novel tricyclic erythromycin compounds selected from the group having the formulas: 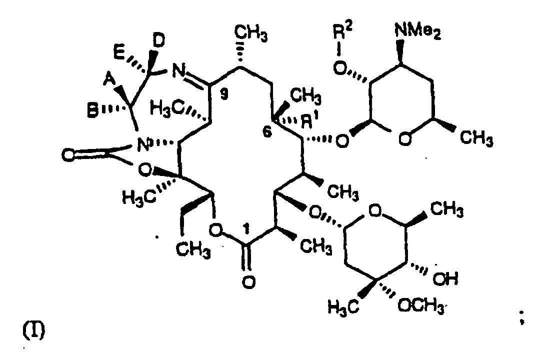 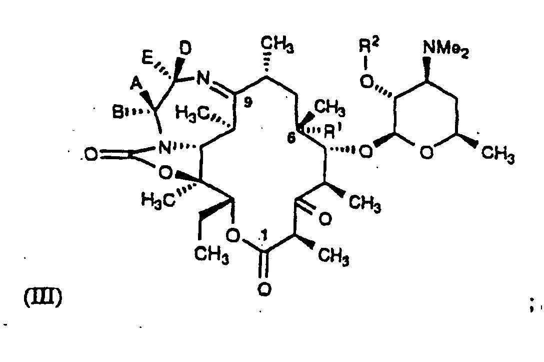 and 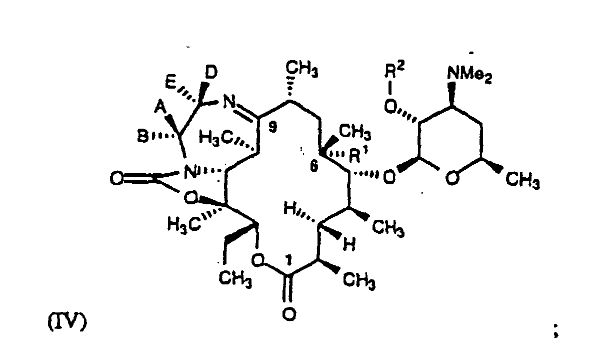 as well as the pharmaceutically acceptable salts and esters thereof wherein:
A, B, D, and E are independently selected from the group consisting of:
   (a) hydrogen;
   (b) C₁-C₆-alkyl, unsubstituted or substituted with one substituent selected from the group consisting of:
      (i) phenyl;
      (ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
      (iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
      (iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
      (v) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
      (vi) hydroxy;
      (vii) C₁-C₆ alkoxy;
      (viii) halogen consisting of Br, Cl, F or I; and
      (ix) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, - N(phenyl-C₁-C₆-alkyl-)-,
         -N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
   (c) C₃-C₇-cycloalkyl;
   (d) phenyl;
   (e) substituted-phenyl as defined above;
   (f) heteroaryl as defined above;
   (g) substituted-heteroaryl as defined above;
   (h) heterocycloalkyl as defined above;
      and
      (i) a group selected from option (b) above in which the substituent is
         -M-R⁵, wherein M is selected from the group consisting of:
         (aa) -C(O)-NH-;
         (bb) -NH-C(O)-;
         (cc) -NH-
         (dd) -N(CH₃)-
         (ee) -O-;
         (ff) -S(O)ₙ-, wherein n is 0, 1 or 2; and
         (gg) -C(=NH)-NH-;
         and R⁵ is selected from the group consisting of:
         (aaa) C₁-C₆-alkyl, optionally substituted with a substituent selected from the group consisting of:
            (i) phenyl;
            (ii) substituted-phenyl as defined above;
            (iii) heteroaryl as defined above;
            (iv) substituted-heteroaryl as defined above;
         (bbb) phenyl;
         (ccc) substituted-phenyl as defined above;
         (ddd) heteroaryl as defined above;
         (eee) substituted-heteroaryl as defined above; and
         (fff) heterocycloalkyl as defined above;
or
any one pair of substituents, consisting of AB, AD, AE, BD, BE, or DE, is taken together with the atom or atoms to which they are attached to form a 3- to 7-membered ring optionally containing a hetero function consisting of

-O-,

- NH-,

-N(C₁-C₆-alkyl-)-,

-N(phenyl-C₁-C₆-alkyl-)-,

- N(substituted-phenyl-C₁-C₆-alkyl-)-

wherein substituted-phenyl is as defined above,

-N(heteroaryl-C₁-C₆-alkyl-)-

wherein heteroaryl is as defined above,

-N(substituted-heteroaryl-C₁-C₆-alkyl-)-

wherein substituted-heteroaryl is as defined above,

- S-, or -S(O)ₙ-,

wherein n is 1 or 2;

- C(O)-NH-;

- C(O)-NR⁵-,

wherein R⁵ is as defined above;

- NH-C(O)-;

- NR⁵-C(O)-,

wherein R⁵ is as defined above; and

- C(=NH)-NH-;

R¹ is -O-C₁-C₃-alkyl; and
R² is hydrogen;
with the provisos that when the compound is of Formulas (I) or (III) then A, B, D, and E may not all be hydrogen, D and E may not be C₁-C₃-alkyl when A and B are hydrogen, nor may one of D and E be hydrogen and the other be C₁-C₃-alkyl when A and B are hydrogen;
or a compound of Formula (III) in which R¹ = OCH₃, R² is H, B = E = H, and A and D taken together is either-CH₂-N(Cbz)-CH₂- or -CH₂-N(benzoyl)-CH₂-;
or a compound of Formula (III) in which B=E=H, R¹ is methoxy, R² is H; and A and D together is CH₂-N(R⁷)-CH₂-, wherein R⁷ is selected from the group consisting of:
(i) phenyl;
(ii) substituted-phenyl as defined above;
(iii) heteroaryl as defined above; and
(iv) substituted-heteroaryl as defined above;
or a compound of Formula (III) in which B=E=H, R¹ is methoxy, R² is H; and A and D together is CH₂-N(-(CH₂)ᵣ-M-(CH₂)ₛ-R⁷)-CH₂-, wherein r is 0, 1 or 2; s = 0, 1 or 2, M is as defined above; and R⁷ is selected from the group consisting of:
(i) C₁-C₃-alkyl;
(ii) phenyl;
(iii) substituted-phenyl as defined above;
(iv) heteroaryl as defined above; and
(v) substituted-heteroaryl as defined above.

In another aspect of the present invention are disclosed pharmaceutical compositions comprising a therapeutically effective amount of a compound of the invention in combination with a pharmaceutically acceptable carrier wherein such compositions can be used in the treatment of bacterial infections.

Suitable carriers and methods of formulation are also disclosed. The compounds and compositions of the present invention have antibacterial activity.

In a further aspect of the present invention are provided processes for the preparation of tricyclic macrolide derivatives of Formulas (I), (III) and (IV) above.

### Detailed Description of the Invention

In one embodiment of the present invention are compounds selected from the group having formula (I) above, wherein A, B, D, E, and R¹-R⁵ are as described above.

In another embodiment of the present invention are compounds selected from the group having formula (III) above, wherein A, B, D, E, and R¹-R⁵ are as described above.

In yet another embodiment of the present invention are compounds selected from the group having formula (IV) above, wherein A, B, D, E, and R¹-R⁵ are as described above.

In one preferred embodiment of the present invention are compounds of formula (III) above wherein R¹ is methoxy, and A, B, D, E and R¹-R⁵ are as described above.

In another preferred embodiment of the present invention are compounds of formula (III) above wherein R¹ is methoxy, and any three of the A, B, D and E groups are hydrogen and the other group is selected from a singly substituted C₁-C₆-alkyl group comprised of -(CH₂)ₘR⁶ where m=1, 2, 3 or 4 and R⁶ is selected from the group consisting of
(a) phenyl,
(b) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(c) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
(d) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
(e) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
(f) hydroxy;
(g) C₁-C₆ alkoxy;
(h) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, - N(phenyl-C₁-C₆-alkyl-)-, - N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
(i) halogen consisting of Br, Cl, F or I;
(j) C₁-C₃-alkyl; or
(k) (CH₂)ᵣ-M-(CH₂)ₛ-R⁷ wherein r = 0, 1, or 2; s = 0, 1 or 2 and M is selected from the group consisting of:
   (aa) -C(O)-NH-;
   (bb) -NH-C(O)-;
   (cc) -NH -
   (dd) -N(CH₃)-
   (ee) -O-;
   (ff) -S(O)ₙ-, wherein n is 0, 1 or 2; and
   (gg) -C(=NH)-NH-;
   and R⁷ is selected from the group consisting of:
   (aaa) C₁-C₃-alkyl;
   (bbb) phenyl;
   (ccc) substituted-phenyl as defined above;
   (ddd) heteroaryl as defined above; and
   (eee) substituted-heteroaryl as defined above.

In yet another preferred embodiment of the present invention are compounds of formula (III) above wherein R¹ is methoxy, B=E=H, and A and D taken together is selected from the group consisting of:
(a) -CH₂-Z-CH₂-, wherein Z is selected from the group consisting of :
   (aa) -C(O)-NH-;
   (bb) -C(O)-NR⁵-, wherein R⁵ is selected from the group consisting of:
      (aaa) C₁-C₆ alkyl, unsubstituted or substituted with a substituent selected from the group consisting of:
         (i) phenyl;
         (ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
         (iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl; and
         (iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
      (bbb) phenyl;
      (ccc) substituted-phenyl as defined above;
      (ddd) heteroaryl as defined above;
      (eee) substituted-heteroaryl as defined above; and
      (fff) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
   (cc) -NH-C(O)-;
   (dd) -NR⁵-C(O)-, wherein R⁵ is as defined above
   (ee) -NH-;
   (ff) -N(CH₃)-;
   (gg) -O-;
   (hh) -S(O)ₙ-, wherein n is 0, 1 or 2; and
   (ii) -C(=NH)-NH-;
(b) -CH₂-N(-(CH₂)ₛ-R⁷)-CH₂-, wherein s = 0, 1 or 2, and R⁷ is selected from the group consisting of:
   (aa) C₁-C₃-alkyl;
   (bb) phenyl;
   (cc) substituted-phenyl as defined above;
   (dd) heteroaryl as defined above; and
   (ee) substituted-heteroaryl as defined above;
(c) -CH₂-N(-(CH₂)ᵣ-M-(CH₂)ₛ-R⁷)-CH₂-, wherein r is 0, 1 or 2; s = 0, 1 or 2, R⁷ is as defined above and M is selected from the group consisting of:
   (i) -C(O)-NH-;
   (ii) -NH-C(O)-;
   (iii) -NH-
   (iv) -N(CH₃) -
   (v) -O-;
   (vi) -S(O)ₙ-, wherein n is 0, 1 or 2; and
   (vii) -C(=NH)-NH-; and
(d) -CH₂-(CH₂)ᵣ-CH₂-, wherein r = 0, 1 or 2.

Representative of the compounds of the invention include:
Compound of Formula (IV); R¹=methoxy; R²=hydrogen; A=B=D=E=hydrogen;
Compound of Formula (III), A=B=E=H, D=benzyl, R¹=methoxy, R²=hydrogen;
Compound of Formula (III), A=B=D=H, E=benzyl, R¹=methoxy, R²=hydrogen;
Compound of Formula (III); A=benzyl, B=D=E=H, R1=methoxy;
Compound of Formula (III); B=benzyl, A=D=E=H, R¹=methoxy, R²=hydrogen;
Compound of Formula (III); A=E=phenyl, B=D=H, R¹=methoxy, R²=hydrogen;
Compound of Formula (III); A=methyl, B=D=E=H, R¹=methoxy, R²=hydrogen;
Compound of Formula (III); B=methyl, A=D=E=H, R¹=methoxy, R²=hydrogen;
Compound of Formula (III); A=D=methyl; B=E=H, R¹=methoxy, R²=hydrogen;
Compound of Formula (III); A=E=methyl; B=D=H, R¹=methoxy, R²=hydrogen;
Compound of Formula (III); B=D=H; A and E taken together is -CH₂CH₂CH₂-, R¹=methoxy, R²=hydrogen;
Compound of Formula (III); B=E=H; A and D taken together is -CH₂CH₂CH₂CH₂-, R¹=methoxy, R²=hydrogen ;
Compound of Formula (III): R¹=OCH3, R²=H; A=B=D=H; E=-CH₂NH₂;
Compound of Formula (III): R¹=OCH3, R²=H; A=B=E=H; D=-CH₂NH₂;
Compound of Formula (III): R¹=OCH3, R²=H; B=E=H; A and D taken together is - CH₂CH₂CH₂-;
Compound of Formula (III): R¹=OCH3, R²=H; B=E=H; A and D taken together is - CH₂OCH₂-;
Compound of Formula (III): R¹=OCH3, R²=H; B=E=H; A and D taken together is - CH₂-NH-CH₂-;
Compound of Formula (III): R¹=OCH3, R²=H; B=E=H; A and D taken together is - CH₂-N(Cbz)-CH₂-;
Compound of Formula (III): R¹=OCH3, R²=H; B=E=H; A and D taken together is - CH₂-N(benzyl)-CH₂-;
Compound of Formula (III): R¹=OCH3, R²=H; B=E=H; A and D taken together is - CH₂-N(benzoyl)-CH₂-;
Compound of Formula (III): R¹=OCH3, R²=H; B=E=H; A and D taken together is - CH₂-N(phenyl-CH₂-CH₂-)-CH₂-;
Compound of Formula (III): R¹=OCH3, R²=H; B=E=H; A and D taken together is - CH₂-N(4-Cl-phenyl-CH₂-)-CH₂-;
Compound of Formula (III): R¹=OCH3, R²=H; B=E=H; A and D taken together is - CH₂-N(4-pyridyl-CH₂-)-CH₂-;
Compound of Formula (III): R¹=OCH3, R²=H; B=E=H; A and D taken together is - CH₂-N(2-pyridyl-CH₂-)-CH₂-;
Compound of Formula (III): R¹=OCH3, R²=H; B=E=H; A and D taken together is -CH₂-N(3-pyridyl-CH₂-)-CH₂-;
Compound of Formula (III): R¹=OCH3, R²=H; B=E=H; A and D taken together is - CH₂=N(4-quinolyl-CH₂-)-CH₂-;
Compound of Formula (III): R¹=OCH3, R²=H; B=E=H; A=D=-CH₂-O-CH₂-phenyl;
Compound of Formula (III): R¹=OCH3, R²=H; B=E=H; A=D=-CH₂-OH;
Compound of Formula (III): R¹=OCH3, R²=H; B=E=H; A=D=-CH₂-O-phenyl;
Compound of Formula (III): R¹=OCH3, R²=H; A=B=H; D and E taken together is - CH₂-CH₂-CH₂-CH₂-;
Compound of Formula (III): R¹=OCH3, R²=H; A and B taken together is - CH₂-CH₂-CH₂-CH₂-; D=E=H;
Compound of Formula (III): R¹=OCH3, R²=H; A=B=H; D and E taken together is -CH₂-O-CH₂-;
Compound of Formula (III): R¹=OCH3, R²=H; A=D=E=H; B=-CH₂-CH₂-phenyl ;
Compound of Formula (III): R¹=OCH3, R²=H; A=D=E=H; B=-CH₂-CH₂-CH₂-phenyl ;
Compound of Formula (III): R¹=OCH3, R²=H; A=D=E=H; B=-CH₂-O-CH₂-phenyl ;
Compound of Formula (III): R¹=OCH3, R²=H; A=D=E=H; B=-CH₂-CH₂-(4-OCH₃-phenyl );
Compound of Formula (III): R¹=OCH3, R²=H; A=-CH₂-CH₂-phenyl; B=D=E=H ;
Compound of Formula (III): R¹=OCH3, R²=H; A=-CH₂-CH₂-CH₂-phenyl; B=D=E=H ;
Compound of Formula (III): R¹=OCH3, R²=H; A=-CH₂-O-CH₂-phenyl ; B=D=E=H ;
Compound of Formula (III): R¹=OCH3, R²=H; B=D=E=H; A=-CH₂-CH₂-(4-OCH₃-phenyl);
Compound of Formula (III): R¹=OCH3, R²=H; A=B=D=H; E=-CH₂-CH₂-Ph;
Compound of Formula (III): R¹=OCH3, R²=H; A=B=E=H; D=-CH₂-CH₂-Ph;
Compound of Formula (III): R¹=OCH3, R²=H; A=B=D=H; E=-CH₂-CH₂-CH₂-Ph;
Compound of Formula (III): R¹=OCH3, R²=H; A=B=E=H; D=-CH₂-CH₂-CH₂-Ph;
Compound of Formula (III): R¹=OCH3, R²=H; A=- CH₂-CH₂-OPh; B=D=E=H;
Compound of Formula (III) R1=OCH3, R2=H; A=-CH₂-CH₂-NH2; B=D=E=H;
Compound of Formula (III) R1=OCH3, R2=H; A=-CH₂-CH₂-OH; B=D=E=H;
Compound of Formula (III): R¹=OCH3, R²=H; A=- CH₂-CH₂-NH(4-Pyridyl-)-; B=D=E=H
Compound of Formula (III): R¹=OCH3, R²=H; A=B=D=H; E=-CH₂-OH;
Compound of Formula (III): R¹=OCH3, R²=H; A=B=E=H; D=-CH₂-OH;
Compound of Formula (III): R¹=OCH3, R²=H; A=B=E=H; D=-CH₂-NHBenzoyl;
Compound of Formula (III): R¹=OCH3, R²=H; A=B=E=H; D=-CH₂-NHBenzyl;
Compound of Formula (III): R¹=OCH3, R²=H; A=B=D=H; E=-CH₂-NHBenzoyl;
Compound of Formula (III): R¹=OCH3, R²=H; A=B=D=H; E=-CH₂-NHBenzyl;
Compound of Formula (III): R¹=OCH3, R²=H; B=D=H; A=E=-CH₂OCH₂(4-Cl-phenyl);
Compound of Formula (III): R¹=OCH3, R²=H; A=B=E=H; D=-CH₂-N(CH₃)-Benzyl;
Compound of Formula (III): R¹=OCH3, R²=H; A=B=D=H; E=-CH₂-N(CH₃)-Benzyl;
Compound of Formula (III): R¹=OCH3, R²=H; A=B=D=H; E=-CH₂-NH-phenyl; and
Compound of Formula (III): R¹=OCH3, R²=H; A=B=E=H; D=-CH₂-NH-phenyl.

A selected group of representative compounds includes:
Compound of Formula (IV); R¹=methoxy; R²=hydrogen; A=B=D=E=hydrogen;
Compound of Formula (III), A=B=E=H, D=benzyl, R¹=methoxy, R²=hydrogen;
Compound of Formula (III), A=B=D=H, E=benzyl, R¹=methoxy, R²=hydrogen;
Compound of Formula (III); A=benzyl, B=D=E=H, R¹=methoxy, R²=hydrogen;
Compound of Formula (III); B=benzyl, A=D=E=H, R¹=methoxy, R²=hydrogen;
Compound of Formula (III); A=E=phenyl, B=D=H, R¹=methoxy, R²=hydrogen;
Compound of Formula (III); A=methyl, B=D=E=H, R¹=methoxy, R²=hydrogen;
Compound of Formula (III); B=methyl, A=D=E=H, R¹=methoxy, R²=hydrogen;
Compound of Formula (III); A=D=methyl; B=E=H, R¹=methoxy, R²=hydrogen;
Compound of Formula (III); A=E=methyl; B=D=H; R¹=methoxy, R²=hydrogen ;
Compound of Formula (III); B=D=H; A and E taken together is -CH₂CH₂CH₂-, R¹=methoxy, R²=hydrogen; and
Compound of Formula (III); B=E=H; A and D taken together is -CH₂CH₂CH₂CH₂-, R¹=methoxy, R²=hydrogen.

One object of the present invention is to provide a process for the preparation of tricyclic macrolide derivatives having the Formulas: 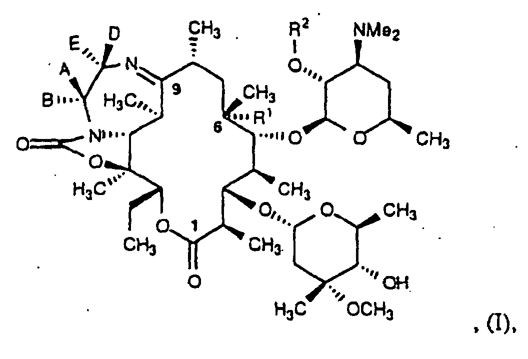 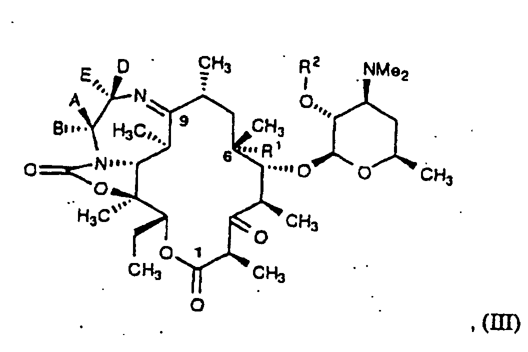 or 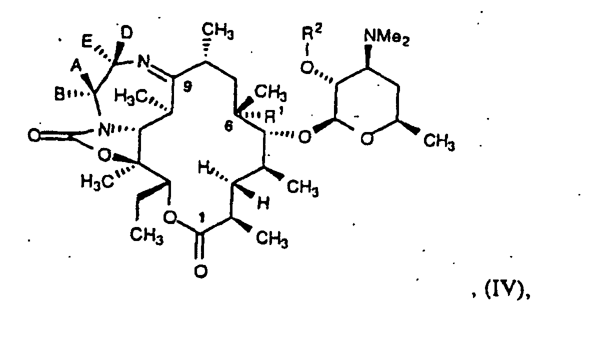 wherein
A, B, D and E are independently selected from the group consisting of:
   (a) hydrogen;
   (b) C₁-C₆-alkyl, unsubstituted or substituted with one substituent selected from the group consisting of:
      (i) phenyl;
      (ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
      (iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
      (iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
      (v) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
      (vi) hydroxy;
      (vii) C₁-C₆ alkoxy;
      (viii) halogen consisting of Br, Cl, F or I; and
      (ix) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(phenyl-C₁-C₆-alkyl-)-, - N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
   (c) C₃-C₇-cycloalkyl;
   (d) phenyl;
   (e) substituted-phenyl as defined above;
   (f) heteroaryl as defined above;
   (g) substituted-heteroaryl as defined above;
   (h) heterocycloalkyl as defined above;
      and
   (i) a group selected from option (b) above in which the substituent is -M-R⁵, wherein M is selected from the group consisting of:
      (aa) -C(O)-NH-;
      (bb) -NH-C(O)-;
      (cc) -NH-
      (dd) -N(CH₃) -
      (ee) -O-;
      (ff) -S(O)ₙ-, wherein n is 0, 1 or 2; and
      (gg) -C(=NH)-NH-;
      and R⁵ is selected from the group consisting of:
      (aaa) C₁-C₆-alkyl, optionally substituted with a substituent selected from the group consisting of:
         (i) phenyl;
         (ii) substituted-phenyl as defined above;
         (iii) heteroaryl as defined above;
         (iv) substituted-heteroaryl as defined above;
      (bbb) phenyl;
      (ccc) substituted-phenyl as defined above;
      (ddd) heteroaryl as defined above;
      (eee) substituted-heteroaryl as defined above; and
      (fff) heterocycloalkyl as defined above;
or
any one pair of substituents, consisting of AB, AD, AE, BD, BE, or DE, is taken together with the atom or atoms to which they are attached to form a 3- to 7-membered ring optionally containing a hetero function consisting of

-O-,

-NH-,

-N(C₁-C₆-alkyl-)-,

- N(phenyl-C₁-C₆-alkyl-)-,

-N(substituted-phenyl-C₁-C₆-alkyl-)-

wherein substituted-phenyl is as defined above,

-N(heteroaryl-C₁-C₆-alkyl-)-

wherein heteroaryl is as defined above,

- N(substituted-heteroaryl-C₁-C₆-alkyl-)-

wherein substituted-heteroaryl is as defined above,

- S-, or -S(O)ₙ-,

wherein n is 1 or 2;

-C(O)-NH-;

- C(O)-NR⁵-,

wherein R⁵ is as defined above;

- NH-C(O)-;

- NR⁵-C(O)-,

wherein R⁵ is as defined above; and

- C(=NH)-NH-;

R¹ is -O-C₁-C₃-alkyl; and
R² is hydrogen;
with the provisos that when the compound is of Formulas (I) or (III) then A, B, D, and E may not all be hydrogen, D and E may not be C₁-C₃-alkyl when A and B are hydrogen, nor may one of D and E be hydrogen and the other be C₁-C₃-alkyl when A and B are hydrogen;
or a compound of Formula (III) in which R¹ = OCH₃, R² is H, B = E = H, and A and D taken together is either -CH₂-N(Cbz)-CH₂- or -CH₂-N(benzoyl)-CH₂-;
or a compound of Formula (III) in which B=E=H, R¹ is methoxy, R² is H; and A and D together is CH₂-N(R⁷)-CH₂-, wherein R⁷ is selected from the group consisting of:
(i) phenyl;
(ii) substituted-phenyl as defined above;
(iii) heteroaryl as defined above; and
(iv) substituted-heteroaryl as defined above;
   or a compound of Formula (III) in which B=E=H, R¹ is methoxy, R² is H; and A and D together is CH₂-N(-(CH₂)ᵣ-M-(CH₂)ₛ-R⁷)-CH₂-, wherein r is 0, 1 or 2; s = 0, 1 or 2, M is as defined above; and R⁷ is selected from the group consisting of:

(i) C₁-C₃-alkyl;
(ii) phenyl;
(iii) substituted-phenyl as defined above;
(iv) heteroaryl as defined above; and
(v) substituted-heteroaryl as defined above,
the method comprising:
(a) treating a compound having the formula 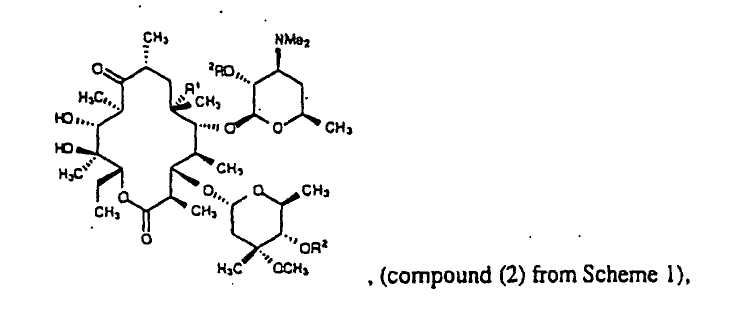 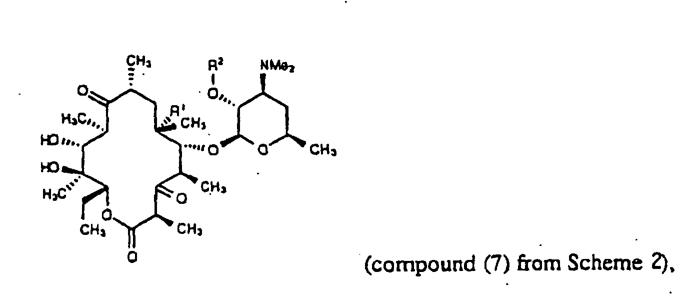 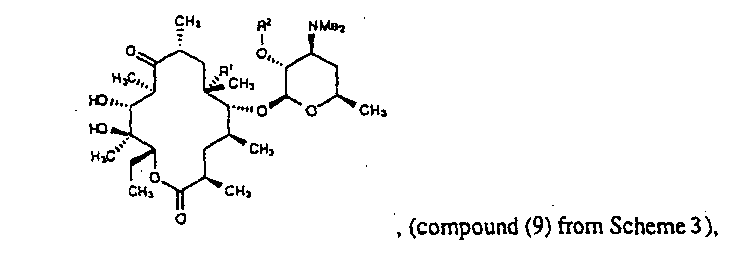 respectively,
   wherein R¹ is as described above and R² is a hydroxy-protecting group, with a base, such as sodium hydride, lithium hydride, potassium carbonate, for example, and followed by reaction with carbonyldiimidazole, in an aprotic solvent, as defined below, which does not adversely affect the reaction, preferably dichloromethane, chloroform, DMF, tetrahydrofuran (THF), N-methyl pyrrolidinone or a mixture thereof, with cooling or heating, depending on the conditions used, at a reaction temperature from -20°C to 70°C, preferably from 0°C to reaction temperature, for a period from 0.5 hours to 10 days, preferably 1-8 hours or up to 1-5 days, to prepare first intermediate compounds having the formulas: 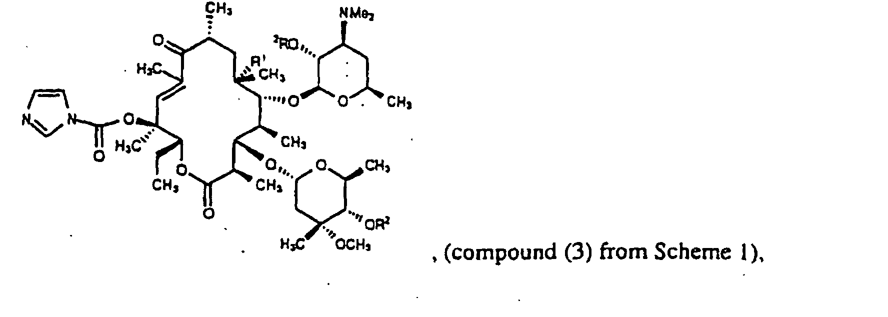 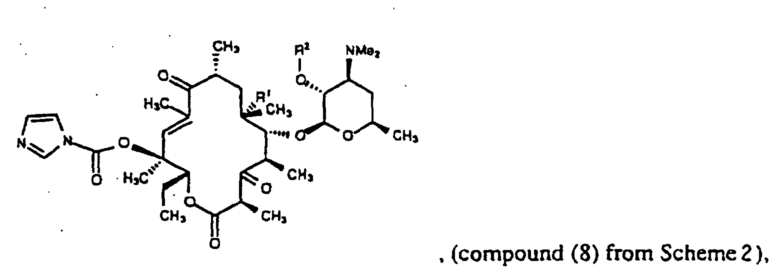 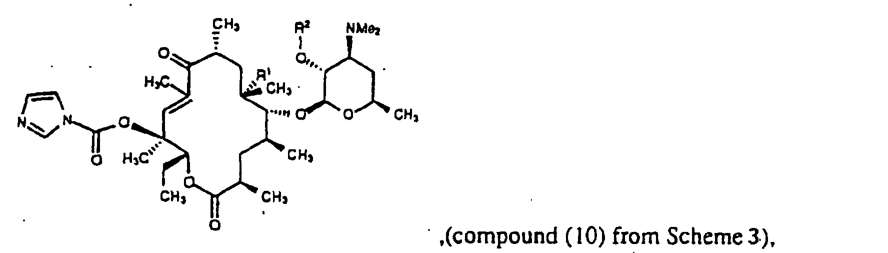 respectively: wherein R¹ and R² are as described above;
(b) reacting said first intermediate compounds (3), (8), or (10) with a compound having the formula: 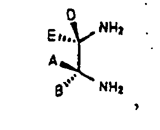 wherein A, B, D, and E are as described above, preferably in a solvent such as aqueous acetonitrile, DMF or aqueous DMF, to give the bicyclic second intermediate compounds having the formulas: 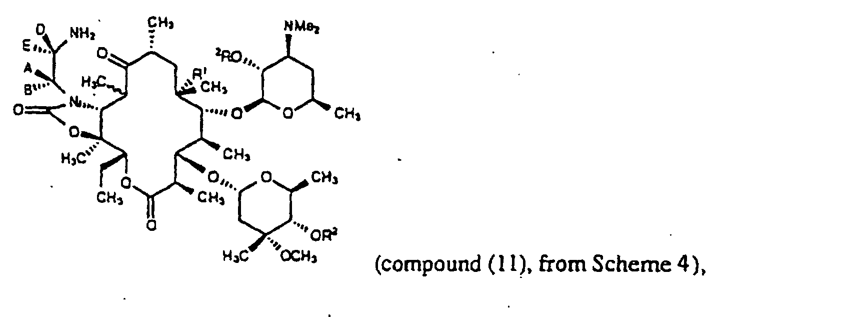 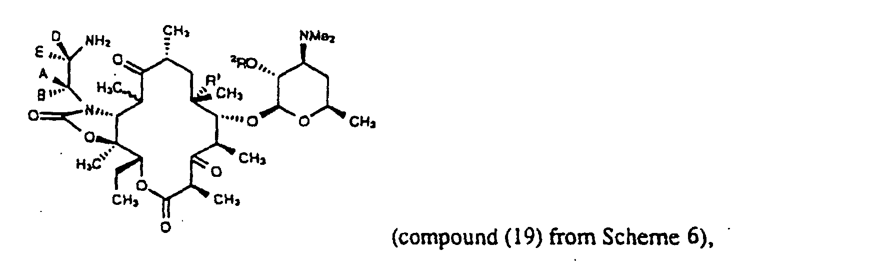 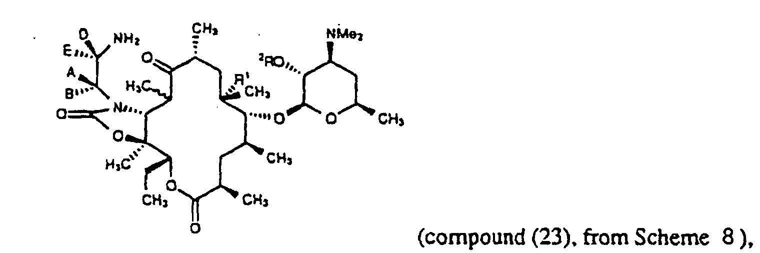 wherein R¹ and R² are as described above;
(c) deprotecting said second intermediate compounds (11), (19) or (23) by treatment with methanol or ethanol when OR² is an ester or with fluoride in THF or acetonitrile when R² is a trialkylsilyl group, for from 1 to 24 hours, to give the third intermediate compounds 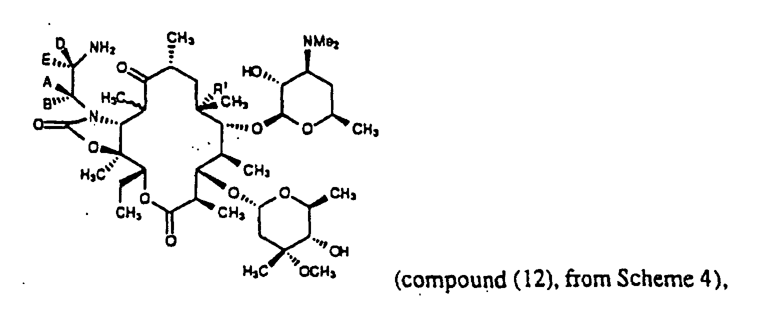 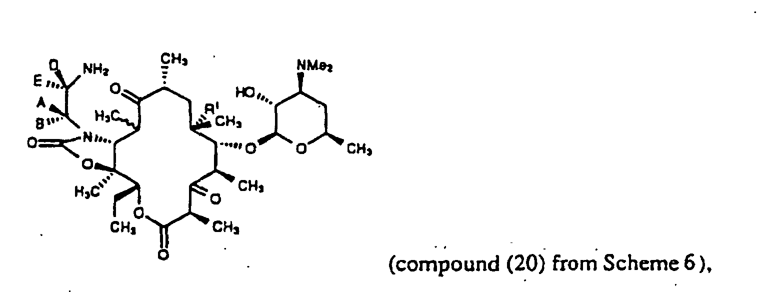 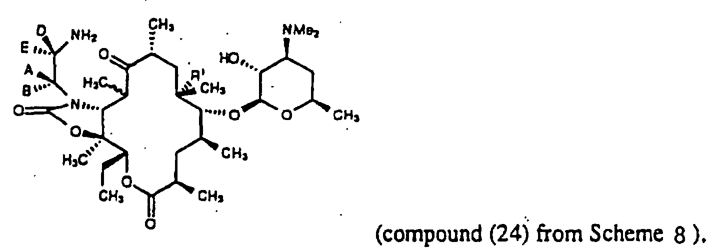
(d) cyclizing said third intermediate compounds (12), (20) or (24) by treatment with dilute acid, such as acetic acid or HCl, for example, in an organic solvent, preferably ethanol or propanol, for a period of from 4 hours to 10 days to give the desired compounds (I), (III) or (IV) above.

Another object of the present invention is to provide an alternate process for the preparation of tricyclic macrolide derivatives having the Formulas: 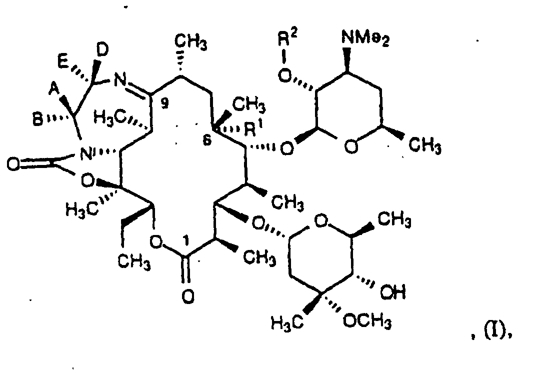 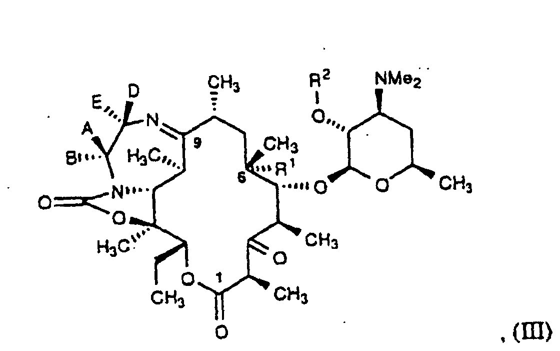 or 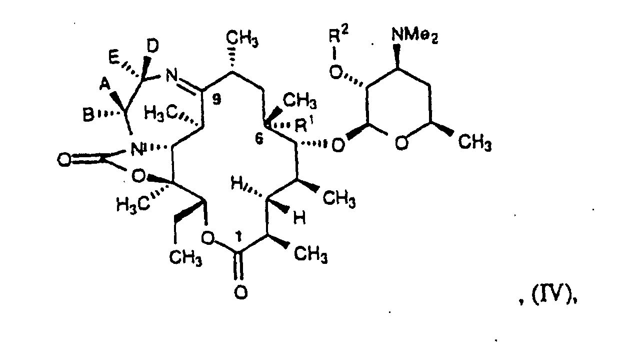 wherein
A, B, D and E are independently selected from the group consisting of:
   (a) hydrogen;
   (b) C₁-C₆-alkyl, unsubstituted or substituted with one substituent selected from the group consisting of:
      (i) phenyl;
      (ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
      (iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
      (iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
      (v) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
      (vi) hydroxy;
      (vii) C₁-C₆ alkoxy;
      (viii) halogen consisting of Br, Cl, F or I; and
      (ix) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(phenyl-C₁-C₆-alkyl-)-, - N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
   (c) C₃-C₇-cycloalkyl;
   (d) phenyl;
   (e) substituted-phenyl as defined above;
   (f) heteroaryl as defined above;
   (g) substituted-heteroaryl as defined above;
   (h) heterocycloalkyl as defined above;
      and
   (i) a group selected from option (b) above in which the substituent is -M-R⁵, wherein M is selected from the group consisting of:
      (aa) -C(O)-NH-;
      (bb) -NH-C(O)-;
      (cc) -NH -
      (dd) -N(CH₃)-
      (ee) -O-;
      (ff) -S(O)ₙ-, wherein n is 0, 1 or 2; and
      (gg) -C(=NH)-NH-;
      and R⁵ is selected from the group consisting of:
      (aaa) C₁-C₆-alkyl, optionally substituted with a substituent selected from the group consisting of:
         (i) phenyl;
         (ii) substituted-phenyl as defined above;
         (iii) heteroaryl as defined above;
         (iv) substituted-heteroaryl as defined above;
      (bbb) phenyl;
      (ccc) substituted-phenyl as defined above;
      (ddd) heteroaryl as defined above;
      (eee) substituted-heteroaryl as defined above; and
      (fff) heterocycloalkyl as defined above;
or
any one pair of substituents, consisting of AB, AD, AE, BD, BE, or DE, is taken together with the atom or atoms to which they are attached to form a 3- to 7-membered ring optionally containing a hetero function consisting of

-O-,

- NH-,

-N(C₁-C₆-alkyl-)-,

-N(phenyl-C₁-C₆-alkyl-)-,

-N(substituted-phenyl-C₁-C₆-alkyl-)- wherein substituted-phenyl is as defined above,

-N(heteroaryl-C₁-C₆-alkyl-)-

wherein heteroaryl is as defined above,

- N(substituted-heteroaryl-C₁-C₆-alkyl-)-

wherein substituted-heteroaryl is as defined above,

- S-, or -S(O)ₙ-,

wherein n is 1 or 2;

- C(O)-NH-;

- C(O)-NR⁵-,

wherein R⁵ is as defined above;

- NH-C(O)-;

-NR⁵-C(O)-,

wherein R⁵ is as defined above; and

- C(=NH)-NH-;

R¹ is -O-C₁-C₃-alkyl; and
R² is hydrogen;
with the provisos that when the compound is of Formulas (I) or (III) then A, B, D, and E may not all be hydrogen, D and E may not be C₁-C₃-alkyl when A and B are hydrogen, nor may one of D and E be hydrogen and the other be C₁-C₃-alkyl when A and B are hydrogen;
or a compound of Formula (III) in which R¹ = OCH₃, R² is H, B = E = H, and A and D taken together is either -CH₂-N(Cbz)-CH₂- or -CH₂-N(benzoyl)-CH₂-;
or a compound of Formula (III) in which B=E=H, R¹ is methoxy, R² is H; and A and D together is CH₂-N(R⁷)-CH₂-, wherein R⁷ is selected from the group consisting of:
(i) phenyl;
(ii) substituted-phenyl as defined above;
(iii) heteroaryl as defined above; and
(iv) substituted-heteroaryl as defined above;
or a compound of Formula (III) in which B=E=H, R¹ is methoxy, R² is H; and A and D together is CH₂-N(-(CH₂)ᵣ-M-(CH₂)ₛ-R⁷)-CH₂-, wherein r is 0, 1 or 2; s = 0, 1 or 2, M is as defined above; and R⁷ is selected from the group consisting of:
(i) C₁-C₃-alkyl;
(ii) phenyl;
(iii) substituted-phenyl as defined above;
(iv) heteroaryl as defined above; and
(v) substituted-heteroaryl as defined above,
the method comprising:
(a) treating a compound having the formula: 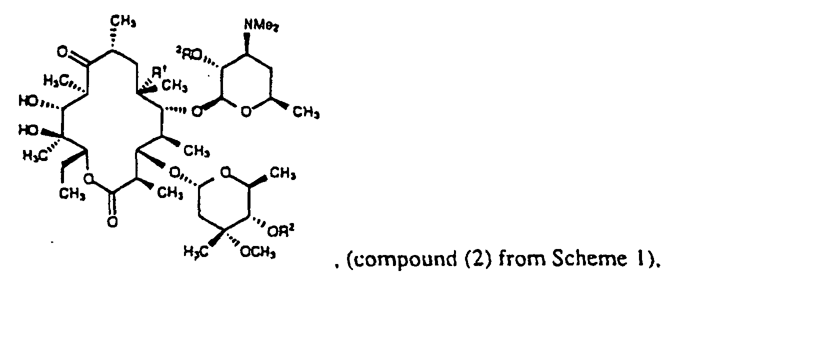 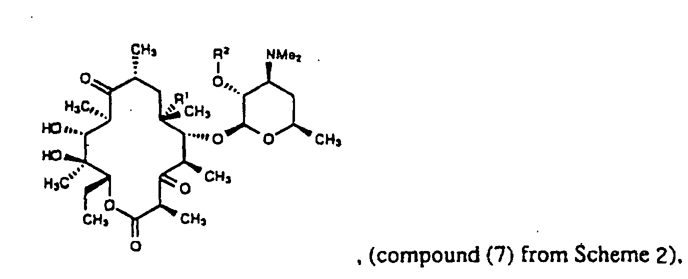 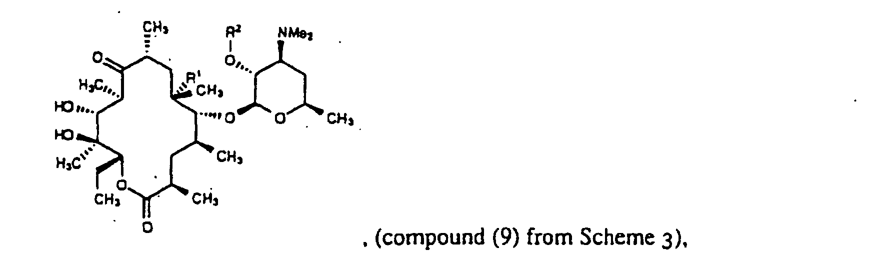 respectively,
   wherein R¹ is as described above and R² is a hydroxy-protecting group, with a base, such as sodium hydride, lithium hydride, potassium carbonate, for example, and followed by reaction with carbonyldiimidazole, in an aprotic solvent, as defined above. which does not adversely affect the reaction, preferably dichloromethane, chloroform, DMF, tetrahydrofuran (THF), N-methyl pyrrolidinone or a mixture thereof, with cooling or heating, depending on the conditions used, at a reaction temperature from -20°C to 70°C, preferably from 0°C to reaction temperature, for a period from 0.5 to 24 hours, preferably 1-8 hours, to prepare first intermediate compounds having the formulas: 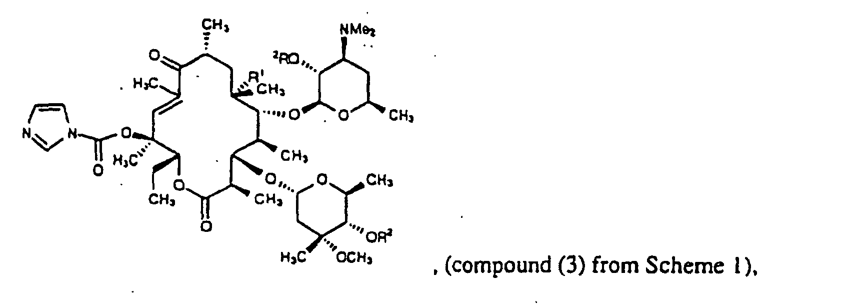 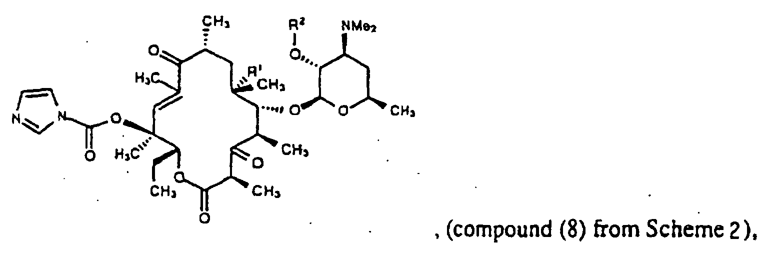 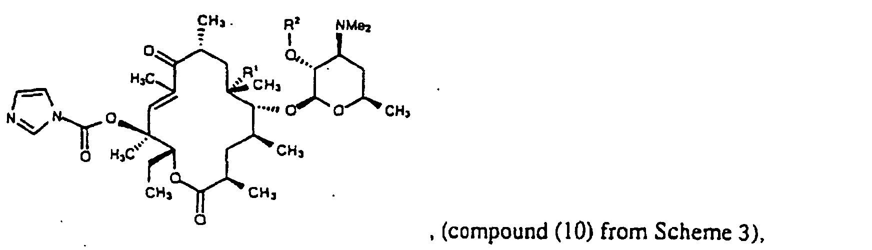 respectively; wherein R¹ and R² are as described above;
(b) reacting said first intermediate compounds (3), (8), or (10) with a compound having the formula: 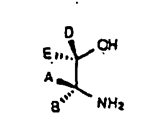 wherein A, B, D; and E are as described above, preferably in a solvent such as aqueous acetonitrile, DMF or aqueous DMF, to give the bicyclic second intermediate compounds: 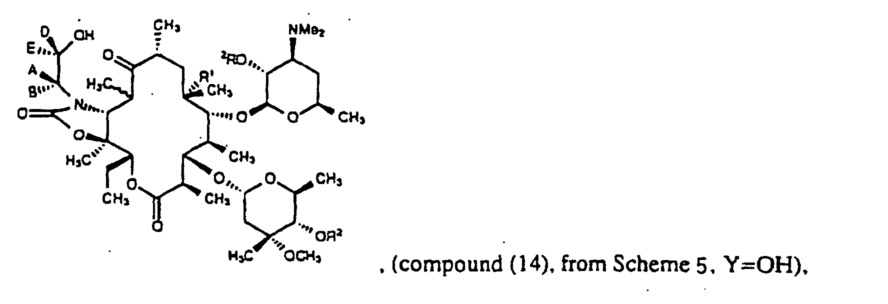 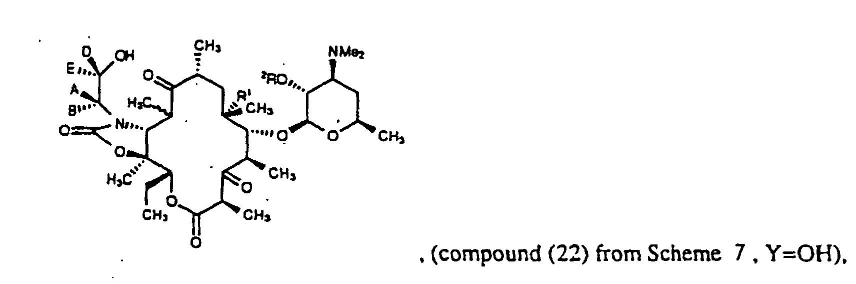 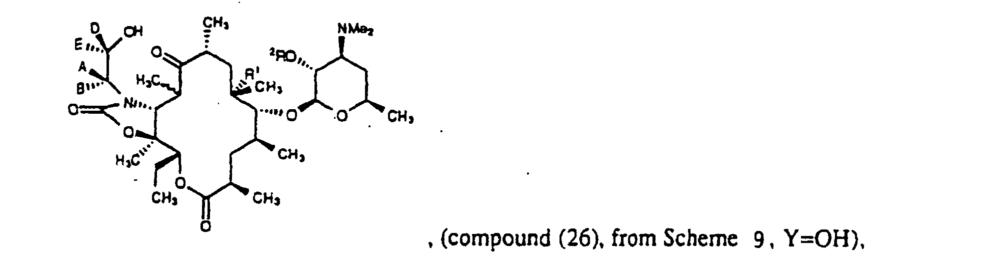 respectively;
(c) reacting the hydroxy group of the said bicyclic second intermediate compounds (14), (22) and (26) by treatment with triphenylphosphine and diphenylphosphoryl azide-DEAD in tetrahydrofuran, under Mitsunobu reaction conditions, to prepare the third intermediate compounds: 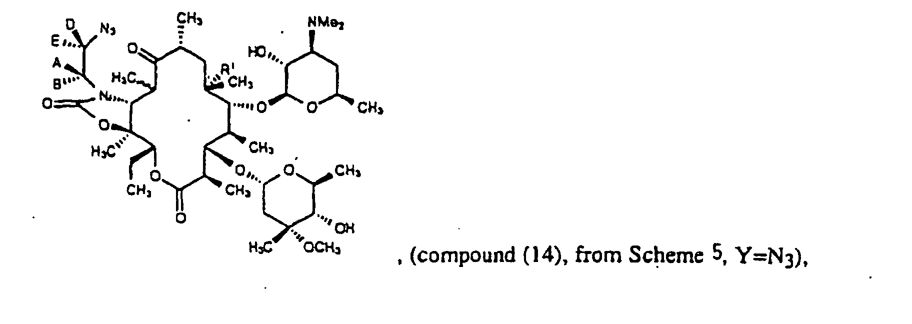 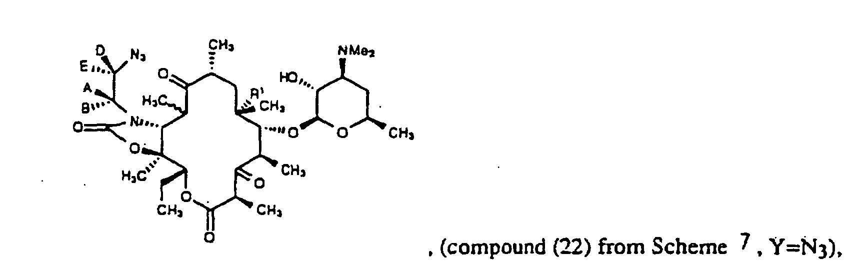 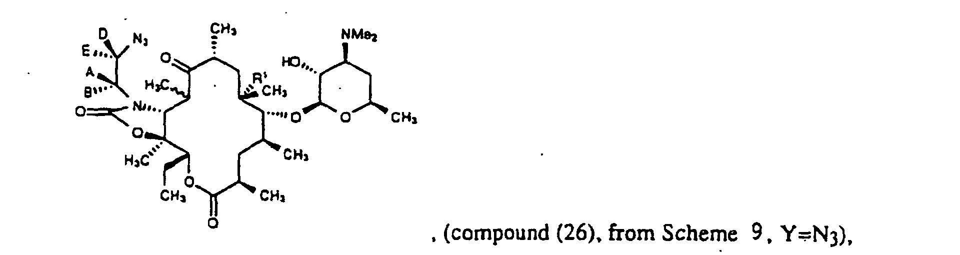 respectively;
(d) reducing the third intermediate compounds having an azido group, with suitable reducing reagents such as triphenylphosphine-water, hydrogen with a catalyst, sodium borohydride, or diallcylaluminum hydride, to prepare the fourth intermediate compounds having the formulas: 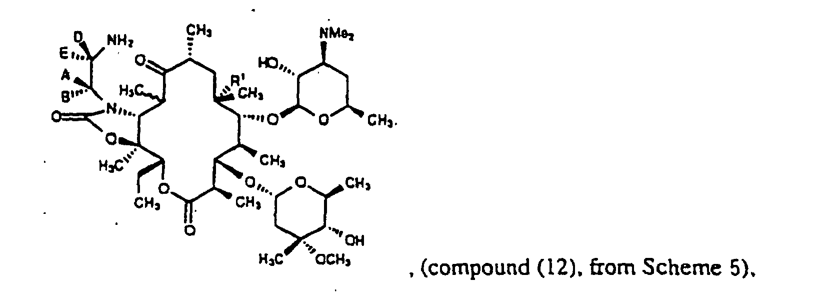 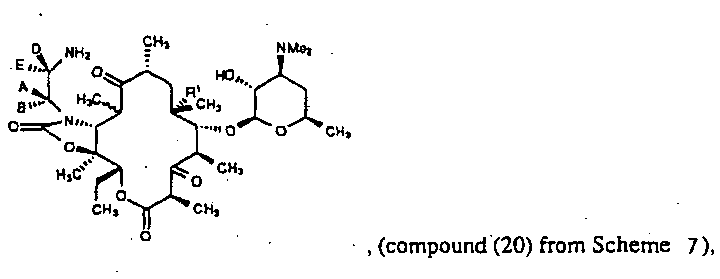 or 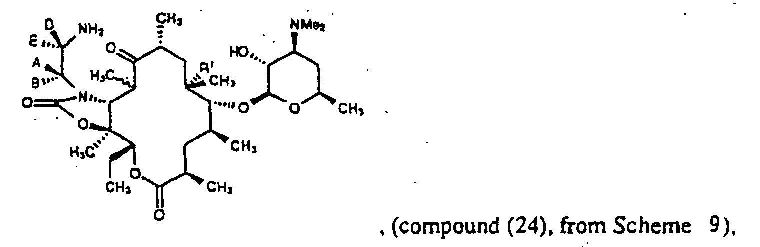 respectively; and
(e) cyclizing said fourth intermediate compounds (12), (20) or (24) by treatment with dilute acid, such as acetic acid or HCl, for example, in an organic solvent, preferably ethanol or propanol, for a period of from 4 hours to 10 days to give the desired compounds of Formulas (I), (III) or (IV).

In an alternate embodiment of the alternate process above, the third intermediate compounds may be prepared by a two-step sequence (replacing step (c) thereof) which comprises (1) reacting the hydroxy group of the bicyclic second intermediate compounds with an alkyl or aryl sulfonyl chloride, an alkyl or aryl sulfonic anhydride or trifluoromethanesulfonic anhydride in an aprotic solvent at -78°C to reaction temperature to give the corresponding sulfonate, and (2) reacting the said sulfonate with lithium azide or sodium azide in an aprotic solvent at 0°C to 100°C to give the third intermediate compound.

### Definitions

The terms "C₁-C₃-alkyl" or "C₁-C₆-alkyl" as used herein refer to saturated, straight- or branched-chain hydrocarbon radicals containing between one and three or one and six carbon atoms, respectively. Examples of C₁-C₃ alkyl radicals include methyl, ethyl, propyl and isopropyl, and examples of C₁-C₆-alkyl radicals include, but are not limited to, methyl, ethyl, propyl, isopropyl, *n*-butyl, *tert*-butyl, neopentyl and n-hexyl.

The term "C₁-C₆-alkoxy" as used herein refers to an C₁-C₆-alkyl group, as previously defined, attached to the parent molecular moiety through an oxygen atom. Examples of C₁-C₆-alkoxy, but are not limited to, methoxy, ethoxy, propoxy, isopropoxy, *n*-butoxy, *tert*-butoxy, neopentoxy and n-hexoxy.

The terms "C₁-C₃-alkyl-amino" and di-(C₁-C₃-alkyl)-amino as used herein refers to one or two C₁-C₃-alkyl groups, as previously defined, attached to the parent molecular moiety through a nitrogen atom. Examples of C₁-C₃-alkyl-amino and di-(C₁-C₃-alkyl)-amino include, but are not limited to methylamino, dimethylamino, ethylamino, diethylamino, and propylamino.

The term "aprotic solvent" as used herein refers to a solvent that is relatively inert to proton activity, *i*.*e*., not acting as a proton-donor. Examples include, but are not limited to, hydrocarbons, such as hexane and toluene, for example, halogenated hydrocarbons, such as, for example, methylene chloride, ethylene chloride, chloroform, and the like, heterocyclic compounds, such as, for example, tetrahydrofuran and N-methylpyrrolidinone, and ethers such as diethyl ether, bis-methoxymethyl ether. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th ed., edited by John A. Riddick *et al*., Vol. II, in the Techniques of Chemistry Series. John Wiley & Sons, NY, 1986.

The term "aryl" as used herein refers to unsubstituted carbocyclic aromatic groups including, but not limited to, phenyl, 1- or 2-naphthyl and the like.

The term "C₃-C₇-cycloalkyl" as used herein refers to carbocyclic groups of 3 to 7 carbons, for example, cyclopropyl, cyclobutyl, cyclopentyl, cyclohexyl, and cycloheptyl.

"Hydroxy-protecting group", as used herein, refers to an easily removable group to which are known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf, for example, T.H. Greene and P. G. M. Wuts, Protective Groups in Organic Synthesis, 2nd edition, John Wiley & Sons, New York (1991). Examples of hydroxy-protecting groups include, but are not limited to, methylthiomethyl, *tert*-dimethylsilyl, *tert*-butyldiphenylsilyl, acyl substituted with an aromatic group and the like.

The term "protected-hydroxy" refers to a hydroxy group protected with a hydroxy protecting group, as defined above, including benzoyl, acetyl, trimethylsilyl, triethylsilyl, methoxymethyl groups, for example.

A protogenic organic solvent as used herein refers to a solvent that tends to provide protons, such as an alcohol, for example, methanol, ethanol, propanol, isopropanol, butanol, t-butanol, and the like. Such solvents are well known to those skilled in the art and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of protogenic solvents may be found in organic chemistry textbooks or in specialized monographs, for example: Organic Solvents Physical Properties and Methods of Purification, 4th ed., edited by John A. Riddick *et al*., Vol. II, in the Techniques of Chemistry Series, John Wiley & Sons, NY, 1986.

Numerous asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereo-orientations or an individual isomer of assigned or unassigned orientation may be present.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al*. describe pharmaceutically acceptable salts in detail in J. Pharmaceutical Sciences, 66: 1-19 (1977), incorporated herein by reference. The salts can be prepared *in situ* during the final isolation and purification of the compounds of the invention, or separately by reacting the free base function with a suitable organic acid. Examples of pharmaceutically. acceptable, nontoxic acid addition salts are salts of an amino group formed with inorganic acids such as hydrochloric acid, hydrobromic acid, phosphoric acid. sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate. ascorbate, aspartate, benzenesulfonate, benzoate, bisulfate, borate, butyrate, camphorate, camphorsulfonate, citrate. cyclopentanepropionate, digluconate. dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than 6 carbon atoms. Examples of particular esters includes formates, acetates, propionates, butyates, acrylates and ethylsuccinates.

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemuisions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, *e*.*g*., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

Bacterial infections are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder, the activity of the specific compound employed; the specific composition employed; the age, body weight, general health, sex and diet of the patient; the time of administration, route of administration, and rate of excretion of the specific compound employed: the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens utilizing compounds according to the present invention comprise administration to a patient in need of such treatment from about 10 mg to about 1000 mg of the compound(s) of this invention per day in single or multiple doses.

### Abbreviations

Abbreviations which have been used in the descriptions of the scheme and the examples that follow are: AIBN for azobisisobutyronitrile; Bu₃SnH for tributyltin hydride: CDI for carbonyldiimidazole; DBU for 1,8-diazabicyclo[5.4.0]undec-7-ene; DEAD for diethylazodicarboxylate; DMF for dimethyl formamide: DPPA for diphenylphosphoryl azide; EtOAc for ethyl acetate; MeOH for methanol; NaN(TMS)₂ for sodium bis(trimethylsilyl)amide; NMMO for N-methylmorpholine N-oxide; TEA for triethylamine; THF for tetrahydrofuran; TPP for triphenylphosphine.

### Synthetic Methods

The compounds and processes of the present invention will be better understood in connection with the following synthetic schemes which illustrate the methods by which the compounds of the invention may be prepared. The groups A, B, D, E, R¹ and R² are as defined above unless otherwise noted below.

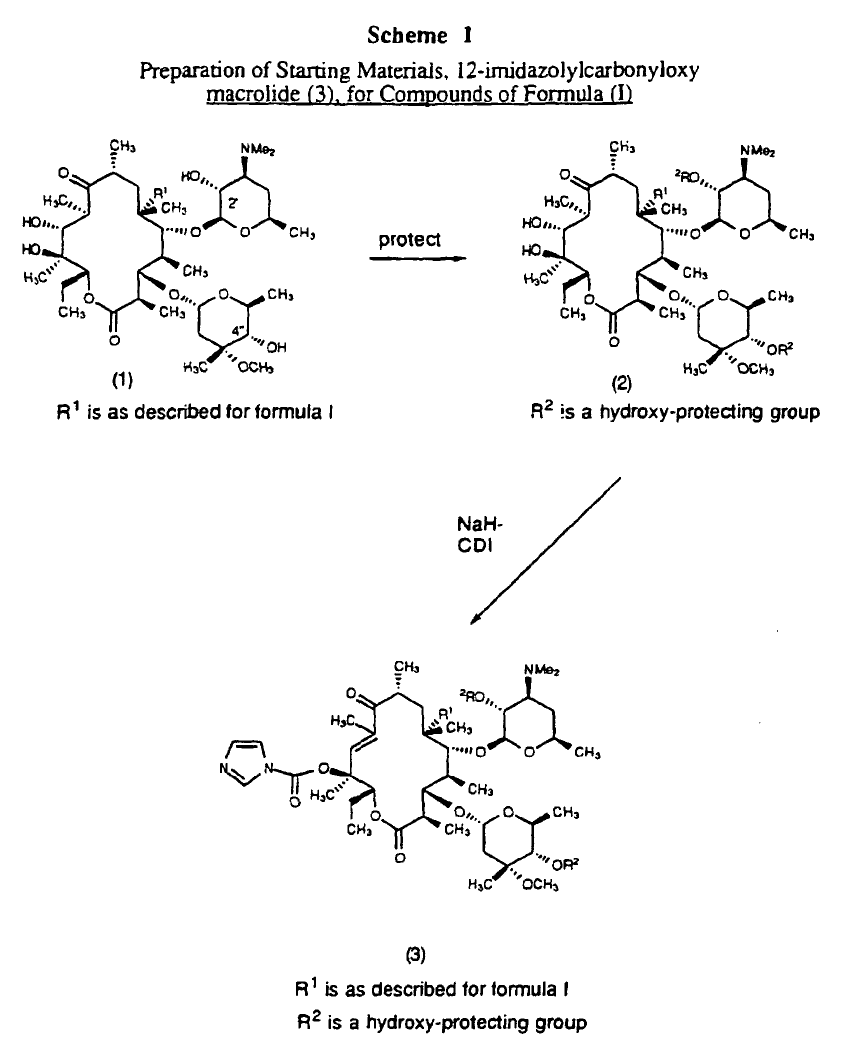

Scheme 1 illustrates a general procedure for preparing the starting material, imidazolylcarbonyloxy macrolide (3), for compounds of Formula (I). The 2'- and 4"-hydroxyl groups of compound (1) are protected by reacting (1) with suitable hydroxy group protecting reagents (cf. T. W. Greene and P. G. M. Wuts, Protective Groups in Organic Synthesis, 2nd ed., John Wiley & Son, Inc., 1991) such as acetic anhydride, benzoic anhydride, benzyl chloroformate or a trialkylsilyl chloride in an aprotic solvent, as defined above, which does not adversely affect the reaction, preferably dichloromethane, chloroform, DMF, tetrahydrofuran (THF), N-methyl pyrrolidinone or a mixture thereof. The protected macrolide (2) is reacted under anhydrous conditions with base such as sodium hydride, lithium hydride, potassium carbonate and followed by carbonyldiimidazole to form compound (3) in an aprotic solvent, as defined above, which does not adversely affect the reaction, preferably dichloromethane, chloroform, DMF, tetrahydrofuran (THF), N-methyl pyrrolidinone or a mixture thereof. The reaction may require cooling or heating, depending on the conditions used. The reaction temperature may be from -20°C to 70°C, and preferably from 0°C to reaction temperature. The reaction may require 0.5 hours to 10 days, and preferably 1-8 hours or up to 1-5 days, to complete.

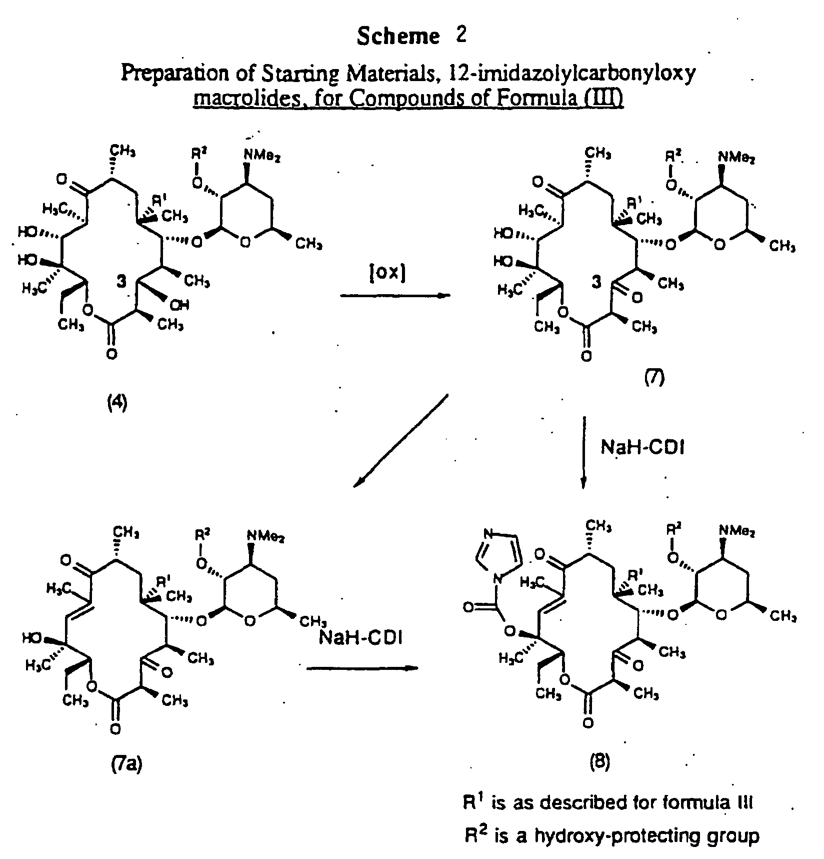

Scheme 2 illustrates two general procedures for the synthesis of 12-imidazolylcarbonyloxy macrolide (8), starting materials for the preparation of compounds of formula (III). In accordance with scheme 2, the 3-hydroxy group of a 2'-protected descladinose macrolide (4) is oxidized to the corresponding 3-oxo compound (7) using a modified Swern oxidation procedure. In scheme 2, suitable oxidizing agents are N-chlorosuccinimide-dimethyl sulfide or carbodiimide-dimethylsulfoxide. In a typical example, (4) is added into a pre-formed N-chlorosuccinimide and dimethyl sulfide complex in a chlorinated solvent such as methylene chloride at -10 to 25 °C. After being stirred for 0.5-4 hours, a tertiary amine such as triethylamine or Hunig's base is added to produce the oxidized compound (7).

In the first approach in scheme 2, (7) is reacted with sodium hydride or lithium hydride and CDI under anhydrous conditions in an aprotic solvent, as defined above, which does not adversely affect the reaction, preferably dichloromethane, chloroform, DMF, tetrahydrofuran (THF), N-methyl pyrrolidinone or a mixture thereof. The resulting alkoxide is then reacted with excess carbonyldiimidazole for 0.5 hours to 10 days in the same reaction mixture to produce (8). The preferred temperature is from -10 °C to reaction temperature.

In the second approach in Scheme 2, (7) is converted to (7a) with sodium hydride or lithium hydride and phosgene, diphosgene or triphosgene under anhydrous conditions followed by aqueous work up. Alternatively, (7) is converted to its corresponding 11-mesylate by reacting (7) with methanesulfonic anhydride in pyridine. The 11-mesylate is then converted to (7a) with an amino base such as DBU or dimethylaminopyridine in acetone or acetonitrile. (7a) is then reacted with sodium hydride or lithium hydride under anhydrous conditions in an aprotic solvent, as defined above, which does not adversely affect the reaction, preferably dichloromethane, chloroform, DMF, tetrahydrofuran (THF), N-methyl pyrrolidinone or a mixture thereof. The reactive alkoxide is then reacted with carbonyldiimidazole for 0.5 hours to 10 days in the same reaction mixture to produce (8). The preferred temperature is from -10 °C to reaction temperature.

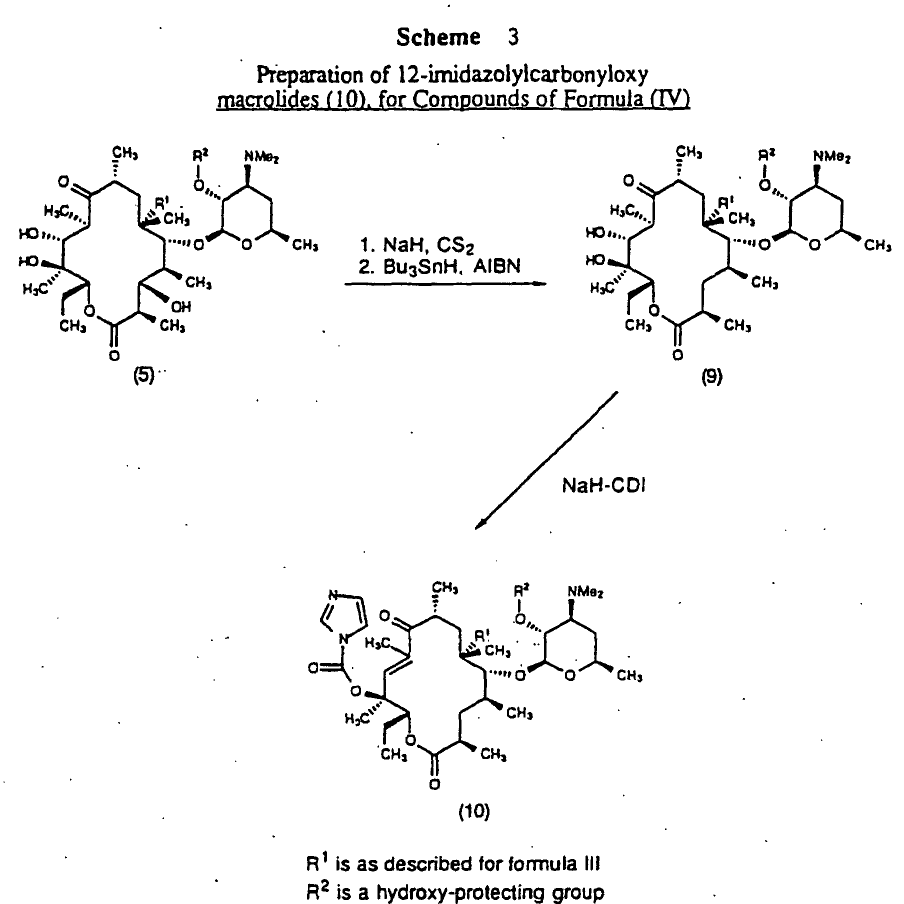

In accordance with Scheme 3, a protected descladinose compound of formula (5) is dissolved in an aprotic solvent such as THF, then reacted with an excess of NaH at from 0°C to -30°C under an inert atmosphere, followed by reaction of the intermediate anion with CS₂, then CH₃I at -5 to 10°C, to form a 3-O-xanthyl compound. This xanthate intermediate is then reacted with 1.1-1.3 equivalents of Bu₃SnH under an inert atmosphere in the presence of a catalytic amount of AIBN in a solvent suitable for a free radical reaction, such as benzene or toluene, for example, at reflux conditions to afford the desired compound (9). Compounds (9) are then reacted with carbonyldiimidazole and NaH under anhydrous conditions in an aprotic solvent, as defined above, which does not adversely affect the reaction, preferably dichloromethane, chloroform, DMF. tetrahydrofuran (THF), N-methyl pyrrolidinone or a mixture thereof, at a temperature from 0°C to reaction temperature for 0.5 hours to 10 days to provide the compounds of formula (10).

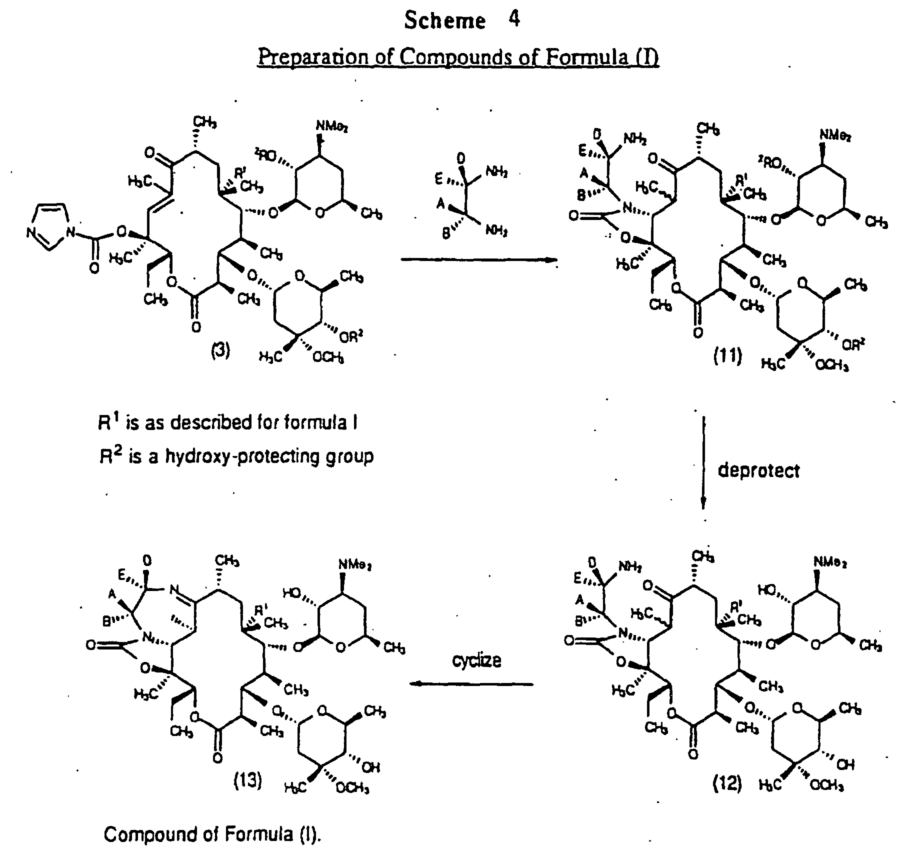

In accordance with Scheme 4, a starting material compound of formula (3) is reacted with a diamine compound having substituents A, B, D and E as defined above but with C2 or Cs symmetry or A=B=H, in a suitable solvent, such as for example, aqueous acetonitrile, DMF or aqueous DMF, to give the bicyclic compound of formula (11). The 2' and 4" hydroxy protecting group of compound (11) are then removed by standard methods (cf. T. W. Greene and P. G. M. Wuts, Protective Groups in Organic Synthesis, 2nd ed., John Wiley & Son, Inc., 1991) to give (12). When OR² is an ester, for example, such as acetate or benzoate, the compound may be deprotected by treatment with methanol or ethanol. When R² is a trialkylsilyl group, the compound may be deprotected by treatment with fluoride in THF or acetonitrile, for example. Compound (12) is then cyclized by treatment with dilute acid, such as acetic acid or HCl, for example, in a suitable organic solvent, such as methanol, ethanol or propanol, for example, for a period of from 4 hours to 10 days, from reaction temperature to reflux, in order to prepare the compound of formula (13).

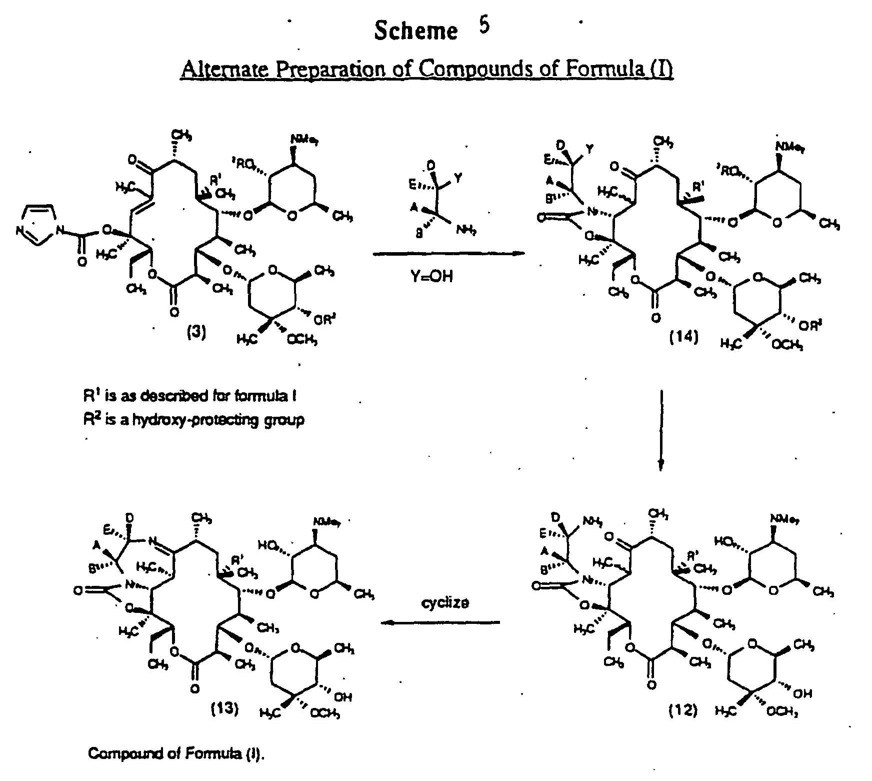

Scheme 5 illustrates an alternative preparation of compounds of formula (I). Starting material (3) is reacted with a beta-aminoalcohol (Y=OH) having substituents A, B, D and E, as defined above, in a suitable solvent system such as aqueous acetonitrile, DMF or aqueous DMF at 0 - 70 °C to give compound (14) where Y=OH. The azido intermediate, compound (14) Y=N₃, is prepared by Mitsunobu reaction by reacting compound (14) Y=OH with triphenylphosphine and diphenylphosphoryl azide-DEAD in tetrahydrofuran. Compound (14) is then deprotected by standard methods (cf. T. W. Greene and P. G. M. Wuts, Protective Groups in Organic Synthesis, 2nd ed., John Wiley & Son, Inc., 1991). When OR² is an ester, for example, such as acetate or benzoate, the compound may be deprotected by treatment with methanol or ethanol. When R² is a trialkylsilyl group, the compound may be deprotected by treatment with fluoride in THF or acetonitrile, for example. The azido intermediate, compound (14) Y=N₃, is then reduced to the amino compound (12). Suitable reducing reagents are triphenylphosphine-water, hydrogen with a catalyst, sodium borohydride, or dialkylaluminum hydride. Compound (12) is then cyclized by treatment with dilute acid, such as acetic acid or HCl, for example, in a suitable organic solvent, such as methanol, ethanol or propanol, for example, for a period of from 4 hours to 10 days, in order to prepare the compound of formula (13).

Alternatively in scheme 5, the hydroxy group (Y=OH) in (14) is activated by treatment with sulfonyl chloride, alkyl or aryl sulfonic anhydride or trifluoromethanesufonic anhydride in an aprotic solvent (*e.g.*, diethyl ether, dichloromethane, tetrahydrofuran, chloroform, pyridine or a mixture thereof). The reaction requires cooling or heating, depending on the conditions used. The reaction temperature is preferably -100 to 10 °C. The reaction may require 20 minutes to 24 hours to complete. The activated hydroxy group in (14) (*e.g.*, Y=-OSO₂CF₃) is then converted to the corresponding azide (Y=N₃, 14) by reacting with lithium azide or sodium azide in the same solvent described above. The reaction temperature is preferably 0-100 °C. The azido compound is then converted to (13) according to the procedures described above.

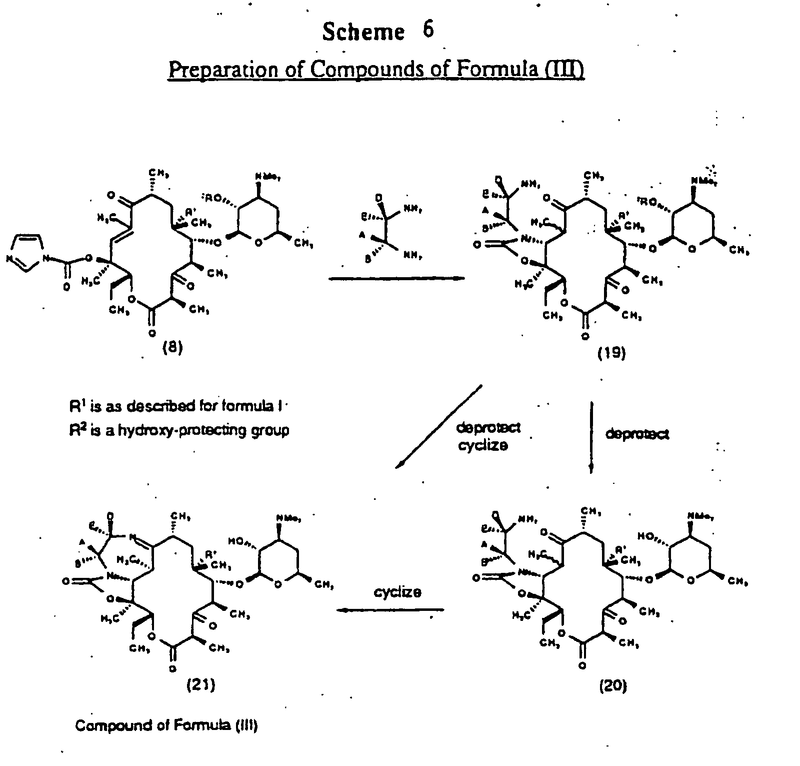

In accordance with Scheme 6, a starting material compound(8) is reacted with a diamine compound having substituents A, B, D and E as defined above but with C2 or Cs symmetry or A=B=H, in a suitable solvent, such as for example, aqueous acetonitrile, DMF or aqueous DMF, to give the bicyclic compound of formula (19). Compound (19) is then deprotected prepare the compound of formula (20). The deprotection is accomplished by standard methods (cf. T. W. Greene and P. G. M. Wuts, Protective Groups in Organic Synthesis, 2nd ed., John Wiley & Son, Inc., 1991). When OR² is an ester, for example, such as acetate or benzoate, the compound may be deprotected by treatment with methanol or ethanol. When R² is a trialkylsilyl group, the compound may be deprotected by treatment with fluoride in THF or acetonitrile, for example. Compound (20) is then cyclized by treatment with dilute acid, such as acetic acid or HCl, for example, in a suitable organic solvent, such as ethanol or propanol, for example, for a period of from 4 hours to 10 days, from reaction temperature to reflux, in order to prepare the compound of formula (21). Alternately, as is readily apparent to those skilled in the art it is possible to cyclize compound (19) first, then deprotect, to obtain the compound (21).

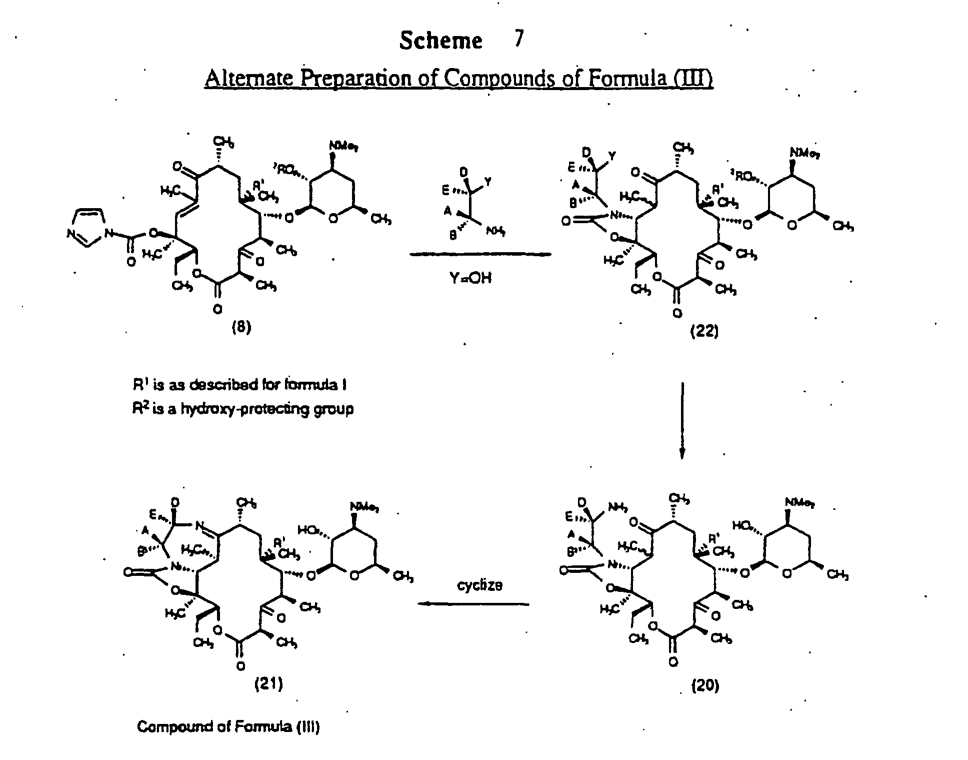

Scheme 7 illustrates an alternative preparation of compounds of formula (III). Starting material (8) is reacted with a beta-aminoalcohol (Y=OH) having substituents A, B, D and E in a suitable solvent system such as aqueous acetonitrile, DMF or aqueous DMF at 0 - 70 °C to give compound (22) where Y=OH. the azido intermediate, compound (22) Y=N₃, is prepared by Mitsunobu reaction by reacting compound (22) Y=OH with triphenylphosphine and diphenylphosphoryl azide-DEAD in tetrahydrofuran. Compound (22) is then deprotected by standard methods (cf. T. W. Greene and P. G. M. Wuts, Protective Groups in Organic Synthesis, 2nd ed., John Wiley & Son, Inc., 1991). When OR² is an ester, for example, such as acetate or benzoate, the compound may be deprotected by treatment with methanol or ethanol. When R² is a trialkylsilyl group, the compound may be deprotected by treatment with fluoride in THF or acetonitrile, for example. The deprotected azido intermediate, compound (22) Y=N₃, is then reduced to the amino compound (20). Suitable reducing reagents are triphenylphosphine-water, hydrogen with a catalyst, sodium borohydride, or dialkylaluminum hydride. Compound (20) is then cyclized by treatment with dilute acid, such as acetic acid or HCl, for example, in a suitable organic solvent, such as methanol, ethanol or propanol, for example, for a period of from 4 hours to 10 days, from reaction temperature to reflux, in order to prepare the compound of formula (21).

Alternatively in scheme 7, the hydroxy group (Y=OH) in (22) is activated by treatment with sulfonyl chloride, alkyl or aryl sulfonic anhydride or trifluoromethanesufonic anhydride in an aprotic solvent (*e*.*g*., diethyl ether, dichloromethane, tetrahydrofuran, chloroform, pyridine or a mixture thereof. The reaction requires cooling or heating, depending on the conditions used. The reaction temperature is preferably -100 to 10 °C. The reaction may require 20 minutes to 24 hours to complete. The activated hydroxy group in (22) (*e*.*g*., Y=-OSO₂CF₃) is then converted to the corresponding azide (Y=N₃, 14) by reacting with lithium azide or sodium azide in the same solvent described above. The reaction temperature is preferably 0-100 °C. The azido compound is then converted to (13) according to the procedures described above.

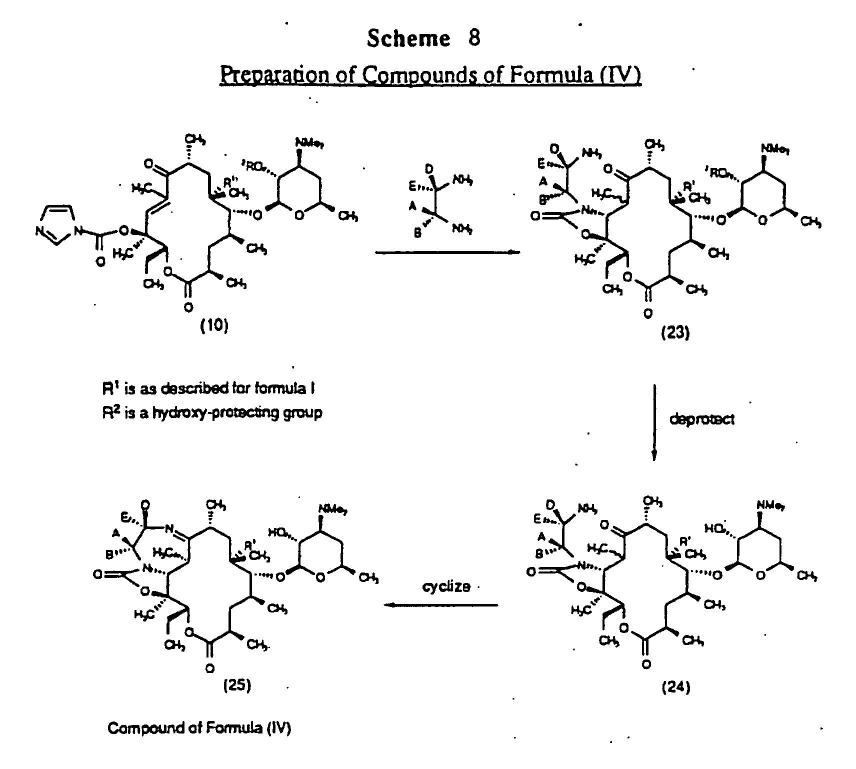

In accordance with Scheme 8, a starting material compound (10) is reacted with a diamine compound having substituents A, B, D and E as defined above but with C2 or Cs symmetry or A=B=H, in a suitable solvent, such as for example, aqueous acetonitrile. DMF or aqueous DMF, to give the bicyclic compound of formula (23). Compound (23) is then deprotected to prepare the compound of formula (24) by standard methods (cf. T. W: Greene and P. G. M. Wuts, Protective Groups in Organic Synthesis, 2nd ed., John Wiley & Son, Inc., 1991). When OR² is an ester, for example, such as acetate or benzoate, the compound may be deprotected by treatment with methanol or ethanol. When R² is a trialkylsilyl group, the compound may be deprotected by treatment with fluoride in THF or acetonitrile, for example. Compound (24) is then cyclized by treatment with dilute acid, such as acetic acid or HCl, for example, in a suitable organic solvent, such as ethanol or propanol, for example, for a period of from 4 hours to 10 days, from reaction temperature to reflux, in order to prepare the compound of formula (25).

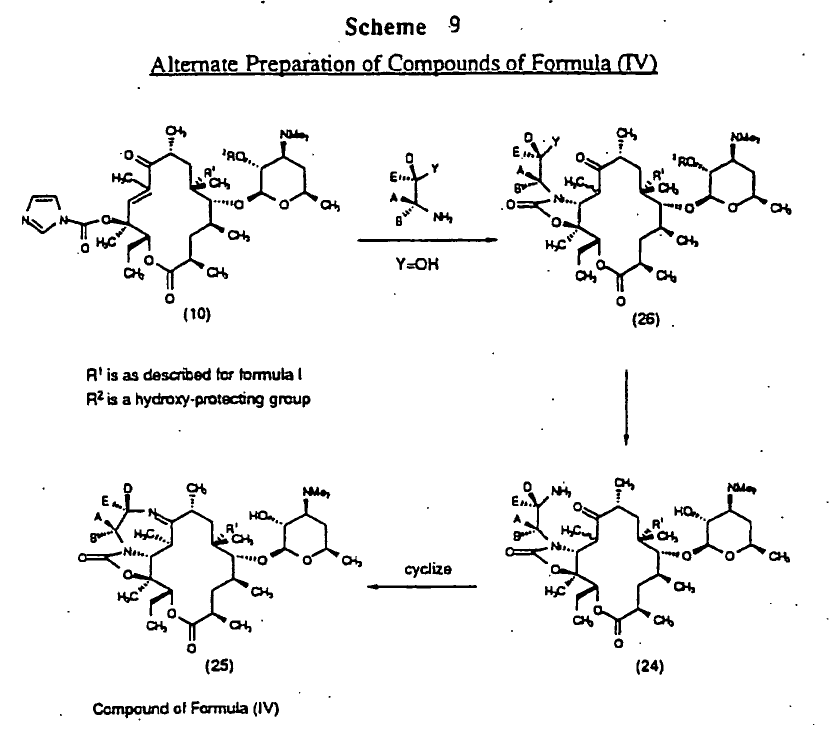

Scheme 9 illustrates an alternative preparation of compounds of formula (IV). Starting material (10) is reacted with an beta-aminoalcohol (Y=OH) having substituents A, B, D and E, as defined above, in a suitable solvent system such as aqueous acetonitrile, DMF or aqueous DMF at 0 - 70 °C to give compound (26) where Y=OH. The azido intermediate, compound (26) Y=N₃, is prepared by Mitsunobu reaction by reacting compound (26) Y=OH with diphenylphosphoryl azide-DEAD in tetrahydrofuran. Compound (26) is then deprotected by standard methods (cf. T. W. Greene and P. G. M. Wuts, Protective Groups in Organic Synthesis, 2nd ed., John Wiley & Son, Inc., 1991). When OR² is an ester, for example, such as acetate or benzoate, the compound may be deprotected by treatment with methanol or ethanol. When R² is a trialkylsilyl group, the compound may be deprotected by treatment with fluoride in THF or acetonitrile, for example. The deprotected azido intermediate, compound (26) Y=N₃, is then reduced to the amino compound (24). Suitable reducing reagents are triphenylphosphine-water, hydrogen with a catalyst, sodium borohydride, or dialkylaluminum hydride. Compound (24) is then cyclized by treatment with dilute acid, such as acetic acid or HCl, for example, in a suitable organic solvent, such as methanol, ethanol or propanol, for example, for a period of from 4 hours to 10 days, in order to prepare the compound of formula (25).

Alternatively in Scheme 9, the hydroxy group (Y=OH) in (26) is activated by treatment with sulfonyl chloride, alkyl or aryl sulfonic anhydride or trifluoromethanesufonic anhydride in a solvent does not adversely affect the reaction (*e*.*g*., diethyl ether, dichloromethane, tetrahydrofuran, chloroform, pyridine or a mixture thereof. The reaction requires cooling or heating, depending on the conditions used. The reaction temperature is preferably -100 to 10 °C. The reaction may require 20 minutes to 24 hours to complete. The activated hydroxy group in (26) (*e*.*g*., Y=-OSO₂CF₃) is then converted to the corresponding azide (Y=N₃, 26) by reacting with lithium azide in the same solvent described above. The reaction temperature is preferably 0-70 °C. The azido compound may be deprotected and converted to (25) according to the procedures described above.

The compounds and processes of the present invention will be better understood in connection with the following examples, which are intended as an illustration of and not a limitation upon the scope of the invention.

### Example 1

### Compound (IV); R¹=methoxy; R2=hydrogen; A=B=D=E=hydrogen

### 1a. 5-O-desosaminyl-6-O-methylerythronolide A

A sample of clarithromycin (3-O-cladinosyl-5-O-desosaminyl-6-O-methyl-erythronolide A, Abbott Labs, 142.38 g, 190.35 mmol) was suspended in an ethanol-water solution (1700/600 mL), and 341 mL of 1N HCl was added. The reaction mixture was stirred for 24 hours, and 2 M NaOH (170 mL) and an additional 250 mL of water were added with vigorous stirring. The precipitate was collected by filtration, washed with water, and dried to afford the title compound (95.00 g, 84%). MS m/z: 590 (M+H)⁺.

### 1b. 3-O-xanthyl-5-O-desosaminyl-6-O-methylerythronolide A

To a solution of 5-O-desosaminyl-6-O-methylerythronolide A (11.79 g, 20 mmoL, from step 1a above) in THF (100 mL) at -20°C under an inert atmosphere NaH (1.80 g, 60 mmoL, 60% dispersion) was added slowly over a 5 minute period. Several minutes later CS₂ (1.2 mL, 20 mmoL) was added. After 5 minutes of stirring CH₃I (1.24 mL, 20 mmol) was added, the reaction mixture was allowed to gradually warm to -5 - 0°C, and the mixture was stirred for 1 hour. The reaction mixture was diluted with EtOAc (400 mL), and the mixture was washed with saturated aqueous NaHCO₃ and brine, dried (MgSO₄), and concentrated to afford the crude product. The residue was purified by chromatography on silica gel, eluting with CHCl₃ and CHCl₃-MeOH (95:5), to afford the tide compound (7.68 g; 56%). MS m/z: 680 (M+H)⁺. Anal. Calcd. for C₃₂H₅₇NO₁₀S₂: C. 56.52; H, 8.45; N, 2.06; Found: C, 56.95; H, 8.65; N, 1.92.

### 1c. 3-deoxy-5-O-desosaminyl-6-O-methylerythronolide A

A solution of 3-O-xanthyl-5-O-desosaminyl-6-O-methyl erythronolide A (20.00 g; 29.41 mmoL, from step 1b), Bu₃SnH (9.49 mL, 35.29 mmol) and AIBN (∼50 mg, catalytic) in benzene (200 mL) was heated at reflux (adding 25 mg portions of AIBN periodically) for 8 hours. The organic layer was separated and washed with 10% aqueous KF and brine, dried (MgSO₄), and concentrated to afford the crude product as an oil. The residue was purified by chromatography on silica gel, eluting with CHCl₃ and CHCl₃-MeOH (97.5:2.5). The material was recrystallizated from hexane to afford the title compound (5.48 g; 32%). MS m/z: 574 (M+H)⁺. Anal. Calcd. for C₃₀H₅₅NO₉: C, 62.80; H. 9.66; N, 2.44; Found: C, 63.02; H, 9.74; N, 2.30.

### 1d. 2'-O-acetyl-3-deoxy-5-O-desosaminyl-6-O-methylerythronolide A

Samples of 3-deoxy-5-O-desosaminyl-6-O-methylerythronolide A (573 mg, 1.0 mmol, from Example 1c above), acetic anhydride (0.188 mL) and TEA (0.278 mL) were dissolved in 10 mL of methylene chloride, and the reaction mixture was stirred at reaction temperature for 20 hours. The reaction mixture was diluted with 40 mL of methylene chloride, and the organic solution was washed with saturated aqueous NaHCO₃ and brine, dried and concentrated to obtain the title compound (600 mg). MS m/z: 616 (M+H)⁺.

### 1e. Compound (10) from Scheme 3; R¹=methoxy; R²=acetyl

A sample of 2'-O-acetyl-3-deoxy-5-O-desosaminyl-6-O-methylerythronolide A (0.63 g, 1.023 mmol, from Example 1d above) was dissolved in 10 mL of THF, and the solution was cooled to -60°C. To this stirred solution was added sodium bis(trimethylsilyl) amide (1.22 mL, 1.0 M in THF). After 4 hours 1,1-carbonyldiimidazole (0.66 g, 4.09 mmol) was added as a solution in 6 mL of 2:3 DMF:THF, and the reaction mixture was slowly warmed to reaction temperature and stirred for 16 hours. The reaction was quenched by addition of 5% aqueous NaH₂PO₄, and the resulting mixture was extracted with chloroform. The organic layer was washed with water, dried over MgSO₄ and concentrated to give the title compound. MS m/z: 692 (M+H)⁺.

### 1f. Compound (23) from Scheme 8; R¹=methoxy; R²=acetyl; A=B=D=E=hydrogen

A sample of the compound from step 1e above (0.25 g, 0.36 mmol) was dissolved in 3 mL of acetonitrile, ethylenediamine (0.24 mL, 3.6 mmol) was added, and the reaction mixture was stirred at reaction temperature for 3 hours. The solvent was removed under vacuum, and the residue was dissolved in ethyl acetate. This solution was washed with saturated aqueous NaHCO₃ solution, dried over Na₂SO₄ and concentrated. The residue was twice purified by flash chromatography on silica gel, eluting with 2-20% ethanol in chloroform containing 0.5-2% NH₄OH to give the title compound. MS m/z: 684 (M+H)⁺.

### 1g. Compound (24) from Scheme 8: R¹-methoxy; A=B=D=E=hydrogen

A sample of the compound from step 1f was dissolved in methanol and stirred at reaction temperature for 64 hours. The title compound (170 mg) was obtained after filtration and removal of the solvent.

### 1h. Compound (IV) : R¹=methoxy; R²=hydrogen; A=B=D=E=hydrogen

A sample of the compound from step 6b (170 mg, 0.265 mmol) was dissolved in 2 mL of ethanol to which was added 0.03 mL of acetic acid, and the reaction mixture was stirred at reaction temperature for 16 hours. The solvent was removed, and the residue was suspended in water. The solution was adjusted to approximately pH 10-11 with 2M NaOH, and the mixture was extracted with ethyl acetate. The organic extract was washed with brine, dried over Na₂SO₄ and concentrated. The residue was purified by flash chromatography on silica gel, eluting with 10% ethanol in chloroform containing 0.1% NH₄OH. The product was re-chromatographed, eluting with 0-15% ethanol in chloroform to give the title compound (55 mg). MS m/z: 624 (M+H)⁺, 646 (M+NH₄)⁺. Anal. Calcd. for C₃₃H₅₇N₃O₈: C, 63.53; H, 9.20; N, 6.73; Found: C, 64.68; H, 9.27; N, 7.01.
¹³CNMR: C=O (16) 156.3, NCH₂ (17) 42.4, NCH₂ (18) 49.1

### Example 2

### Preparation of Intermediate Starting Material: Compound (8) from Scheme ²: R¹=methoxy; R²=benzoyl

### 2a. 5-O-desosaminyl-6-O-methylerythronolide A

A sample of clarithromycin (3-O-cladinosyl-5-O-desosaminyl-6-O-methylerythronolide A, Abbott Labs, 900 g, 1.2 mole) was suspended in water (10.8 L) and ethanol (4.0 L), and the resulting slurry was stirred at reaction temperature until homogeneous (about 20 minutes). HCl (1.00 M, 2.16 L) was added over 15 minutes, and the reaction mixture was stirred for 20 hours. NaOH solution (2.00 M, 1.20 L) was added over 30 minutes until pH 10.5-11.0 was reached, and the reaction mixture was stirred for 2 hours. The precipitate was collected and washed with cold water, which was dried under vacuum at 50°C to afford 601 g of the title compound. MS m/z (M+H)⁺: 590.

### 2b. 2'-O-benzoyl-5-O-desosaminyl-6-O-methylerythronolide A

To a solution of 5-O-desosaminyl-6-O-methylerythronolide A, (600 g, 1.01 mol from step 2a above) in methylene chloride (2.0 L) was added 90% technical grade benzoic anhydride (380 g, 1.59 mol). Triethylamine (222 mL, 1.59 mol) was added over 10 minutes, and the thick solution was stirred for 48 hours. Sodium bicarbonate solution (10%, 1.5 L) was added, and the mixture was stirred for 30 minutes. The layers were separated, and the organic fraction was washed with water (3 x 600 mL) and brine (600 mL). The organic layer was dried (Na₂SO₄) and filtered, and the volatiles were removed on a rotary evaporator to leave a syrup. Trituration with a warm solution of hexane (2.0 L) and ethyl acetate (100 mL) converted the product to white crystals. The product was filtered, washed with hexane and dried in a vacuum oven overnight at ambient temperature to give the title compound (691 g). MS m/z (M+H)⁺: 694.

### 2c. 2'-O-benzoyl-5-O-desosaminyl-3-deoxy-3-oxo-6-O-methylerythronolide A

A sample of *N*-chlorosuccinimide (57.0 g, 0.42 mol) was slurried in anhydrous methylene chloride (600 mL), and dimethyl sulfide (36.0 mL, 0.49 mol) was added dropwise over 30 minutes. A sample of the compound from step 2b (200.0 g, 0.29 mol) was dissolved in methylene chloride (1.20 L), and this solution was added to the reaction mixture over 45 minutes. After stirring for 30 minutes a solution of triethylamine (40.0 mL) in methylene chloride (200 mL) was added dropwise over 30 minutes at 0°C under nitrogen. The resulting solution was washed with sodium bicarbonate (10%, 3 x 600 mL) and brine (600 mL). The organic fraction was dried (Na₂SO₄) and filtered, and the volatiles were removed on a rotary evaporator to give a thick syrup, which became a solid upon standing. The solid was crushed and dried overnight at ambient temperature in a vacuum oven to give the title compound (196 g). MS m/z (M+H)⁺: 692.

### 2d. 2'-O-benzoyl-5-O-desosaminyl-3-deoxy-3-oxo-6-O-methyl-11-O-methanesulfonyl-6-O-methylerythronolide A

To a solution of 2'-O-benzoyl-5-O-desosaminyl-3-deoxy-3-oxo-6-O-methyl-erythronolide A from step 2c above (20.00 g, 28.9 mmole) in pyridine (40 mL) cooled to 0° and held under N₂ was added methanesulfonic anhydride (14.6 g, 83.81 mmole), and the reaction was allowed to stir at reaction temperature for 17 hours. The pyridine was removed under vacuum, and the residue was dissolved in EtOAc (400 mL). This solution was washed with saturated aqueous NaHCO₃, H₂O and brine, dried (MgSO₄), decolorized with charcoal, and filtered through a diatomaceous earth filter aid. The solvent was removed under vacuum to afford the crude product (24.46 g). This material was taken directly to the next step without further purification.

### 2e. 10,11-anhydro-2'-O-benzoyl-5-O-desosaminyl-3-deoxy-3-oxo-6-O-methylerythronolide A

The mesylate from step 2d above was dissolved in acetone (70 mL), and DBU (5.22 mL, 34.9 mmole) added. After stirring at reaction temperature for 22 hours the acetone was removed under vacuum, EtOAc (250 mL) was added, and the organic layer was washed with 100-mL portions of sat. aq. NaHCO₃ (2x), H₂O (1x), and brine (1x). The solution was dried (MgSO₄), decolorized with charcoal and filtered through a diatomaceous earth filter aid. The solvent was removed under vacuum to afford the crude product (18.54 g). This material was purified by chromatography on silica gel, eluting with 40% ethyl acetate/hexanes containing 0.25 % concentrated NH₄OH. The appropriate fractions were combined and concentrated to give the product. MS m/z (M+H)⁺: 674.

### 2f. Compound (8) from Scheme 2; R¹=methoxy; R²=benzoyl

A 500 mL flask was charged with 60% NaH (1.05 g, 26.3 mmole). The NaH was rinsed with 3 portions of hexanes, and dried under a N₂ stream. Freshly distilled THF (90 mL) was added, and the solution was cooled to 0 °C under N₂. The 10,11-anhydro compound from step 2e above (8.40 g, 12.5 mmol) was then added over a one minute period. After stirring for 15 minutes, a solution of carbonyl diimidazole (5.98 g, 36.9 mmole) in 60 mL of THF was added to the reaction mixture via cannula over a period of 15 minutes. After stirring for 5 hours, the reaction mixture was quenched with 5% KH₂PO₄ solution, and the mixture was stirred at 0 °C for 20 minutes. The mixture was extracted with ethyl acetate. The combined organic extracts were washed with brine, dried over Na₂SO₄ and concentrated under reduced pressure. The residue was purified by chromatography on silica gel, eluting with 25% -40% acetone/hexanes. The appropriate fractions were combined and concentrated to give the product. MS m/z (M+H)⁺: 768. ¹H NMR (CDCl₃): 0.90(t, 3H), 0.95(d, 3H), 1.21(d, 3H), 1.27(d, 3H), 1.32(s, 3H), 2.25(s, 6H), 2.78(s, 3H), 2.97(m, 1H), 3.58(m, 1H), 2.63(q, 1H), 4.14(d, 1H), 4.50(d, 1H), 5.00(dd, 1H), 5.65(dd, 1H), 6.75(s, 1H), 7.05(m, 1H), 7.35(m, 1H), 7.43(dd, 2H), 7.54(t, 1H), 8.02(d, 2H), 8.07(s, 1H); ¹³C NMR (CDCl₃): 204.8, 168.8, 165.0, 145.9, 138.4, 138.1, 137.0, 132.7, 130.8, 130.5, 129.7, 128.2, 117.0, 102.1, 84.5, 81.0, 78.5, 76.9, 72.0, 69.2, 63.7, 50.9, 50.2, 47.2, 40.7, 40.3, 38.8, 31.1, 30.8, 22.5, 20.9, 20.7, 20.0, 18.8, 14.8, 14.2, 13.2, 10.4.

### Example 3

### Compound of Formula (III), A, B & E=hydrogen, D=benzyl, R¹=methoxy; R²=hydrogen

### 3a. 2-(R)-(BOC-amino)-3-phenyl-1-propanol

To a 5.2 g (23.8 mmole) sample of di-t-butyl dicarbonate in 20 mL of methylene chloride held at 0°C was added (R)-2-amino-3-phenyl-1-propanol (3.0 g, 19.8 mmole, Aldrich), and the reaction mixture was stirred 1.5 hours at reaction temperature. The solvent was removed, and the residue was dried under high vacuum and taken directly to the next step.

### 3b. 2-(R)-(BOC-amino)-1-O-methanesulfonyloxy-3-phenylpropane

The material from step 3a was dissolved in 20 mL of methylene chloride and 5 mL of THF, and the solution was cooled to 0°C. Triethylamine (4.1 mL, 29.4 mmole) was added, then methanesulfonyl chloride (1.9 mL, 24.5 mmole) was added slowly. The mixture was stirred 45 minutes at reaction temperature, then the solvent was removed under vacuum. The residue was dissolved in ethyl acetate, and the solution was washed with water and brine, dried (Na₂SO₄) and filtered. The solvent was removed under vacuum to afford 6.38 g of the title compound. MS m/z (M+H)⁺: 330, MS m/z (M+NH₄)⁺: 347.

### 3c. 1-azido-2-(R)-(BOC-amino)-3-phenylpropane

The compound from step 3b above (6.36 g, 193 mmole) was dissolved in 25 mL of DMF, and 2.5 g (38 mmole) of NaN₃ was added. The reaction mixture was stirred for 24 hours at 62°C. The solution was cooled to reaction temperature, then extracted with ethyl acetate. The organic extract was washed with water and brine, dried (Na₂SO₄) and filtered. The solvent was removed under vacuum to afford 4.34 g of the title compound. MS m/z (M+H)⁺: 277, MS m/z (M+NH₄)⁺: 294.

### 3d. 1-azido-2-(R)-amino-3-phenylpropane

The compound from step 3c (4.3 g,15.6 mmole) was dissolved in 30 mL of 4 N HCl in ethanol, and the reaction mixture was stirred for 1.5 hours at reaction temperature. The solvent was stripped and chased with ether. The residue was dissolved in water, NaCl was added, and the mixture was extracted with ethyl ether, which was discarded. The aqueous layer was adjusted to pH 12 with K₂CO₃, saturated with NaCl, then extracted with CHCl₃. The organic extract was washed with brine, dried (Na₂SO₄) and filtered. The solvent was removed under vacuum to afford 2.17 g of the title compound. MS m/z (M+H)⁺: 177, MS m/z (M+NH₄)⁺: 194.

### 3e. 1,2-(R)-diamino-3-phenylpropane

A sample of the compound from step 3d (1.2 g, 6.8 mmole) was hydrogenated (4 atm) in ethanol over 1.2 g of 10% Pd/C for 21.5 hours at reaction temperature. The mixture was filtered to remove the catalyst, and the solvent was removed to afford the title compound (1.055 g). MS m/z (M+H)⁺: 151, MS m/z (M+NH₄)⁺: 168.

### 3f. Compound (19) from Scheme 6, A=B=E=H, D=benzyl, R¹=methoxy, R²=benzoyl

Samples of the compound of formula (8) (Scheme 2; R¹=methoxy; R²=benzoyl; from Example 2, 750 mg, 0.98 mmole) and 1, 2-(R)-diamino-3-phenylpropane (from step 3e above, 1.04 g, 6.92 mmole) were dissolved in 4 mL of acetonitrile and 0.5 mL of water. The reaction mixture was stirred for 24 hours at reaction temperature. The solvent was removed, and the residue was dissolved in ethyl acetate. The solution was washed with water and brine, dried (Na₂SO₄), filtered, and the solvent was removed. The residue was purified by chromatography on silica gel, eluting with hexane to 30% acetone in hexane to give 236 mg of the title compound. MS m/z (M+H)⁺: 850.

### 3g. Compound (20) from Scheme 6, A=B=E=H, D=benzyl, R¹=methoxy, R²=hydrogen

A sample (217 mg, 0.26 mmole) of the compound from step 3f above in 6 mL of methanol was stirred at reflux for 4 hours. The solvent was removed, and the residue was purified by column chromatography on silica gel, eluting with 1.5% methanol in chloroform containing 1% NH₄OH to afford 176 mg of the title compound. MS m/z (M+H)⁺: 746.

### 3h. Compound of Formula (III), A=B=E=H. D=benzyl, R¹=methoxy, R²=hydrogen

A sample of the compound from step 3g (148 mg, 0.20 mmole) was dissolved in 2.6 mL of ethanol, and of acetic acid (0.026 mL. 0.45 mmole) was added. The reaction mixture was stirred for 24 hours at reflux, then the solvent was removed. The residue was dissolved in ethyl acetate, which was washed with aqueous K₂CO₃, water and brine. The solution was dried (Na₂SO₄), filtered, and the solvent was removed under vacuum to afford 150 mg of product. This material was purified by column chromatography on silica gel, eluting with 0.5% to 0.6% methanol in chloroform containing 0.5% NH₄OH to afford 134 mg of the title compound. MS m/z (M+H)⁺: 728. ¹H NMR (CDCl₃): 0.82(t, 3H), 1.02(d, 3H), 1.18(d, 3H), 1.32(s, 3H), 1.35(d, 3H), 1.43(s, 3H), 1.50(m, 1H). 1.87(m, 1H), 2.27(s, 6H), 2.32(s, 3H), 2.56(dd. 1H), 2.66(m, 2H), 3.03(m. 1H), 3.19(dd, 1H), 3.63(s, 1H), 3.75(q, 1H), 3.94(dd, 1H), 4.04(m, 1H), 4.13(d, 1H), 4.28(d, 1H), 4.85(dd, 1H), 7.10-7.35(m, 5H); ¹³C NMR (CDCl₃): 204.0, 177.8, 169.4, 156.0, 138.3, 130.1, 127.9, 126.1, 103.9, 81.4, 79.4, 78.5, 76.4, 70.3, 69.5, 65.9, 59.5, 57.9, 51.2, 48.7, 48.3, 46.5, 42.8, 40.2, 38.5, 36.0, 31.6, 28.2, 22.0, 21.2, 19.6, 19.0, 16.7, 14.4, 14.1, 12.7, 11.0, 10.4;

### Example 4

### Compound of Formula (III), A=B=D=H, E=benzyl, R¹=methoxy; R²=hydrogen

### 4a. 1-azido-2-(S)-amino-3-phenyl-propane

Following the procedure of Example 3a, except substituting (S)-2-amino-3-phenyl-1-propanol (Aldrich) for the (R)-2-amino-3-phenyl-1-propanol thereof, and carrying the product forward as in Example 3, steps a-d, the title compound was prepared (1.74 g). MS m/z (M+H)⁺: 177, MS m/z (M+NH₄)⁺: 194.

### 4b. 1,2-(S)-diamino-3-phenylpropane

A sample of the compound from step 4a (790 mg, 4.48 mmole) was dissolved in 30 mL of THF and 6 mL of water, triphenylphosphine (5.0 g, 19.1 mmole) was added, and the reaction mixture was stirred for 24 hours at reflux. The solvent was removed, and the residue was dissolved in 2 N HCl. NaCl was added, and the solution was extracted with ethyl ether. The aqueous layer was adjusted to pH 12 with K₂CO₃, saturated with NaCl. then extracted with CHCl₃ and CHCl₃ containing 15% isopropanol. The solvent was removed under vacuum to afford 439 mg of the title compound. MS m/z (M+H)⁺: 151, MS m/z (M+NH₄)⁺: 168.

### 4c. Compound (19) Scheme 6, A=B=D=H, E=benzyl, R¹=methoxy, R²=benzoyl

Samples of the compound of formula (8) (Scheme 2; R¹=methoxy; R²=benzoyl; from Example 2, 450 mg, 0.59 mmole) and 1,2-(S)-diamino-3-phenylpropane (from step 4b above, 435 mg, 2.90 mmole) were dissolved in 2 mL of acetonitrile and 0.25 mL of water. The reaction mixture was stirred for 24 hours at reaction temperature. The solvent was removed, and the residue was dissolved in ethyl acetate. The solution was washed with brine, dried (Na₂SO₄), filtered, and the solvent was removed. The residue was purified by chromatography on silica gel, eluting with hexane to 25% acetone in hexane to give 405 mg of the title compound. MS m/z (M+H)⁺: 850.

### 4d. Compound (20), A=B=D=H, E=benzyl, R¹=methoxy, R²=hydrogen

A sample (386 mg, 0.45 mmole) of the compound from step 4c above in 7 mL of methanol was stirred at reflux for 4 hours. The solvent was removed, and the residue was purified by column chromatography on silica gel, eluting with 1.5 % methanol in chloroform containing 1% NH₄OH to afford 315 mg of the title compound. MS m/z (M+H)⁺: 746.

### 4e. Compound of Formula (III), A=B=D=H, E=phenyl, R¹=methoxy; R²=hydrogen

Following the procedure of Example 3h, except substituting the (S)-compound (150 mg, 0.20 mmole) from step 4d for the (R)-isomer of 3h, the title compound (136 mg) was prepared. MS m/z (M+H)⁺: 728. ¹H NMR (CDCl₃): 0.86(t, 3H), 1.08(d, 3H), 1.19(d, 3H), 1.33(s, 3H), 1.39(d, 3H), 1.47(s, 3H), 1.54(m, 1H), 1.92(m, 1H), 2.28(s, 6H), 2.58(s, 3H), 3.19(dd, 1H), 3.52(m, 1H), 3.67(s, 1H), 3.77(q, 1H), 4.12(d, 1H), 4.27(d, 1H), 4.92(dd, 1H), 7.10-7.40(m, 5H); ¹³C NMR (CDCl₃): 203.7, 176.0, 169.4, 156.5, 139.2, 130.0, 129.7, 128.3, 127.9, 126.3, 126.1, 104.1, 80.9, 80.5, 78.6, 78.1, 70.3, 69.5, 65.9, 62.0, 61.1, 51.2, 49.5, 48.8, 48.6, 45.9, 43.3, 42.9, 41.4, 40.2, 40.0, 35.2, 28.2, 22.2, 21.1, 19.7, 19.4, 17.1, 16.8, 15.3, 14.5; 14.1, 10.4;

### Example 5

### Compound of Formula (III); A=benzyl, B=D=E=H, R¹=methoxy, R²=hydrogen

### 5a. Compound (22) (Scheme 7), A=benzyl; B=D=E=H, R¹=methoxy, R²=benzoyl; Y=OH

Compound (8) (Scheme 2; R¹=methoxy; R²=benzoyl; from Example 2, 450 mg, 0.59 mmole) and (S)-2-amino-3-phenyl-1-propanol (1.97 g, 13.0 mmole, Aldrich) were dissolved in 4.5 mL of acetonitrile and 0.5 mL of water. The reaction mixture was stirred for 7 days at reaction temperature. The solvent was removed, and the residue was dissolved in ethyl acetate. The solution was washed with 20% aqueous KH₂PO₄, water and brine, then dried (Na₂SO₄) and filtered. The solvent was removed, and the residue dried under high vacuum to afford 1.09 g of product. This material was purified by chromatography on silica gel, eluting with hexane to 20% acetone in hexane to give 644 mg of the title compound. MS m/z (M+H)⁺: 851.

### 5b. Compound (22) (Scheme 7), A=benzyl; B=D=E=H, R¹=methoxy, R²=benzoyl; Y=azido

A sample of the compound from step 5a above (617 mg, 0.72 mmole) was dissolved in 12mL of THF, and triphenylphosphine (610 mg, 2.33 mmole) was added. This solution was cooled to 0°C, DEAD (0.375 mL, 2.38 mmole) was added dropwise over 3 minutes, and the mixture was stirred for 10 minutes. Next was added DPPA (0.515 mL, 2.37 mmole) dropwise over 3 minutes, and the mixture was stirred for 2 hours at 0°C and 48 hours at reaction temperature. The volatiles were removed under vacuum to leave an oily residue. The residue was purified by chromatography on silica gel, eluting with hexane to 30% ethyl acetate in hexane followed by 20% acetone in hexane to afford 321 mg of the title compound. MS m/z (M+H)⁺: 876.

### 5c. Compound (22) (Scheme 7), A=benzyl; B=D=E=H, R¹=methoxy, R²=hydrogen; Y=azido

A sample of the compound from step 5b above (317 mg, 0.36 mmole) was dissolved in 5 mL of methanol, and the mixture was stirred at reflux for 4.5 hours. The solvent was removed, the residue was purified by chromatography on silica gel, eluting with 1:1 acetone in hexane, and the residue was dried under high vacuum to afford 218 mg of the title compound. MS m/z (M+H)⁺: 772.

### 5d. Compound (20) (Scheme 7), A=benzyl; B=D=E=H, R¹=methoxy, R²=hydrogen

A sample of the compound from step 5c above (208 mg, 0.27 mmole) was dissolved in 3 mL of THF and 0.5 mL of water, triphenylphosphine (425 mg, 1.62 mmole) was added, and the reaction was stirred for 24 hours at reflux. The solvent was removed under vacuum, chased with toluene, and the residue dried under high vacuum. The residue was purified by chromatography on silica gel, eluting with chloroform containing 1.5% methanol and 1% NH₄OH to afford 196 mg of the title compound. MS m/z (M+H)⁺: 746.

### 5e. Compound of Formula (III); A=benzyl, B=D=E=H, R¹=methoxy, R²=hydrogen

A sample of the compound from step 5d above (128 mg, 0.17 mmole) was dissolved in 2.3 mL of ethanol and 0.023 mL (0.40 mmole) of acetic acid was added. The reaction mixture was stirred at reflux for 48 hours, and the solvent was removed. The residue was dissolved in ethyl acetate, and the solution was washed with aqueous K₂CO₃, water and brine. The solution was dried (Na₂SO₄), filtered, and the solvent was removed under vacuum to afford 100 mg of a tan compound. The residue was purified by chromatography on silica gel, eluting with chloroform containing 0.6% methariol and 0.5% NH₄OH progressing to chloroform containing 0.7% methanol and 0.5% NH₄OH to afford 52 mg of the title compound. MS m/z (M+H)⁺: 728. ¹H NMR (CDCl₃): 0.86(t, 3H), 1.08(d, 3H), 1.34(d, 3H), 1.37(d, 3H), 1.44(s, 3H), 1.52(s, 3H), 1.93(m, 1H), 2.29(s, 6H), 2.48(m, 1H), 2.67(dd, 1H), 2.86(s, 3H), 3.06(dd, 1H), 3.57(m, 1H), 3.74(q, 1H), 3.86(s, 1H), 4.33(d, 1H), 4.39(d, 1H), 4.95(dd, 1H), 7.18-7.40(m, 5H); ¹³C NMR (CDCl₃): 204.4, 169.6, 155.8, 138.0, 129.5, 129.3, 128.6, 128.5, 126.5, 103.8, 81.4, 78.8, 77.3, 70.3, 69.6, 65.9, 57.3, 53.7, 51.2, 50.8, 49.8, 47.4, 42.7, 40.3, 38.5, 35.6, 31.6, 28.2, 22.6; 22.2, 21.2, 20.6, 20.3, 15.4, 14.9, 14.1, 13.4, 11.1, 10.4;

### Example 6

### Compound of Formula (III); B=benzyl, A=D=E=H, R¹=methoxy, R²=hydrogen

Following the procedures of Example 5, except replacing the (S)-2-amino-3-phenyl-1-propanol of step 5a with (R)-2-amino-3-phenyl-1-propanol (1.97 g, 13.0 mmole, Aldrich), and carrying the product forward according to the procedures of steps 5b-5e, the title compound (83 mg) was prepared. MS m/z (M+H)⁺: 728. ¹H NMR (CDCl₃): 0.73(t, 3H), 1.05(d, 3H), 1.24(d, 3H), 1.29(d, 3H), 1.33(s, 3H), 1.37(d, 3H), 1.41(s, 3H), 2.26(s, 6H), 2.44(m, 1H), 2.68(m, 1H), 2.79(s, 3H), 3.18(dd, 1H), 3.46(m, 1H), 3.60(s, 1H), 3.71(dd, 1H), 3.81(q, 1H), 3.94(dd, 1H), 4.30(dd, 1H), 4.72(dd, 1H), 7.10-7.38(m, 5H); ¹³C NMR (CDCl₃): 204.4, 180.7, 169.3, 154.5, 138.4, 128.9, 128.8, 128.6, 128.3, 126.8, 126.2, 125.1, 104.2, 103.7, 81.1, 78.5, 78.3, 78.1, 77.2, 76.2, 70.3, 69.5, 65.8, 65.7, 62.4, 58.1, 53.6, 51.2, 51.0, 49.4, 47.6, 42.9, 40.2, 38.5, 36.7, 36.0, 31.5, 28.2, 25.2, 22.6, 21.6, 21.1, 19.7, 19.3, 16.2, 15.6, 14.6, 14.3, 14.0, 12.6, 11.1, 10.4, 10.2;

### Example 7

### Compound of Formula (III); A=E=phenyl, B=D=H, R¹=methoxy, R²=hydrogen

### 7a. Compound (19) from Scheme 6: A=E=phenyl, B=D=H, R¹=methoxy, R²=benzoyl

Compound (8) (Scheme 2; R¹=methoxy; R²=benzoyl; from Example 2, 400 mg, 0.52 mmole) and (1S,2S)-1,2-diphenyl-1,2-ethylenediamine (500 mg, 2.36 mmole, Aldrich) were dissolved in 2 mL of acetonitrile and 0.25 mL of water. The reaction mixture was stirred for 10 days at reaction temperature. The mixture was diluted with methylene chloride, the solution was dried over NaCl and Na₂SO₄, filtered, and the solvent was removed. The residue (958 mg) was taken directly to the next step. MS (m/z): 666 (M+H)⁺.

### 7b. Compound (19) from Scheme 6; A=E=phenyl, B=D=H, R¹=methoxy, R²=hydrogen

The compound from step 7a (958 mg) was dissolved in 15 mL of methanol, and the solution was heated at reflux for 24 hours. The solvent was removed, and the residue was purified by column chromatography on silica gel, eluting with 0 to 5% methanol in methylene chloride to afford 340 mg of the title product as the C10-epi-isomer. MS (m/z) : 808 (M+H)⁺. ¹H NMR (300 MHz, CDCl₃) : δ 6.90-7.20 (m, 10H), 5.48 (dd, 1H), 5.04 (d, 1H), 4.27 (d, 1H), 4.23 (d, 2H), 3.93 (q, 1H), 3.76 (d, 1H), 3.62 (d, 1H, H11), 3.52 (m, 2H)3.12-3.22 (m, 4H), 3.07 (s, 3H, OMe), 2.75 (dd, 1H), 2.75 (m, 1H), 2.2-2.5 (m), 2.24 (s, 6H, NMe₂), 1.94 (dq, 2H), 1.59 (s, 3H), 1.46 (d, 3H), 1.29 (d, 3H), 1.22 (d, 3H), 0.90 (t, 3H), 0.84 (d, 3H), 0.73 (d, 3H). ¹³C NMR (75 MHz, CDCl₃) : δ 214.2, 205.5, 171.0, 156.3, 143.5, 140.5, 128.0, 128.0, 127.9, 127.2, 126.9, 126.8, 104.1, 83.5, 78.8, 78.7, 78.2, 77.4, 77.0, 76.6, 70.2, 69.9, 69.3, 67.6, 65.6, 57.9, 51.7, 50.7, 49.0,48.5, 41.1, 41.0, 40.1, 28.2, 21.2, 21.1, 21.0, 19.3, 18.0, 16.0, 14.4, 10.5, 9.9.

### 7c. Compound of Formula (III); A=E=phenyl, B=D=H, R¹=methoxy, R²=hydrogen

Following the procedure of Example 5e, except replacing the compound from 5d with the compound from 7b, the title compound was prepared. MS (m/z) : 790 (M+H)⁺. ¹³C NMR (125 MHz, CDCl₃): selected signals: δ 203.3, 175.7, 169.5, 156.5, 104.1.

### Example 8

### Compound of Formula (III); A=methyl, B=D=E=hydrogen, R¹=methoxy, R²=hydrogen

### 8a. Compound (22) from Scheme 7; A=methyl, B=D=E=H, R¹=ethoxy, R²=benzoyl, Y=OH

Compound (8) (Scheme 2; R¹=methoxy; R²=benzoyl; from Example 2, 400 mg, 0.52 mmole) and (S)-2-amino-1-propanol (0.200 mL, 2.6 mmole, Aldrich) were dissolved in 0.9 mL of acetonitrile, 0.1 mL of water was added, and the reaction was stirred for 40 hours at reaction temperature. The solvent was removed, and the residue was dissolved in ethyl acetate. The solution was washed with water and brine, dried (Na₂SO₄), filtered, and the solvent was removed. The residue was purified by chromatography on silica gel, eluting with 20% to 30% acetone in hexane to give 135 mg of the title compound. MS m/z (M+H)⁺: 775.

### 8b. Compound (22) from Scheme 7 ; A=methyl, B=D=E=H, R¹=methoxy, R²=benzoyl, Y=azido

A sample of the compound from step 8a above (277 mg, 0.362 mmole) was dissolved in 6 mL of THF, and triphenylphosphine (302 mg, 1.15 mmole) was added. This solution was cooled to 0°C, DEAD (0.190 mL, 1.21 mmole) was added dropwise over 3 minutes, and the mixture was stirred for 10 minutes. Next was added DPPA (0.260 mL. 1.21 mmole) dropwise over 3 minutes, and the mixture was stirred for 3 hours at 0°C and 24 hours at reaction temperature. The volatiles were removed under vacuum. The residue was purified by chromatography on silica gel, eluting with 15% to 20% acetone in hexane, then rechromatographed on silica gel, eluting with 40% ethyl acetate in hexane to 25% acetone in hexane, to afford 140 mg of the title compound. MS m/z (M+K)⁺: 838.

### 8c. Compound (22) from Scheme 7; A=methyl, B=D=E=H, R¹=methoxy, R²=hydrogen, Y=azido

A sample of the compound from step 8b (133mg, 0.17 mmole) was dissolved in 2 mL of methanol, and the solution was stirred for 4 hours at reflux. The solvent was removed under vacuum, and the residue was dried under high vacuum to give a glassy residue. The residue was purified by chromatography on silica gel, eluting with 1:1 acetone in hexane to 100% acetone to afford the title compound (98 mg). MS m/z (M+H)⁺: 696.

### 8d. Compound (20) from Scheme 7: A=methyl, B=D=E=H, R¹=methoxy, R²=hydrogen

A sample of the compound from step 8c above (185 mg, 0.27 mmole) was dissolved in 4 mL of THF, and 2 mL of H₂O and triphenylphosphine (425 mg, 1.62 mmole) was added. This solution was stirred at reaction temperature for 40 hours and at reflux for 24 hours. The volatiles were removed under vacuum. The residue was purified by chromatography on silica gel, eluting with 3% methanol and 1% NH₄OH in chloroform to afford the title compound (128 mg). MS m/z (M+H)⁺: 670.

### 8e. Compound of Formula (III): A=methyl, B=D=E=H, R¹=methoxy, R²=hydrogen

A sample of the compound from step 8d above (123 mg, 0.18 mmole) was dissolved in 2.6 mL of ethanol and 0.24 mL of acetic acid, the reaction was heated at reflux for 24 hours, then stirred at reaction temperature for 48 hours. The solvent was removed under vacuum, and the residue was dissolved in ethyl acetate. The solution was washed with 10% aqueous K₂CO₃, water and brine, then dried (Na₂SO₄) and filtered. The solvent was removed, and residue was dried under high vacuum. The residue was purified by chromatography on silica gel, eluting with 0.5% NH₄OH and 2% to 3% methanol in chloroform to afford the title compound (38 mg). MS m/z (M+H)⁺: 652. ¹H NMR (CDCl₃): 0.86 (t, 3H), 1.04 (d, 3H), 1.21 (d, 3H), 1.24 (d, 3H), 1.50 (s, 3H), 1.87-1.98 (m, 2H), 2.26 (s, 6H), 2.41-2.50 (m, 1H), 2.78 (s, 3H), 3.11-3.23 (m, 3H), 3.51-3.59 (m, 1H), 3.76 (dd, 1H), 3.76 (s, 1H), 3.85 (q, 1H), 3.92 (dd, 1H), 4.31 (d, 1H), 4.37 (d, 1H), 4.90 (dd, 1H); ¹³C NMR (CDCl₃): 204.3, 181.5, 169.6, 155.6, 103.7, 81.3, 78.6, 77.9, 76.6, 70.3, 69.5, 65.8, 56.5, 53.4, 51.1, 50.6, 47.4, 47.2, 42.6, 40.2, 38.4, 35.5, 28.1, 22.0, 21.2. 20.5, 20.1, 15.8, 15.2, 14.8. 13.2, 10.6, 10.3; High resolution mass spectrum: calculated (M+H)⁺ *m*/*z* for C₃₄H₅₈N₃O₉=652.4173; observed (M+H)⁺ *m*/*z*=652.4175.

### Example 9

### Compound of Formula (III); B=methyl, A=D=E=hydrogen, R¹=methoxy, R²=hydrogen

Following the procedures of Example 8, except with a larger amount of the starting compound from Example 2 (1.23 g, 1.6 mmole) and replacing the (S)-2-amino-1-propanol of step 8a with an appropriately larger amount of (R)-2-amino-1-propanol (1.23 mL, 16.2 mmole, Aldrich), carrying the product forward as in steps 8b-8e, the title compound was prepared (71 mg). ¹H NMR (CDCl₃): 0.87 (t, 3H), 1.45 (s, 3H), 1.82-2.01 (dd, 2H), 2.26 (s, 6H), 2.37-2.51 (m, 1H), 2.72 (s, 3H), 3.07 (dd, 1H), 3.18 (dd, 1H), 3.22-3.36 (m, 1H), 3.47-3.59 (m, 1H), 3.61 (s, 1H), 3.77-3.91 (m, 2H), 4.25 (d, 1H), 4.29 (d, 1H), 5.01 (dd, 1H); ¹³C NMR (CDCl₃): 204.1, 180.7, 169.7, 154.7, 103.8, 80.6, 78.9, 78.5, 76.4, 70.3, 69.6, 65.9, 62.3, 56.6, 53.1, 51.1, 49.3, 47.8, 42.8, 40.2, 38.6, 36.5, 34.6, 31.5, 28.2, 25.3, 22.6, 21.9, 21.2, 20.7, 19.6, 19.3, 17.1, 15.8, 14.4, 14.1, 12.9, 11.0, 10.3; MS (M+H)⁺ *m*/*z*=652. High resolution mass spectrum: calculated (M+H)⁺ *m*/*z* for C₃₄H₅₈N₃O₉=652.4173; observed (M+H)⁺ *m*/*z*=652.4188.

### Example 10

### Compound of Formula (III); A=D=methyl; B=E=H, R¹=methoxy, R²=hydrogen

### 10a. meso-2,3-bis(methanesulfonyloxy)butane

Samples of meso-2,3-butanediol (10 g, 111 mmole, Aldrich) and triethylamine (92.8 mL, 666 mmole) were dissolved in methylene chloride. The solution was cooled to -78°C, and methanesulfonyl chloride (25.8 mL, 333 mmole) was added dropwise. A precipitate formed. The mixture was diluted with additional methylene chloride, and the mixture was stirred for 20 minutes at -78°C and at 0°C for 2 hours. The reaction mixture was warmed to reaction temperature, diluted with additional solvent, and washed with H₂O, aqueous NaHCO₃ and aqueous NaCl. The organic solution was dried over MgSO₄, and the solvent was removed to afford the title compound (25.01 g). ¹H NMR (300 MHz, CDCl₃) : δ 4.91 (q, 2H), 3.10 (s, 6H), 1.45 (d, 6H).

### 10b. meso-2,3-diazidobutane

A sample of the compound from step 10a (25 g) was dissolved in 250 mL of DMF, and NaN₃ (40 g) was added. The mixture was stirred vigorously at 85°C for 24 hours, then cooled to reaction temperature. The mixture was diluted with 800 mL of ether, washed with H₂O, aqueous NaHCO₃ and aqueous NaCl, then dried over MgSO₄. The solution was filtered and concentrated to afford the title compound (13.00 g). ¹H NMR (300 MHz, CDCl₃) : δ 3.50 (m, 2H), 1.30 (d, 6H).

### 10c. meso-2,3-butanediamine

A sample of the compound from step 10b (13.0 g, 125 mmole) was dissolved in ethanol and hydrogenated at 4 atm over 10% Pd/C for 20 hours at reaction temperature. The catalyst was removed by filtration, and the solvent was removed under vacuum to afford the title compound. ¹H NMR (300 MHz, CDCl₃) : δ 2.70 (m, 2H), 1.45 (br, 4H), 1.05 (d, 6H). MS (m/z) : 89 (M+H)⁺.

### 10d. Compound (19) from Scheme 6; A=D=methyl; B=E=H, R¹=methoxy, R²=benzoyl

A sample of 10,11-anhydro-2'-O-benzoyl-5-O-desosaminyl-6-O-methyl-3-oxo-erythronolide A 12-O-imidazolyl carbamate (from Example 2, 500 mg, 0.651 mmole) and meso-2,3-butanediamine (500 mg, from step 10d above) were dissolved in 3 mL of acetonitrile and 0.3 mL of water, and the reaction was stirred for 72 hours at reaction temperature and 17 hours at reflux. The solution was diluted with methylene chloride, dried over NaCl and MgSO₄, and filtered. The solvent was removed, and the residue was taken directly to the next step.

### 10e. Compound (20) from Scheme 6; A=D=methyl; B=E=H, R¹=methoxy

The compound from step 10d above was dissolved in methanol, and the solution was heated at reflux for 12 hours. The solvent was removed, and the residue was taken directly to the next step. MS (m/z) : 684 (M+H)⁺.

### 10f. Compound of Formula (III); A=D=methyl; B=E=H, R¹=methoxy, R²=hydrogen

To the material from the previous step, dissolved in methanol, acetic acid was added, and the reaction mixture was heated at reflux for 24 hours. A solution of NH₃ in methanol was added, and the solution was concentrated. The residue was dissolved in ethyl acetate, and the solution was washed with 1 N NaOH, H₂O and brine, then dried over Na₂SO₄. The solvent was removed, and the residue was purified on silica gel, eluting with 10% methanol in methylene chloride to 10% methanol (containing NH₃) in methylene chloride to afford the title compound. This material was rechromatographed on pH 8 silica gel, eluting with 4:1 ethyl acetate:hexane to 100% ethyl acetate to afford 116 mg of the title compound. The NMR analysis confirmed the product to be the A=D=methyl isomer. MS (m/z): 666 (M+H)⁺. HRMS Calc. for C₃₅H₆₀N₃O₉: 666.4330; Observed: 666.4326. ¹H NMR (300 MHz, CDCl₃): δ 4.81 (dd, 1H), 4.27 (d, 1H), 4.22 (d, 2H), 4.05 (m, 1H), 3.96 (m, 1H), 3.78 (q, 1H), 3.65 (s, 1H, H11), 3.48 (m, 1H), 3.10 (dd, 1H), 3.06 (m, 1H), 2.75 (q, 1H), 2.67 (s, 3H), 2.65 (m, 1H), 2.37 (m, 2H), 2.19 (s, 6H), 1.85, 1.49 (m, 2H), 1.59, 1.15 (m, 2H), 1.55 (m, 2H), 1.42 (s, 3H), 1.27 (d, 3H), 1.27 (s, 3H), 1.25 (d, 2H), 1.24 (d, 3H), 1.17 (d, 3H), 1.16 (d, 3H), 1.04 (d, 3H), 0.96 (d, 3H), 0.78 (t, 3H). ¹³C NMR (75 MHz, CDCl₃) : δ 204.2, 179.0, 169.5, 155.6, 118.8, 103.7, 81.3, 78.6, 77.7, 76.8, 70.2, 69.4, 65.8, 56.1, 55.5, 51.9, 51.0, 50.4, 47.0, 42.5, 40.1, 38.4, 35.2, 28.1, 22.3, 22.0, 21.1, 20.7, 20.1, 15.0, 14.8, 13.1, 11.5, 10.6, 12.2. IR (film) : 3460 (w), 2972, 1750, 1718 (w), 1647, 1456, 1423, 1372, 1305, 1246, 1163, 1107, 1051, 989, 757 cm⁻¹.

### Example 11

### Compound of Formula (III): A=E=methyl; B=D=H. R¹=methoxy, R²=hydrogen

### 11a. 2S, 3S-butanediamine

Following the procedures of Example 10 steps a-c, except substituting (R,R)-2,3-butanediol (1 g, 11.1 mmole, Aldrich) for the meso-isomer thereof of step 10a, and carrying the product forward as in steps 10b and 10c, the title compound (494 mg) was prepared. MS (m/z) : 89 (M+H)⁺. ¹H NMR (300 MHz, CDCl₃) : δ 2.62 (m, 2H), 1.45 (br s, 4H), 1.08 (d, 6H).

### 11b. Compound (19) from Scheme 6: A=E=methyl; B=D=H, R¹=methoxy, R²=benzoyl

A sample of 10,11-anhydro-2'-O-benzoyl-5-O-desosaminyl-6-O-methyl-3-oxo-erythronolide A 12-O-imidazolyl carbamate (from Example 2, 1.56 g, 2.03 mmole) and 2S, 3S-butanediamine (400 mg, 4.54 mmole, from step 11a above) were dissolved in 16 mL of 20% aqueous acetonitrile, and the reaction mixture was stirred at reaction temperature for 7 days. The solution was diluted with methylene chloride, dried over NaCl and MgSO₄, and filtered. The solvent was removed, and the residue was taken directly to the next step.

### 11c. Compound (20) from Scheme 6: A=E=methyl; B=D=H. R¹=methoxy

The compound from step 11b above was dissolved in methanol, and the solution was heated at reflux for 12 hours. The solvent was removed, and the residue was taken directly to the next step.

### 11d. Compound of Formula (III): A=E=methyl; B=D=H, R¹=methoxy, R²=hydrogen

The material from the previous step was dissolved in 20 mL of ethanol, acetic acid (0.80 mL) was added, and the reaction mixture was heated at reflux for 3 days. NH₃/methanol was added, and the solvent was removed. The residue was dissolved in ethyl acetate, and the solution was washed with 1 N NaOH, H₂O and brine, then dried over Na₂SO₄. The solvent was removed, and the residue was purified on neutral silica gel, eluting with 4:1 ethyl acetate:hexane to 5% methanol in ethyl acetate to afford 682 mg of the title compound. MS (m/z) : 666 (M+H)⁺. HRMS Calc. for C₃₅H₆₀N₃O₉: 666.4330; Observed: 666.4333. ¹H NMR (300 MHz, CDCl₃): δ 4.85 (dd, 1H), 4.35 (d, 1H), 4.30 (d, 1H), 4.12 (q, 2H), 3.65 (s to d, 1H, J=1.2 Hz), 3.56 (m, 1H), 3.46 (br s, 1H), 3.21 (dd, 1H), 3.13 (t, 1H), 2.86 (q, 1H), 2.78 (s, 3H), 2.68 (m, 2H), 2.46 (dt, 1H), 2.28 (s, 6H), 1.95 (m, 1H), 1.68 (m, 3H), 1.52 (s, 3H), 1.38 (d, 3H), 1.36 (d, 3HO, 1.33 (s, 3H), 1.32 (d, 3H), 1.31 (d, 3H), 1.26 (d, 3H), 1.21 (d, 3H), 1.05 (d, 3H); 0.87 (t, 3H). ¹³C NMR (75 MHz, CDCl₃) : δ 203.9, 177.9, 169.5, 156.4, 103.8, 81.1, 78.5. 78.4, 77.6, 70.3, 69.5, 65.8, 60.4, 56.9, 52.3, 50.9, 50.6, 47.6, 43.9, 40.2, 39.5, 35.0, 28.1, 21.7, 21.1, 20.8, 20.2, 19.9, 18.3, 16.3, 15.6, 14.7, 14.1, 13.6, 10.1. IR (KBr): 3441, 2972, 2938, 1755, 1117 (w), 1653, 1457, 1425, 1376, 1306, 1248, 1167, 1109, 1053 cm⁻¹.

### Example 12

### Compound of Formula (III); B=D=H; A and E taken together is -CH₂CH₂CH₂-, R¹=methoxy, R²=hydrogen

### 12a. racemic-trans-1,2-cyclopentanediamine

Following the procedures of Example 10 steps a-c, except substituting (DL)-1,2-cyclopentanediol (5.0 g, 49.0 mmole, Aldrich) for the diol of step 10a, and carrying the product forward as in steps 10b and 10c, the title compound (2.76 g) was prepared. MS (m/z) : 101 (M+H)⁺. ¹H NMR (300 MHz, CDCl₃) : δ 2.75 (m, 2H), 2.00 (m, 2H), 1.65 (m, 2H), 1.50 (s, 4H), 1.30 (m, 2H).

### 12b. Compound (19) from Scheme 6; B=D=H; A and E taken together is -CH₂CH₂CH₂-, R¹=methoxy, R²=benzoyl

Samples of 10,11-anhydro-2'-O-benzoyl-5-O-desosaminyl-6-O-methyl-3-oxo-erythronolide A 12-O-imidazolyl carbamate (from Example 2, 200 mg, 0.26 mmole) and *racemic-trans*-1,2-cyclopentanediamine (104 mg, 1.04 mmole, from step 12a) were dissolved in 20% aqueous aceconitrile, and the reaction mixture was stirred at reaction temperature for 4 days and at reflux for 8 hours. The solution was diluted with 100 mL of ethyl acetate, dried over NaCl and MgSO₄, and filtered. The solvent was removed, and the residue was purified by chromatography on silica gel, eluting with 5% to 10% methanol in methylene chloride to afford the title compound (88 mg). MS (m/z) : 800 (M+H)⁺.

### 12c. Compound (20) from Scheme 6; B=D=H; A and E taken together is -CH₂CH₂CH₂-, R¹=methoxy

The compound from step 12b above (88 mg) was dissolved in methanol, and the solution was heated at reflux for 10 hours. The solvent was removed, and the residue was purified by chromatography on silica gel, eluting with 5% methanol in methylene chloride to afford the title compound. MS (m/z) : 696 (M+H)⁺.

### 12d. Compound of Formula (III); B=D=H; A and E taken together is -CH₂CH₂CH₂-, R¹=methoxy, R²=hydrogen

Following the procedure of Example 11d the title compound was prepared. MS (m/z): 678 (M+H)⁺.

### Example 13

### Compound of Formula (III); B=E=H; A and D taken together is -CH₂CH₂CH₂CH₂-, R¹=methoxy, R²=hydrogen

### 13a. Compound (19) from Scheme 6; B=E=H; A and D taken together is -CH₂CH₂CH₂CH₂-, R¹=methoxy, R²=benzoyl

Following the procedure of Example 11, step 11b, except substituting cis-1,2-cyclohexanediamine (800 mg, 1.04 mmole, Aldrich) for the diamine thereof, the title compound was prepared.

### 13b. Compound of Formula (III); B=E=H; A and D taken together is -CH₂CH₂CH₂CH₂-, R¹=methoxy, R²=hydrogen

Following the procedures of Example 11 steps c and d, substituting the compound of step 13a for the compound of 11b thereof, the title compound was prepared. ¹H NMR (300 MHz, CDCl₃) : δ 4.87 (dd, 1H), 4.34 (d, 1H), 4.31 (d. 1H), 4.10 (br s, 1H), 4.07 (br m, 1H), 3.84 (q, 1H), 3.70 (s, 1H, H11), 3.56 (m, 1H), 3.22 (dd, 1H), 3.14 (pent, 1H), 2.78 (s, 3H, OMe), 2.49 (m, 1H), 2.30 (s, 6H), 1.30-2.00 (m), 1.49 (s, 1H), 1.39 (s, 3H), 1.37 (d, 3H), 1.32 (d, 3H), 1.26 (d, 3H), 1.22 (d, 3H), 1.06 (d, 3H), 0.85 (t, 3H).
¹³C NMR (75 MHz, CDCl₃) : δ 203.8, 178.1, 169.3, 155.6, 103.7, 81.1, 78.6, 78.1, 76.64, 70.2, 69.3, 65.7, 56.3, 55.9, 55.8, 51.0, 50.4, 47.4, 42.6, 40.1, 38.2, 35.4, 34.4, 28.1, 25.2, 25.1, 25.0, 21.9, 21.0, 20.2, 20.1, 19.2, 15.6, 14.7, 12.9, 10.6, 10.2. MS (m/z) : 692 (M+H)⁺.

### Example 14 (comparative example) Compound of Formula (I): A=B=D=E=H, R¹=hydrogen, R²=hydrogen

### 14a. 2'-O-acelyl-6-deoxy-erythromycin A

A sample of 6-deoxyerythromycin A (4.34 g, 6.04 mmole, Abbott Labs) was dissolved in 250 mL of methylene chloride, and acetic anhydride (1.54 g, 15 mmole) and triethylamine (1.22 g, 12 mmole) were added. The reaction mixture was stirred at reaction temperature for 16 hours, then poured into 5% aqueous NaHCO₃. The mixture was extracted with methylene chloride, and the organic layer was dried, filtered and concentrated. The residue was chased with toluene, ethylene dichloride, chloroform and methylene chloride. The residue then dried under high vacuum to afford 4.46 g of the title product, which was taken to the next step without further purification. MS m/z (M+H)⁺: 760.

### 14b. 2'-O-acetyl-6-deoxy-4"-triethylsilyl-erythromycin A

A sample of the compound from step 14a (4.44 g, 5.84 mmole) was dissolved in 100 mL of dry methylene chloride, and imidazole (1.59 g, 23.3 mmole) was added. The mixture was cooled to 0°C, and a solution of triethylsilyl chloride (1.76 g, 117 mmole) in 25 mL of methylene chloride dropwise. The reaction mixture was stirred for 1 hour at 0°C and at reaction temperature for 16 hours. The reaction mixture was poured into 5% aqueous NaHCO₃, and the mixture was extracted with CHCl₃. The organic extract was washed with saturated brine, dried over MgSO₄, filtered and concentrated. The residue was dried under high vacuum for 48 hours to afford 5.38 g of the title product, which was taken to the next step without further purification.. MS m/z (M+H)⁺: 874.

### 14c. 2'-O-acetyl-10,11-anhydro-6-deoxy-4"-triethylsilyl-erythromycin A 12-O-imidazolyl carbamate (Compound (3) from Scheme 4, R¹=hydrogen, 2'-R²=acetyl; 4"-R²=triethylsilyl)

A sample of the compound from step 14b (5.36 g, 6.13 mmole) was dissolved in 45 mL of dry DMF, and carbonyldiimidazole (4.98 g, 30 mmole) was added. After the reagent had dissolved, the solution was diluted with 15 mL of THF, and the solution was cooled to 0°C. To this solution was added NaH (807 mg, 60% dispersion, 20.2 mmole) in portions. The reaction mixture was stirred for 30 minutes, and 10 mL of H₂O was added. The reaction mixture was poured into saturated brine, and the resulting mixture was extracted with ethyl acetate. The ethyl acetate solution was washed with brine, dried over MgSO₄, filtered and concentrated. The residue was co-distilled with ethylene dichloride, chloroform and methylene chloride. The residue was dried under high vacuum for 16 hours to afford 6.95 g of the title product, which was taken to the next step without further purification. MS m/z (M+H)⁺: 986.

### 14d. Compound (11) from Scheme 4; A=B=D=E=H; R¹=hydrogen, 2'-R²=acetyl; 4"-R²=triethylsilyl

A sample of the compound from step 14c (6.93 g, 6.13 mmole) was dissolved in 50 mL of acetonitrile, ethylene diamine was added (4.31 g, 7.19 mmole), and the solution was stirred for 17 hours at reaction temperature. The reaction mixture was poured into saturated brine containing NH₄OH. This mixture was extracted with chloroform, and the organic extracts were combined, filtered and concentrated. The residue was co-distilled with ethylene dichloride, chloroform and methylene chloride. The residue was dried under high vacuum for 72 hours to afford 5.77 g of the title product which was taken to the next step without further purification. MS m/z (M+H)⁺: 924.

### 14e. Compound (12) from Scheme 4; A=B=D=E=H; R¹=hydrogen, 2'-R²=4"-R²=hydrogen

A sample of the compound from step 14d (5.75 g) was dissolved in THF (120 mL) and 14.3 mL of tetrabutylammonium fluoride (1 M in THF) was added in one portion. The reaction mixture was stirred at reaction temperature for 4 hours, diluted with 400 mL of methanol, and stirred at reaction temperature for 64 hours. The solution was concentrated and then poured into 5% aqueous NaHCO₃ containing 0.5% NH₄OH. The mixture was extracted with CHCl₃, the extract was washed with brine, and dried over MgSO₄. The solvent was removed, and the residue was co-distilled with ethylene chloride and methylene chloride. The residue was further dried under vacuum for 16 hours to afford the title compound (5.59 g).

### 14f. Compound of Formula (I): A=B=D=E=H: R¹=hydrogen, R²=hydrogen

A sample of the compound from step 14e (5.56 g, 7.07 mmole) was dissolved in 60 mL of ethanol, and acetic acid (637 mg, 106 mmole) was added. The solution was heated at reflux for 3.5 hours, cooled to reaction temperature, then poured into 5% aqueous NaHCO₃. This mixture was extracted with CHCl₃, and the organic extracts were combined, dried over MgSO₄, filtered and concentrated. The residue was co-distilled with ethylene dichloride, chloroform and methylene chloride, then dried under high vacuum for 72 hours to afford 4.12 g of the product. This material was purified by chromatography on silica gel, eluting with 1:6 TEA:ethyl acetate, to afford the title compound (1.29 g). MS m/z (M+H)⁺: 768. ¹H NMR (CDCl₃) δ: 0.88 (m), 2.28 (s), 3.28 (s), 4.23 (m), 4.85 (m), 4.92 (m). ¹³C NMR (CDCl₃) δ: 40.3 (N(CH₃)₂), 42.8 (NCH2), 49.4 (OCH₃), 49.9 (NCH₂), 155.8 (O-CO-N). IR (CDCl₃): 1755 cm⁻¹.

### Example 15

### Compound of Formula (III): R¹=OCH₃, R²=H; A=B=D=H; E=-CH₂NH₂

### 15a. 1,2-diazido-3-(BOC-amino)propane

A sample of 3-(BOC-amino)propene (Aldrich, 15.72 g, 0.1 mole), manganese triacetate (80.43 g, 0.3 mole) and NaN₃ (65.01 g, 1.0 mmole) were suspended in 400 mL of acetic acid, and the mixture was heated at 100°C for 10 minutes. The mixture was cooled in an ice bath and diluted with 400 mL of 1N NaOH. The mixture was extracted with ethyl acetate, which was washed with 1 N cold NaOH, H₂O, NaHCO₃, brine and dried over Na₂SO₄. The solvent was removed, and the residue was chromatographed on silica gel, eluting with 4: 1 hexane:ether to give 7.18 g of the title compound. MS m/z (M+NH₄)⁺: 259.

### 15b. 1,2-diamino-3-(BOC-amino)propane

The compound of the previous step was dissolved in ethanol and hydrogenated (4 atm H₂) over Pd/C for 22 hours at reaction temperature. The solvent was removed, and the product was taken to the next step. MS m/z (M+H)⁺: 190.

### 15c. Compound of Formula (III): R¹=OCH₃, R²=H; A=B=D=H; E=-CH₂NH₂

Following the procedures of Example 3 steps f-h, samples of the compound of formula (8) (Scheme 2; R¹=methoxy; R²=benzoyl; from Example 2, 400 mg, 0.52 mmole) and the compound from step 15b (1.478 g, 7.81 mmol) were reacted and the title product was obtained. Chromatographic separation of the isomers of the N-BOC compound, followed by deprotection with HCl in ethanol at reaction temperature gives the title compound. MS m/z (M+H)⁺: 889.

### Example 16

### Compound of Formula (III): R¹=OCH₃, R²=H; A=B=E=H; D=-CH₂NH₂

The compound is obtained by deprotection with HCl in ethanol at reaction temperature of the isomer separated by chromatography from the mixture obtained in Example 15c.

### Example 17

### Compound of Formula (III): R¹=OCH₃, R²=H; B=E=H; A and D taken together is -CH₂CH₂CH₂

### 17a. cyclopentane cis-1,2-diamine

Following the procedures of Example 10 steps a-c, except substituting cis-1,2-cyclopentanediol for the diol of step 10a, and carrying the product forward as in steps 10b and 10c, the title compound is prepared.

### 17b. Compound of Formula (III): R¹=OCH₃, R²=H; B=E=H; A and D taken together is -CH₂CH₂CH₂

Following the procedures of Example 10 steps d-f, except substituting *cis*-1,2-cyclopentanediamine, from the previous step, for the meso-2,3-butanediamine thereof, the title compound is prepared.

### Example 18

### Compound of Formula (III): R¹=OCH₃, R²=H; B=E=H; A and D taken together is -CH₂OCH₂

### 18a. tetrahydrofuran cis-3,4-diamine

Following the procedures of Example 10 steps a-c, except substituting 1,4-anhydroerythritol for the diol of step 10a, and carrying the product forward as in steps 10b and 10c, the title compound is prepared.

### 18b. Compound of Formula (III): R¹=OCH₃, R²=H; B=E=H; A and D taken together is -CH₂OCH₂

Following the procedures of Example 10 steps d-f, except substituting tetrahydrofuran cis-3,4-diamine, from the previous step, for the meso-2,3-butanediamine of step 10d, the title compound is prepared.

### Example 19

### Compound of Formula (III): R¹=OCH₃, R²=H; B=E=H; A and D taken together is -CH₂-NH-CH₂-

### 19a. cis-3,4-diamino-pyrrolidine

Pyrolline (Aldrich) is N-protected by treatment with benzyloxycarbonyloxysuccinimide in ethyl acetate at reaction temperature. The N-Cbz-pyrroline is oxidized with catalytic OsO₄ and excess N-methylmorpholine N-oxide (NNMO) in THF and t-butanol according to the procedure of *Tetrahedron Lett*., **1976**: 1973 to give the N-Cbz-pyrroline-3,4-diol. Following the procedures of Example 10 steps a-c, except substituting N-Cbz-pyrroline-3,4-diol for the diol of step 10a, and carrying the product forward as in steps 10b and 10c, the title compound is prepared.

### 19b. Compound of Formula (III): R¹=OCH₃, R²=H; B=E=H; A and D taken together is -CH₂-NH-CH₂

Following the procedures of Example 10 steps d-f, except substituting cis-3,4-diamino-(1-Cbz-pyrrolidine), from the previous step, for the meso-2,3-butanediamine of step 10d, the title compound is prepared.

### Example 20

### Compound of Formula (III): R¹=OCH₃, R²=H; B=E=H; A and D taken together is -CH₂-N(Cbz)-CH₂-

Starting with the Compound of Formula (III): R¹=OCH₃, R²=H; B=E=H; A and D taken together is -CH₂-NH-CH₂-, from Example 19 above, adding the Cbz group by treatment with benzyloxycarbonyloxysuccinimide in ethyl acetate at reaction temperature, the title compound is prepared.

### Example 21

### Compound of Formula (III): R¹=OCH₃, R²=H; B=E=H; A and D taken together is -CH₂-N(benzyl)-CH₂-

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with benzyl chloride in the presence of triethylamine, the title compound is prepared.

### Example 22

### Compound of Formula (III): R¹=OCH₃, R²=H; B=E=H; A and D taken together is -CH₂-N(benzoyl)-CH₂

Starting with the Compound of Formula (III): R¹=OCH3, R²=H; B=E=H; A and D taken together is -CH₂-NH-CH₂-, from Example 19 above, reacting the amine of the -CH₂-NH-CH2- group with benzoyl chloride in the presence of triethylamine, the title compound is prepared.

### Example 23

### Compound of Formula (III): R¹=OCH₃, R²=H; B=E=H; A and D taken together is -CH₂-N(phenyl-CH₂-CH₂-)-CH₂-

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with 2-phenylethyl bromide in the presence of triethylamine, the title compound is prepared.

### Example 24

### Compound of Formula (III): R¹=OCH₃, R²=H; B=E=H; A and D taken together is -CH₂-N(4-Cl-phenyl-CH₂-)-CH₂-

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with 4-chlorobenzyl chloride in the presence of triethylamine, the title compound is prepared.

### Example 25

### Compound of Formula (III): R¹=OCH₃, R²=H; B=E=H; A and D taken together is -CH₂-N(4-pyridyl-CH₂-)-CH₂

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with 4-picolyl chloride in the presence of triethylamine, the title compound is prepared.

### Example 26

### Compound of Formula (III): R¹=OCH₃, R²=H; B=E=H; A and D taken together is -CH₂-N(2-pyridyl-CH₂-)-CH₂-

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with 2-picolyl chloride in the presence of triethylamine, the title compound is prepared.

### Example 27

### Compound of Formula (III): R¹=OCH₃, R²=H; B=E=H; A and D taken together is -CH₂-NH(3-pyridyl-CH₂-)-CH₂-

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with 3-picolyl chloride in the presence of triethylamine, the title compound is prepared.

### Example 28

### Compound of Formula (III): R¹=OCH₃, R²=H; B=E=H; A and D taken together is -CH₂-N(4-quinolyl-CH₂-)-CH₂-

Starting with the Compound of Formula (III): R¹=OCH3, R²=H; B=E=H; A and D taken together is -CH₂-NH-CH₂-, from Example 19 above, reacting the amine of the -CH₂-NH-CH₂- group with 4-chloromethylquinoline in the presence of triethylamine, the title compound is prepared.

### Example 29

### Compound of Formula (III): R¹=OCH₃, R²=H; B=E=H; A=D=-CH₂-O-CH₂-phenyl

### 29a. PhCH₂OCH₂CH(NH₂)CH(NH₂)CH₂OCH₂Ph

Meso-erythritol (Aldrich) is 1,4-dibenzylated by reaction with NaH and benzyl bromide in DMF according to the procedure of El Amin *et al., J. Org. Chem*. 44:3442, (1979). Following the procedures of Example 10 steps a-c, except substituting 1,4-dibenzyl-meso-erythritol for the diol of step 10a, and carrying the product forward as in steps 10b and 10c, the title compound is prepared.

### 29b. Compound of Formula (III): R¹=OCH₃, R²=H; B=E=H; A=D=-CH₂-O-CH₂-phenyl

Following the procedures of Example 10 steps d-f, except substituting PhCH₂OCH₂CH(NH₂)CH(NH₂)CH₂OCH₂Ph, from the previous step, for the meso-2,3-butanediamine of step 10d, the title compound is prepared.

### Example 30

### Compound of Formula (III): R¹=OCH₃, R²=H; B=E=H: A=D=-CH₂-OH

Starting with the Compound of Formula (III): R¹=OCH3, R²=H; B=E=H; A=D=-CH₂-O-CH₂-phenyl, from Example 29 above, removing the benzyl groups by hydrogenation over Pd/C, the title compound is prepared.

### Example 31

### Compound of Formula (III): R¹=OCH₃, R²=H; B=E=H; A=D=-CH₂-O-phenyl

Starting with the Compound of Formula (III): R¹=OCH3, R²=H; B=E=H; A=D=-CH₂-OH, from Example 30 above, reacting the -CH₂-OH groups (of substituents A and D) with phenol, triphenylphosphine and DEAD under Mitsunobu conditions the title compound is prepared.

### Example 32

### Compound of Formula (III): R¹=OCH₃, R²=H; A=B=H; D and E taken together is -CH₂-CH₂-CH₂-CH₂-

Following the procedures of Example 3 except substituting 1-amino-1-cyclo-pentanemethanol (Aldrich) for the 2-(R)-amino-3-phenyl-1-propanol thereof, the title compound is obtained.

### Example 33

### Compound of Formula (III): R¹=OCH₃, R²=H; A and B taken together is -CH₂-CH₂-CH₂-CH₂-; D=E=H

Following the procedures of Example 5, except replacing the (S)-2-amino-3-phenyl-1-propanol of step 5a with 1-amino-1-cyclopentanemethanol (Aldrich), the title compound is obtained.

### Example 34

### Compound of Formula (III): R¹=OCH₃, R²=H; A=B=H; D and E taken together is -CH₂-O-CH₂-

### 34a. 3-amino-3-aminomethyloxetane

A sample of tris(hydroxymethyl)methylamine (Aldrich) is reacted with di-t-butyldicarbonate and triethylamine to give N-BOC-tris(hydroxymethyl)methylamine. This compound is reacted with 1 equivalent of methanesulfonyl chloride and triethylamine to give 3-(BOC-amino)-3-hydroxymethyloxetane. This compound is converted to the title compound according to the procedures of Example 3 steps b-e.

### 34b. Compound of Formula (III): R¹=OCH₃, R²=H; A=B=H; D and E taken together is -CH₂-O-CH₂-

Following the procedures of Example 3 steps f-h except substituting the compound from step 34a for the (R)-3-phenyl-1,2-propanediamine thereof, the title compound is obtained.

### Example 35

### Compound of Formula (III): R¹=OCH₃, R²=H; A=D=E=H; B=-CH₂-CH₂-phenyl

Following the procedures of Example 5, except replacing the (S)-2-amino-3-phenyl-1-propanol of step 5a with D-homophenylalaninol (prepared by LiAlH₄ reduction of the D-homophenylalanine available from Aldrich), the title compound is obtained.

### Example 36

### Compound of Formula (III): R¹=OCH₃, R²=H; A=D=E=H; B=-CH₂-CH₂-CH₂-phenyl

### 36a. 2-(R)-amino-5-phenylpentanoic acid

(±)-2-Amino-5-phenylpentanoic acid (35 g, from Example 40a) was suspended in water (3 L) and solubilized by adjusting the pH to 12 with 7 N NaOH solution. The pH was readjusted to pH 8 with 1 M phosphoric acid while stirring at 45°C. The solution was cooled to 40°C, and L-amino acid oxidase (Sigma, 0.7 unit/mg) was added. The reaction was stirred with good aeration for 2 weeks. The reaction mixture was concentrated to 500 mL under vacuum, the pH was adjusted to 5, and the precipitate was collected. The material was recrystallized from ethanol-water to afford 17.32 g of the title compound).

### 36b. 2-(R)-amino-5-phenylpentanol

The compound from step 36a is reduced with LiAlH₄ under standard conditions to give the title compound.

### 36c. Compound of Formula (III): R¹=OCH₃, R²=H; A=D=E=H; B=-CH₂-CH₂-CH₂-phenyl

Following the procedures of Example 5, except replacing the (S)-2-amino-3-phenyl-1-propanol of step 5a with the 2-(R)-amino-5-phenylpentanol from step 36b, the title compound is obtained.

### Example 37

### Compound of Formula (III): R¹=OCH₃, R²=H; A=D=E=H; B=-CH₂-O-CH₂-phenyl

### 37a. 1-N-(CBZ)-2-(S)-diamino-3-O-benzylpropane

N-BOC-O-benzyl-D-serine (Bachem) is reduced under the reaction conditions described by Kotokos, *Synthesis*, 299-301 (**1990**) to give the N-BOC-O-benzyl-D-serinol. This compound is treated according to the procedures of Example 10a-c to give the corresponding amine. The amine is converted to the benzyloxycarbonyl (CBZ) derivative, and the BOC group is removed to give the title compound.

### 37b. Compound of Formula (III): R¹=OCH₃, R²=H; A=D=E=H; B=-CH₂-O-CH₂-phenyl

Following the procedures of Example 10 steps d-f, except substituting the material from the previous step, for the meso-2,3-butanediamine of step 10d, the title compound is prepared.

### Example 38

### Compound of Formula (III): R¹=OCH₃, R²=H; A=D=E=H; B=-CH₂-CH₂-(4-OCH₃-phenyl)-

### 38a. D-homo-O-methyltyrosinol

D-Homo-O-methyltyrosine (prepared according to the procedure of Melillo *et al., J. Org. Chem*., 52:5149-5150 (**1987**)) is reduced with LiAlH₄ under standard conditions to give the title compound.

### 38b. Compound of Formula (III): R¹=OCH₃, R²=H; A=D=E=H; B=-CH₂-CH₂-(4-OCH₃-phenyl)

Following the procedures of Example 5, except replacing the (S)-2-amino-3-phenyl-1-propanol of step 5a with the compound of step 38a, the title compound is obtained

### Example 39

### Compound of Formula (III): R¹=OCH₃, R²=H; A=-CH₂-CH₂-phenyl; B=D=E=H

Following the procedures of Example 5, except substituting L-homophenylalaninol (prepared by LiAlH₄ reduction of the L-homophenylalanine (Aldrich) compound) for the (S)-2-amino-3-phenyl-1-propanol of step 5a , the title compound is obtained.

### Example 40

### Compound of Formula (III): R¹=OCH₃, R²=H; A=-CH₂-CH₂-CH₂-phenyl; B=D=E=H

### 40a. (±)-2-Amino-5-phenylpentanoic acid

Diethyl acetamidomalonate (220 g) in 1 L of absolute ethanol was added to a stirred solution of sodium ethoxide in ethanol, prepared by dissolving sodium (24 g) in absolute ethanol (500 mL), under nitrogen. The reaction mixture was refluxed under nitrogen for 30 minutes, then 1-bromo-3-phenylpropane (200g) was added. The reaction mixture was refluxed overnight, cooled to ambient temperature, filtered, and the solvent was removed under vacuum. Concentrated HCl (800 mL) was added to the residue, and the reaction mixture was refluxed for 14 hours. The cooled aqueous solution was washed with ether, and the residual ether in the aqueous phase was removed by bubbling nitrogen through the solution. The pH of the aqueous phase was adjusted to pH 7-8 by the addition of ammonium hydroxide. The title compound was collected by filtration, air dried and recrystallized from ethanol-water (150 g) m.p. 255-257°C. MS m/z: 194 (M+H)⁺.

### 40b. (±)-2-amino-5-phenylpentanol

The compound from step 40a is reduced with LiAlH₄ under standard conditions to give the title compound.

### 40c. Compound of Formula (III): R¹=OCH₃, R²=H; A=-CH₂-CH₂-CH₂-phenyl; B=D=E=H

Following the procedures of Example 5, except replacing the (S)-2-amino-3-phenyl-1-propanol of step 5a with the (±)- 2-amino-5-phenylpentanol from step 40b, and separating the isomers by chromatography the title compound is obtained.

### Example 41

### Compound of Formula (III): R¹=OCH₃, R²=H; A=-CH₂-O-CH₂-phenyl; B=D=E=H

Following the procedures of Example 37, except substituting the N-BOC-O-benzyl-L-serine (Sigma) for the N-BOC-O-benzyl-D-serine thereof the title compound is prepared.

### Example 42

### Compound of Formula (III): R¹=OCH₃, R²=H; B=D=E=H; A=-CH₂-CH₂-(4-OCH₃-phenyl)

### 42a. L-homo-O-methyltyrosinol

L-Homo-O-methyltyrosine (prepared according to the procedure of Melillo *et al*., *J. Org. Chem*., 52:5149-5150 (1987)) is reduced with LiAlH₄ under standard conditions to give the title compound.

### 42b. Compound of Formula (III): R¹=OCH₃, R²=H; B=D=E=H; A=-CH₂-CH₂-(4-OCH₃-phenyl)

Following the procedures of Example 5, except replacing the (S)-2-amino-3-phenyl-1-propanol of step 5a with the compound of step 42a, the title compound is obtained.

### Example 43

### Compound of Formula (III): R¹=OCH₃, R²=H; A=B=D=H; E=-CH₂CH₂Ph

Following the procedures of Example 3 except replacing the 2-(R)-amino-3-phenyl-1-propanol of step 3a thereof with L-homo-phenylalaninol (prepared by LiAlH₄ reduction under standard conditions of the L-homo-phenylalanine compound available from Aldrich) the title compound is prepared.

### Example 44

### Compound of Formula (III): R¹=OCH₃, R²=H; A=B=E=H; D=-CH₂CH₂Ph

Following the procedures of Example 3 except replacing the 2-(R)-amino-3-phenyl-1-propanol of step 3a thereof with D-homo-phenylalaninol (prepared by LiAlH₄ reduction under standard conditions of the D-homo-phenylalanine compound available from Aldrich) the title compound is prepared.

### Example 45

### Compound of Formula (III): R¹=OCH₃, R²=H; A=B=D=H; E=-CH₂CH₂CH₂Ph

Following the procedures of Example 3 except replacing the 2-(R)-amino-3-phenyl-1-propanol of step 3a thereof with (±)- 2-amino-5-phenylpentanol (prepared in Example 40b) and separating the desired isomer by chromatography the title compound is prepared.

### Example 46

### Compound of Formula (III): R¹=OCH₃, R²=H; A=B=E=H; D=-CH₂CH₂CH₂Ph

Following the procedures of Example 3 except replacing the 2-(R)-amino-3-phenyl-1-propanol of step 3a thereof with 2-(R)-amino-5-phenylpentanol (prepared in Example 36b) the title compound is prepared.

### Example 47

### Compound of Formula (III) R1=OCH₃, R2=H; A=-CH2CH2OPh; B=D=E=H

### 47a. N-α-Boc-L-homo-serine benzyl ester

The title compound is prepared from N-α-Boc-L-aspartic acid α-benzyl ester, ethyl chloroformate, N-methylmorpholine and sodium borohydride in tetrahydrofuran and methanol according to the procedures described by Kokotos, *Synthesis,* (1990): 299-301.

### 47b. 4-Phenoxy-2-(S)-Boc-aminobutyric acid benzyl ester

A solution of N-α-Boc-L-homo-serine benzyl ester, phenol and triphenylphosphine in THF is treated with diethylazodicarboxylate (DEAD) at 0 °C. After being stirred at reaction temperature for 2 hours, the reaction is worked up and product purified by silica gel chromatography. (cf. Organic Reactions, Vol. 42, John Wiley & Son, Inc, 1992).

### 47c. 4-phenoxy-2-(S)-Boc-aminobutane-1-ol

Lithium aluminum hydride is added into a stirred solution of 4-phenoxy-2-(S)-Boc-aminobutyric acid benzyl ester, from step 47b, in THF at 0 °C. The reaction mixture is heated to reflux for 0.5 hour and cooled to ice-cold temperature. Water, equal weight to the lithium aluminum hydride used, is added and stirred at reaction temperature overnight. The reaction is diluted with ethyl acetate and dried over sodium sulfate. After filtration and removal of solvent in vacuo, the product is purified by silica gel chromatography.

### 47d. 4-phenoxy-2-(S)-aminobutane-1-ol

The amino protecting group of 4-phenoxy-2-(S)-Boc-aminobutane-1-ol from step 47c is removed by treatment of the protected compound with hydrogen chloride in dioxane. (cf. T. W. Greene and P. G. M. Wuts: Protective Groups in Organic Synthesis. 2nd ed., John Wiley and Son, 1991, pp 309-315.)

### 47e. Compound (22) Scheme 7: R¹=OCH₃; R²=Bz; A=-CH2CH2-OPh; B=D=E=H; Y=OH

The title compound is prepared from 4-phenoxy-2-(S)-anzinobutane-1-ol and compound (8) in aqueous acetonitrile according to the procedures described in Example 5.

### 47f. Compound (20) Scheme 7: R¹=OCH₃; A=-CH2CH2-OPh; B=D=E=H

The title compound is prepared from the compound of step 47d according to the procedures described in Example 5.

### 47g. Compound of Formula (III) R1=OCH₃, R2=H; A=-CH2CH2OPh; B=D=E=H

The title compound of Example 47 is prepared from the compound of 47f according to the procedures described in Example 5.

### Example 48

### Compound of Formula (III) R1=OCH₃, R2=H; A=-CH2CH2NHCbz; B=D=E=H

### 48a. N-α-(S)-Boc-N-γ-Cbz-2,4-diaminobutyric acid benzyl ester

Diphenylphosphoryl azide is added into a solution of N-α-(S)-Boc-L-glutamic acid α -benzyl ester in THF and the resulting solution is refluxed for 2 hours. Benzyl alcohol is added and the reaction mixture is refluxed for an additional hour. The product is purified by silica gel chromatography.

### 48b. N-α-(S)-Boc -N-γ-Cbz-2,4-diamino-butane-1-ol

Lithium aluminum hydride is added into a stirred solution of N-α-(S)-Boc -N-γ-Cbz-diaminobutyric acid benzyl ester, from step 48a, in THF at 0 °C. The reaction mixture is heated to reflux for 0.5 hour and cooled to ice-cold temperature. Water, equal weight to the lithium aluminum hydride used, is added and stirred at reaction temperature overnight. The reaction is diluted with ethyl acetate and dried over sodium sulfate. After filtration and removal of solvent in vacuo, the product is purified by silica gel chromatography.

### 48c. 2-(S)-4-N-Cbz-diamino-1-butanol

The amino protecting group of N-α-(S)-Boc -N-γ-Cbz-2,4-diamino-butane-1-ol from step 48b is removed by treatment of the protected compound with hydrogen chloride in dioxane according to literature method. (cf. T. W. Greene and P. G. M. Wuts: Protective Groups in Organic Synthesis. 2nd ed., John Wiley and Son, 1991, pp 309-315.)

### 48d. Compound (22) Scheme 7 : R¹=OCH₃; R²=Bz; A=-CH2CH2-NHCbz; B=D=E=H; Y=OH

The title compound is prepared from 2-(S)-amino-4-N-γ-Cbz-diamino-butane-1-ol, from step 48c, and compound (8) from Scheme 2 in aqueous acetonitrile according to the procedures described in Example 5.

### 48e. Compound (20) Scheme 7: R¹=OCH₃; A=-CH2CH2-NHCbz; B=D=E=H

The title compound is prepared from the compound of step 48d according to the procedures described in Example 5.

### 48f. Compound of Formula (III) R1=OCH₃, R2=H; A=-CH2CH2NHCbz; B=D=E=H

The title compound is prepared from the compound of step 48e according to the procedures described in Example 5.

### Example 48-A

### Compound of Formula (III) R1=OCH₃, R2=H; A=-CH2CH2NH2; B=D=E=H

The title compound is prepared from the compound of Example 48 and hydrogen in the presence of Pd-C in ethanol.

### Example 49

### Compound of Formula (III) R1=OCH₃, R2=H; A=-CH2CO2Bzl; B=D=E=H

### 49a. 4-hydroxy-3-N-(S)-Boc-aminobutyric acid benzyl ester

The title compound is prepared from N-α-Boc-L-aspartic acid γ-benzyl ester, ethyl chloroformate, N-methylmorpholine and sodium borohydride in tetrahydrofuran and methanol according to the procedures described by Kokotos, *Synthesis*, (1990): 299-301.

### 49b. 4-hydroxy-3-(S)-aminobutyric acid benzyl ester

The amino protecting group of 4-hydroxy-3-N-(S)-Boc-aminobutyric acid benzyl ester, from step 49a, is removed by treatment of the protected compound with hydrogen chloride in dioxane according to literature method.(cf. T. W. Greene and P. G. M. Wuts: Protective Groups in Organic Synthesis. 2nd ed., John Wiley and Son, 1991, pp 309-315.)

### 49c. Compound (22) Scheme 7 : R¹=OCH₃; R²=Bz; A=-CH2CO2Bzl; B=D=E=H; Y=OH

The title compound is prepared from 4-hydroxy-3-(S)-aminobutyric acid benzyl ester, from step 49b and compound (8) of Scheme 2 in aqueous acetonitrile according to the procedures described in Example 5.

### 49d. Compound (20) Scheme 7 : R¹=OCH₃; A=-CH2CO2Bzl; B=D=E=H

The title compound is prepared from the compound of step 49c according to the procedures described in Example 5.

### 49e. Compound of Formula (III) R1=OCH₃, R2=H; A=-CH2CO2Bzl; B=D=E=H

The title compound is prepared from compound from the compound of step 49d according to the procedures described in Example 5.

### Example 49-A

### Compound of Formula (III) R1=OCH₃, R2=H; A=-CH2COOH; B=D=E=H

The title compound is prepared from the compound of Example 49 and hydrogen in the presence of Pd-C in ethanol.

### Example 49-B

### Formula (III) R1=OCH₃, R2=H; A=-CH2CH2OH; B=D=E=H

The title compound is prepared from the title compound of Example 49-A, ethyl chloroformate, N-methylmorpholine and sodium borohydride in tetrahydrofuran and methanol according to the procedures described by Kokotos, *Synthesis,* (1990): 299-301.

### Example 50

### Compound of Formula (III) R1=OCH₃, R2=H; A=-CH2CH2NH(4'-Pyridyl); B=D=E=H

The title compound is prepared from the compound of Example 48-A, 4-chloropyridine, and CuO or CuBr-K₂CO₃ heated at 70-90 °C overnight. The reaction mixture is partitioned between ethyl acetate and water. The organic phase is washed once with dilute hydrochloric acid followed by saturated sodium bicarbonate. The product is purified by silica gel chromatography.

### Example 51

### Compound of Formula (III): R¹=OCH₃, R²=H; A=B=D=H; E=-CH₂OH

### 51a. 1,2-diamino-3-propanol

Allyl acetate (Aldrich) is reacted with manganese triacetate, sodium azide and acetic acid. The resulting compound is treated with NaHCO₃ and methanol followed by hydrogenation to give the title compound.

### 51b. Compound of Formula (III): R¹=OCH₃, R²=H; A=B=D=H; E=-CH₂OH

Following the procedures of Example 3 steps f-h, except substituting 1,2-diamino-3-propanol from the previous step, for the 1, 2-(R)-diamino-3-phenylpropane thereof, the title compound is prepared, and the diastereomers are separated by chromatography.

### Example 52

### Compound of Formula (III): R¹=OCH₃, R²=H; A=B=E=H; D=-CH₂OH

The compound is separated from the diastereomeric mixture of Example 51b by chromatography.

### Example 53

### Compound of Formula (III): R¹=OCH₃, R²=H; A=B=E=H; D=-CH₂NHBenzoyl

The compound is prepared from the compound of Example 16 by treatment with benzoyl chloride and TEA.

### Example 54

### Compound of Formula (III): R¹=OCH₃, R²=H; A=B=E=H; D=-CH₂NHBenzyl

The compound is prepared from the compound of Example 16 by treatment with benzaldehyde, NaCNBH₃ and acetic acid in methanol at reaction temperature.

### Example 55

### Compound of Formula (III): R¹=OCH₃, R²=H; A=B=D=H; E=-CH₂NHBenzoyl

The compound is prepared from the compound of Example 15 by treatment with benzoyl chloride and TEA.

### Example 56

### Compound of Formula (III): R¹=OCH₃, R²=H; A=B=D=H; E=-CH₂NHBenzyl

The compound is prepared from the compound of Example 15 by treatment with benzaldehyde, NaCNBH₃ and acetic acid in methanol at reaction temperature.

### Example 57

### Compound of Formula (III): R¹=OCH₃, R²=H; B=D=H; A=E=-CH₂OCH₂(4-Cl-phenyl-)

### 57a. (R,R)-(+)-1,4-bis-O-(4-chlorobenzyl)-2,3-butanediamine

Following the procedure of Example 10 steps a-c, replacing the meso-2,3-butanediol thereof with (R,R)-(+)-1,4- bis-O-(4-chlorobenzyl)-D-threital (Aldrich) the title compound is obtained.

### 57b. Compound of Formula (III): R¹=OCH₃, R²=H; B=D=H; A=E=-CH₂OCH₂(4-Cl-phenyl-)

Following the procedures of Example 10 steps d-f, replacing the meso-2,3-butanediamine of step d thereof with the diamine from step 57a, the title compound is prepared.

### Example 58

### Compound of Formula (III): R¹=OCH3, R²=H; A=B=E=H; D=-CH₂-N(CH₃)-Benzyl

The title compound is prepared by treating the compound of Example 54 with HCHO, NaCNBH₃ and acetic acid in methanol at reaction temperature.

### Example 59

### Compound of Formula (III): R¹=OCH3, R²=H; A=B=D=H; E=-CH₂-N(CH₃)-Benzyl

The title compound is prepared by treating the compound of Example 56 with HCHO, NaCNBH₃ and acetic acid in methanol at reaction temperature.

### Example 60

### Compound of Formula (III): R¹=OCH3, R²=H; A=B=D=H; E=-CH₂-NH-phenyl

The title compound is prepared by treating the compound of Example 51 with triphenyl phosphine, DEAD and aniline under Mitsunobu conditions.

### Example 61

### Compound of Formula (III): R¹=OCH3, R²=H; A=B=E=H; D=-CH₂-NH-phenyl

The title compound is prepared by treating the compound of Example 52 with triphenyl phosphine, DEAD and aniline under Mitsunobu conditions.

### Example 62

### In Vitro Assay of Antibacterial Activity

Representative compounds of the present invention were assayed *in vitro* for antibacterial activity as follows: Twelve petri dishes containing successive aqueous dilutions of the test compound mixed with 10 mL of sterilized Brain Heart Infusion (BHI) agar (Difco 0418-01-5) were prepared. Each plate was inoculated with 1:100 (or 1:10 for slow-growing strains, such as *Micrococcus* and *Streptococcus*) dilutions of up to 32 different microorganisms, using a Steers replicator block. The inoculated plates were incubated at 35-37 °C for 20 to 24 hours. In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was visually inspected. The minimum inhibitory concentration (MIC) was defined as the lowest concentration of drug yielding no growth, a slight haze, or sparsely isolated colonies on the inoculum spot as compared to the growth control. The results of this assay, shown below in Table 2 demonstrate the antibacterial activity of the compounds of the invention.

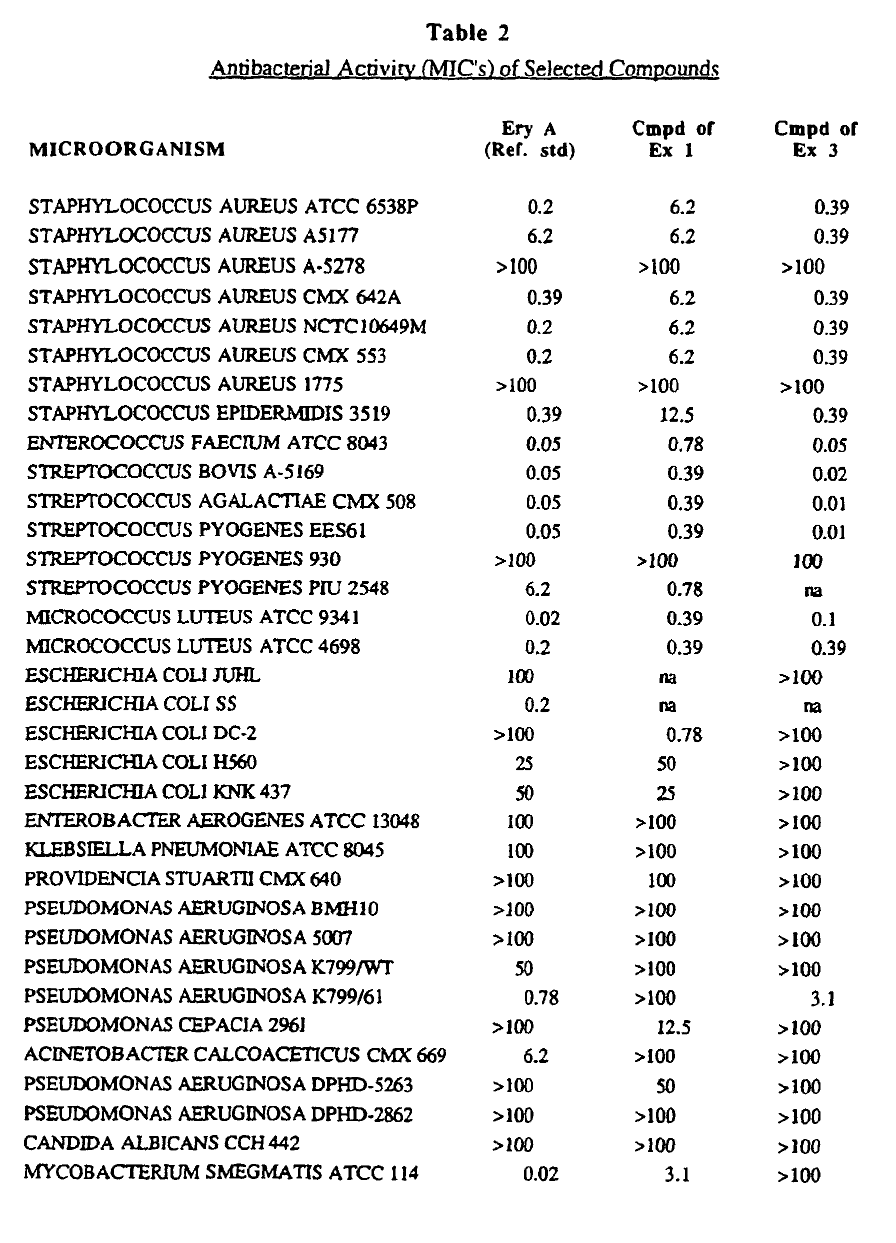

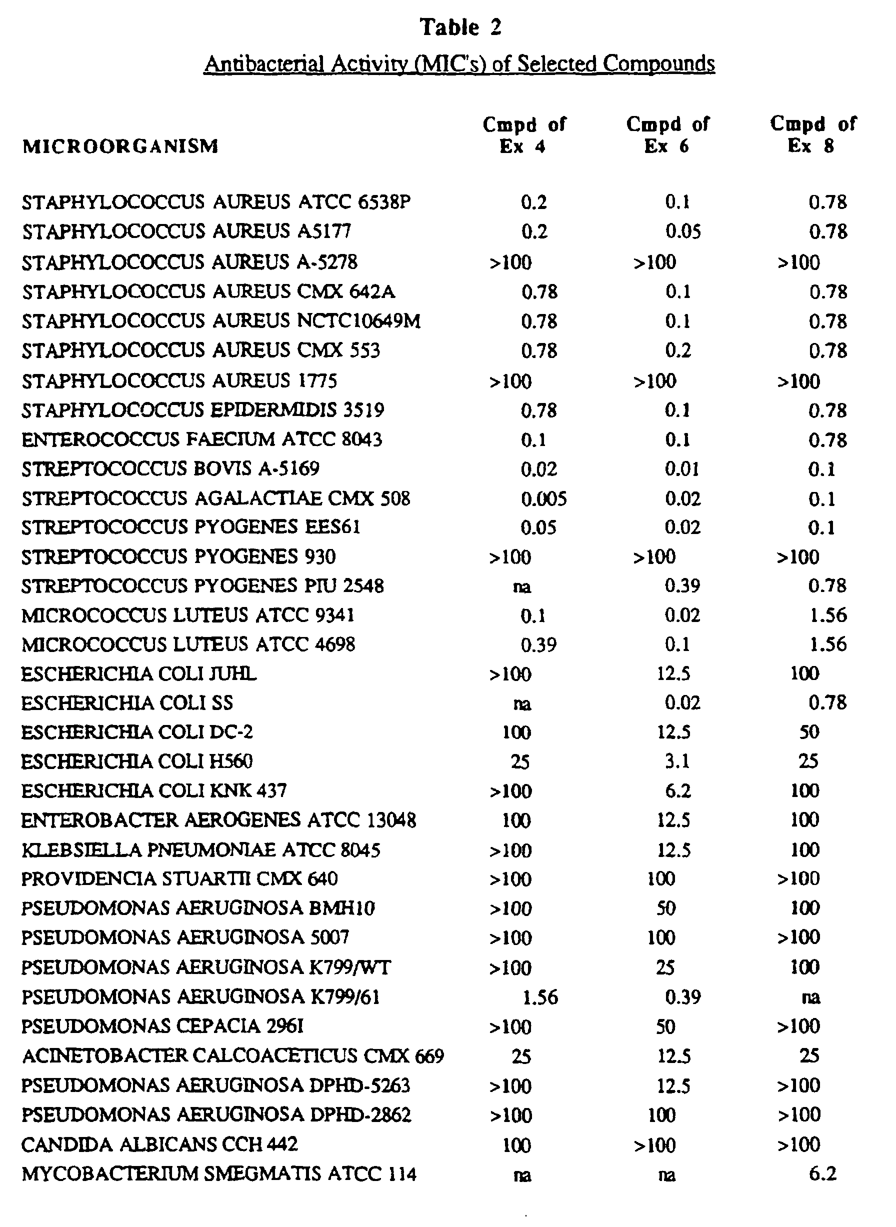

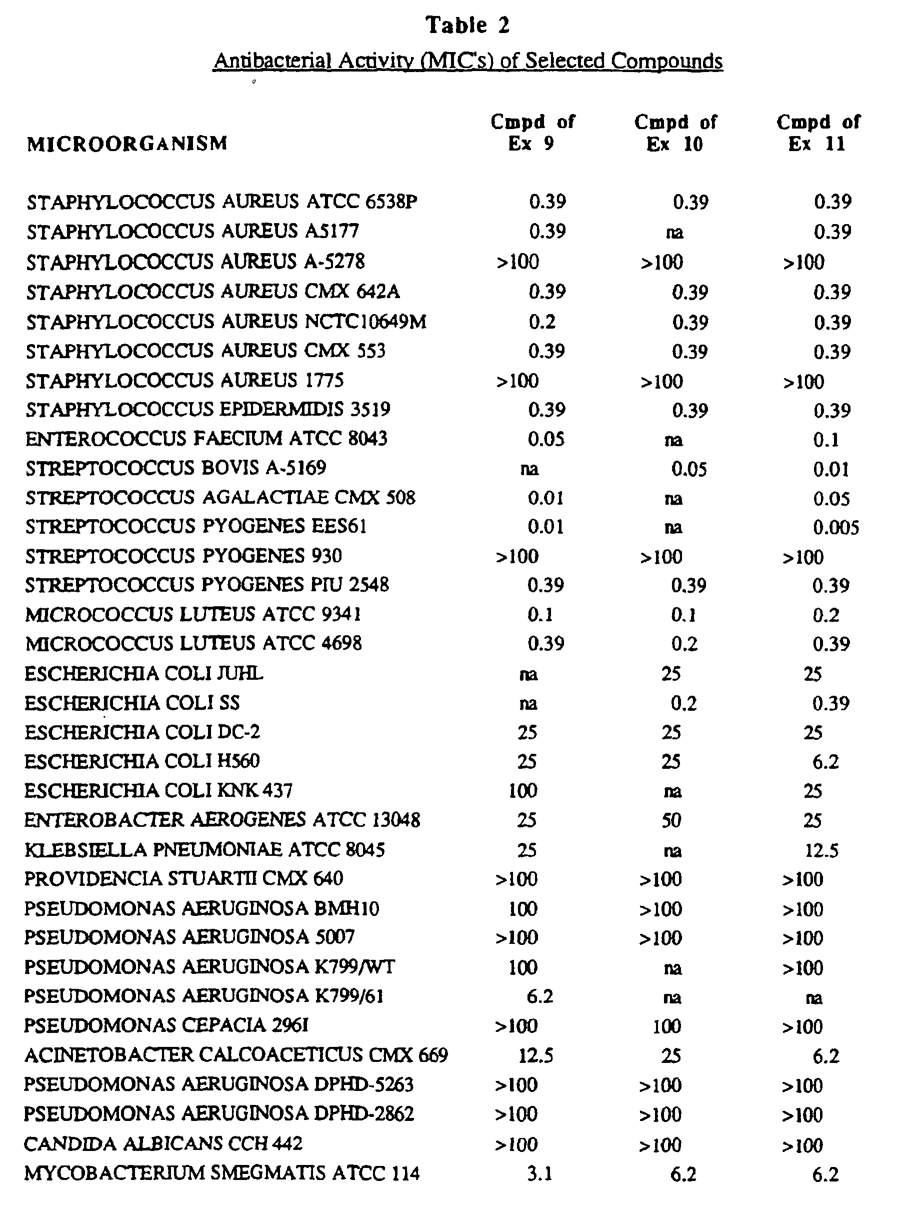

## Claims

1. A compound selected from the group having the formulas: 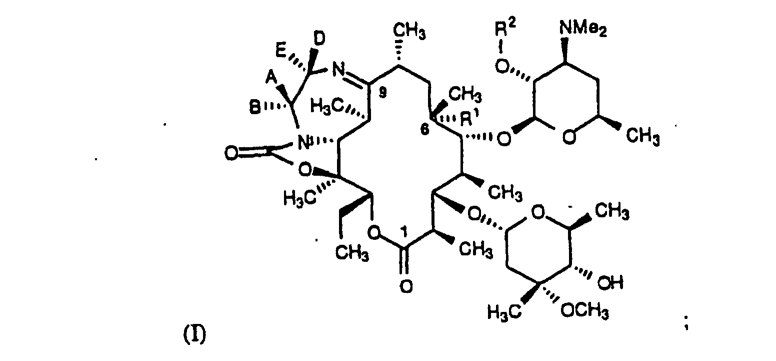 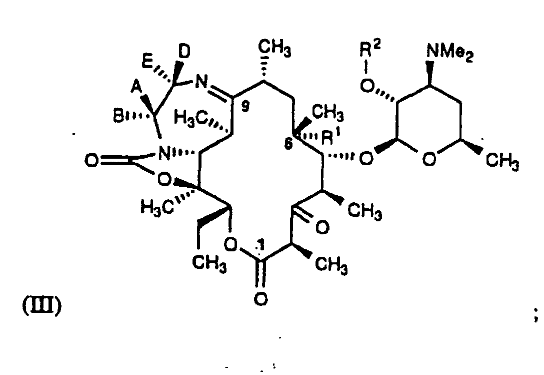 and 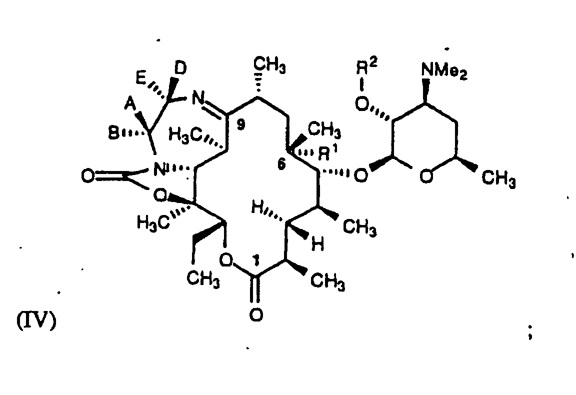 as well as the pharmaceutically acceptable salts and esters thereof wherein:
A, B, D, and E are independently selected from the group consisting of:
(a) hydrogen;
(b) C₁-C₆-alkyl, unsubstituted or substituted with one substituent selected from the group consisting of:
(i) phenyl;
(ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
(iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
(v) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
(vi) hydroxy;
(vii) C₁-C₆ alkoxy;
(viii) halogen consisting of Br, Cl, F or I; and
(ix) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, - N(phenyl-C₁-C₆-alkyl-)-, -N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
(c) C₃-C₇-cycloalkyl;
(d) phenyl;
(e) substituted-phenyl as defined above;
(f) heteroaryl as defined above;
(g) substituted-heteroaryl as defined above;
(h) heterocycloalkyl as defined above;
and
(i) a group selected from option (b) above in which the substituent is
-M-R⁵, wherein M is selected from the group consisting of:
(aa) -C(O)-NH-;
(bb) -NH-C(O)-;
(cc) -NH-
(dd) -N(CH₃) -
(ee) -O-;
(ff) -S(O)ₙ-, wherein n is 0, 1 or 2; and
(gg) -C(=NH)-NH-;
and R⁵ is selected from the group consisting of:
(aaa) C₁-C₆-alkyl, optionally substituted with a substituent selected from the group consisting of:
(i) phenyl;
(ii) substituted-phenyl as defined above;
(iii) heteroaryl as defined above;
(iv) substituted-heteroaryl as defined above;
(bbb) phenyl;
(ccc) substituted-phenyl as defined above;
(ddd) heteroaryl as defined above;
(eee) substituted-heteroaryl as defined above; and
(fff) heterocycloalkyl as defined above;
or
any one pair of substituents, consisting of AB, AD, AE, BD, BE, or DE, is taken together with the atom or atoms to which they are attached to form a 3- to 7-membered ring optionally containing a hetero function consisting of
-O-,
- NH-,
- N(C₁-C₆-alkyl-)-,
- N(phenyl-C₁-C₆-alkyl-)-,
- N(substituted-phenyl-C₁-C₆-alkyl-)-
wherein substituted-phenyl is as defined above,
- N(heteroaryl-C₁-C₆-alkyl-)-
wherein heteroaryl is as defined above,
- N(substituted-heteroary)-C₁-C₆-alkyl-)-
wherein substituted-heteroaryl is as defined above,
- S-, or -S(O)ₙ-,
wherein n is 1 or 2;
-C(O)-NH-;
- C(O)-NR⁵-,
wherein R⁵ is as defined above;
-NH-C(O)-;
- NR⁵-C(O)-,
wherein R⁵ is as defined above; and
- C(=NH)-NH-;
R¹ is -O-C₁-C₃-alkyl; and
R² is hydrogen;
with the provisos that when the compound is of Formulas (I) or (III) then A, B, D, and E may not all be hydrogen, D and E may not be C₁-C₃-alkyl when A and B are hydrogen, nor may one of D and E be hydrogen and the other be C₁-C₃-alkyl when A and B are hydrogen;
or a compound of Formula (III) in which R¹ = OCH₃, R² is H, B = E = H, and A and D taken together is either -CH₂-N(Cbz)-CH₂- or -CH₂-N(benzoyl)-CH₂-;
or a compound of Formula (III) in which B=E=H, R¹ is methoxy, R² is H; and A and D together is CH₂-N(R⁷)-CH₂-, wherein R⁷ is selected from the group consisting of:
(i) phenyl;
(ii) substituted-phenyl as defined above;
(iii) heteroaryl as defined above; and
(iv) substituted-heteroaryl as defined above;
or a compound of Formula (III) in which B=E=H, R¹ is methoxy, R² is H; and A and D together is CH₂-N(-(CH₂)ᵣ-M-(CH₂)ₛ-R⁷)-CH₂-, wherein r is 0, 1 or 2; s = 0, 1 or 2, M is as defined above; and R⁷ is selected from the group consisting of:
(i) C₁-C₃-alkyl;
(ii) phenyl;
(iii) substituted-phenyl as defined above;
(iv) heteroaryl as defined above; and
(v) substituted-heteroaryl as defined above.

2. A compound according to Claim 1 having the formula: 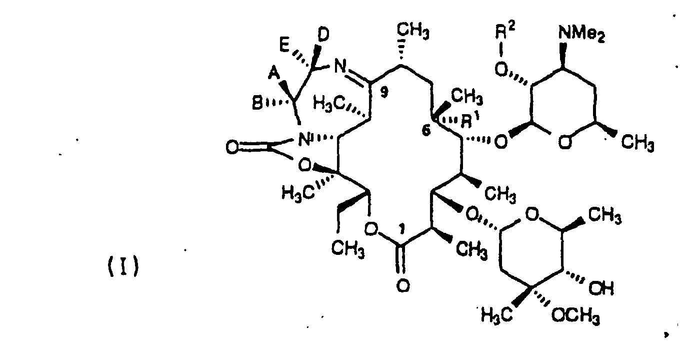 wherein A, B, D, E, and R¹-R⁵ are as described therein.

3. A compound according to Claim 1 having the formula: 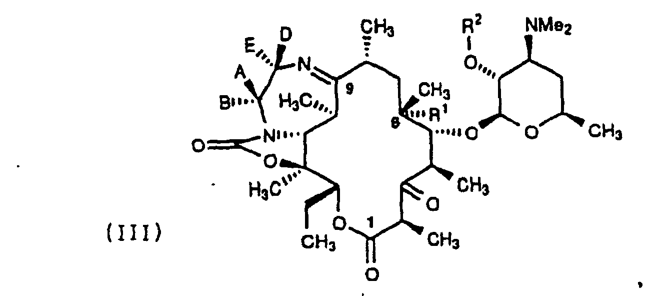 wherein A, B, D, E, and R¹-R⁵ are as described therein.

4. A compound according to Claim 1 having the formula: 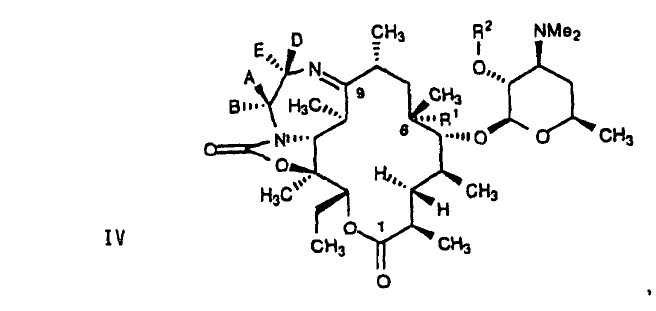 wherein A, B, D, E, and R¹-R⁵ are as described therein.

5. A compound according to Claim 3 wherein R¹ is methoxy, and A, B, D, E and R¹-R⁵ are as described therein.

6. A compound according to Claim 5 wherein any three of the A, B, D and E groups are hydrogen and the other group is selected from a singly substituted C₁-C₆-alkyl group comprised of -(CH₂)ₘR⁶ where m = 1, 2, 3 or 4 and R⁶ is selected from the group consisting of:
(a) phenyl;
(b) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(c) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
(d) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
(e) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
(f) hydroxy;
(g) C₁-C₆ alkoxy;
(h) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, - N(phenyl-C₁-C₆-alkyl-)-, - N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
(i) halogen consisting of Br, Cl, F or I;
(j) C₁-C₃-alkyl; or
(k) (CH₂)ᵣ-M-(CH₂)ₛ-R⁷ wherein r = 0, 1, or 2; s = 0, 1 or 2 and M is selected from the group consisting of:
(aa) -C(O)-NH-;
(bb) -NH-C(O)-;
(cc) -NH-
(dd) -N(CH₃)-
(ee) -O-;
(ff) -S(O)ₙ-, wherein n is 0, 1 or 2; and
(gg) -C(=NH)-NH-;
and R⁷ is selected from the group consisting of:
(aaa) C₁-C₃-alkyl;
(bbb) phenyl;
(ccc) substituted-phenyl as defined above;
(ddd) heteroaryl as defined above; and
(eee) substituted-heteroaryl as defined above.

7. A compound according to Claim 5 wherein B = E = H, and A and D together is selected from the group consisting of:
(a) -CH₂-Z-CH₂-, wherein Z is selected from the group consisting of :
(aa) -C(O)-NH-;
(bb) -C(O)-NR⁵-, wherein R⁵ is selected from the group consisting of:
(aaa) C₁-C₆ alkyl, unsubstituted or substituted with a substituent selected from the group consisting of:
(i) phenyl;
(ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl; and
(iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(bbb) phenyl;
(ccc) substituted-phenyl as defined above;
(ddd) heteroaryl as defined above;
(eee) substituted-heteroaryl as defined above; and
(fff) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
(cc) -NH-C(O)-;
(dd) -NR⁵-C(O)-, wherein R⁵ is as defined above
(ee) -NH-;
(ff) -N(CH₃)-;
(gg) -O-;
(hh) -S(O)ₙ-, wherein n is 0, 1 or 2; and
(ii) -C(=NH)-NH-;
(b) -CH₂-N(-(CH₂)ₛ-R⁷)-CH₂-, wherein s = 0, 1 or 2, and R⁷ is selected from the group consisting of:
(aa) C₁-C₃-alkyl;
(bb) phenyl;
(cc) substituted-phenyl as defined above;
(dd) heteroaryl as defined above; and
(ee) substituted-heteroaryl as defined above;
(c) -CH₂-N(-(CH₂)ᵣ-M-(CH-₂)ₛ-R⁷)-CH₂-, wherein r is 0, 1 or 2; s = 0, 1 or 2, R⁷ is as defined above and M is selected from the group consisting of:
(i) -C(O)-NH-;
(ii) -NH-C(O)-;
(iii) -NH-
(iv) -N(CH₃)-
(v) -O-;
(vi) -S(O)ₙ-, wherein n is 0, 1 or 2; and
(vii) -C(=NH)-NH-; and
(d) -CH₂-(CH₂)ᵣ-CH₂-, wherein r = 0, 1 or 2.

8. A compound according to Claim 1 which is:
Compound of Formula (IV); R¹=methoxy; R²=hydrogen; A=B=D=E=hydrogen;
Compound of Formula (III), A=B=E=H, D=benzyl, R¹=methoxy, R²=hydrogen;
Compound of Formula (III), A=B=D=H, E=benzyl, R¹=methoxy, R²=hydrogen;
Compound of Formula (III); A=benzyl, B=D=E=H, R¹=methoxy, R²=hydrogen;
Compound of Formula (III); B=benzyl, A=D=E=H, R¹=methoxy, R²=hydrogen;
Compound of Formula (III); A=E=phenyl, B=D=H, R¹=methoxy, R²=hydrogen;
Compound of Formula (III); A=methyl, B=D=E=H, R¹=methoxy, R²=hydrogen;
Compound of Formula (III); B=methyl, A=D=E=H, R¹=methoxy, R²=hydrogen;
Compound of Formula (III); A=D=methyl; B=E=H, R¹=methoxy, R²=hydrogen;
Compound of Formula (III); A=E=methyl; B=D=H, R¹=methoxy, R²=hydrogen ;
Compound of Formula (III); B=D=H; A and E taken together is -CH₂CH₂CH₂-, R¹=methoxy, R²=hydrogen;
Compound of Formula (III); B=E=H; A and D taken together is -CH₂CH₂CH₂CH₂-, R¹=methoxy, R²=hydrogen ;
Compound of Formula (III): R¹=OCH3, R²=H; A=B=D=H; E=-CH₂NH₂;
Compound of Formula (III): R¹=OCH3, R²=H; A=B=E=H; D=-CH₂NH₂:
Compound of Formula (III): R¹=OCH3, R²=H; B=E=H; A and D taken together is - CH₂CH₂CH₂-;
Compound of Formula (III): R¹=OCH3, R²=H; B=E=H; A and D taken together is - CH₂OCH₂-;
Compound of Formula (III): R¹=OCH3, R²=H; B=E=H; A and D taken together is -CH₂-NH-CH₂-;
Compound of Formula (III): R¹=OCH3, R²=H; B=E=H; A and D taken together is -CH₂-N(Cbz)-CH₂-;
Compound of Formula (III): R¹=OCH3, R²=H; B=E=H; A and D taken together is - CH₂-N(benzyl)-CH₂-;
Compound of Formula (III): R¹=OCH3, R²=H; B=E=H; A and D taken together is - CH₂-N(benzoyl)-CH₂-;
Compound of Formula (III): R¹=OCH3, R²=H; B=E=H; A and D taken together is - CH₂-N(phenyl-CH₂-CH₂-)-CH₂-;
Compound of Formula (III): R¹=OCH3, R²=H; B=E=H; A and D taken together is - CH₂-N(4-Cl-phenyl-CH₂-)-CH₂-;
Compound of Formula (III): R¹=OCH3, R²=H; B=E=H; A and D taken together is - CH₂-N(4-pyridyl-CH₂-)-CH₂-;
Compound of Formula (III): R¹=OCH3, R²=H; B=E=H; A and D taken together is - CH₂-N(2-pyridyl-CH₂-)-CH₂-;
Compound of Formula (III): R¹=OCH3, R²=H; B=E=H; A and D taken together is - CH₂-N(3-pyridyl-CH₂-)-CH₂-;
Compound of Formula (III): R¹=OCH3, R²=H; B=E=H; A and D taken together is - CH₂-N(4-quinolyl-CH₂-)-CH₂-;
Compound of Formula (III): R¹=OCH3, R²=H; B=E=H; A=D=-CH₂-O-CH₂-phenyl;
Compound of Formula (III): R¹=OCH3, R²=H; B=E=H; A=D-CH₂-OH;
Compound of Formula (III): R¹=OCH3, R²=H; B=E=H; A=D=-CH₂-O-phenyl;
Compound of Formula (III): R¹=OCH3, R²=H; A=B=H; D and E taken together is - CH₂-CH₂-CH₂-CH₂-;
Compound of Formula (III): R¹=OCH3, R²=H; A and B taken together is - CH₂-CH₂-CH₂-CH₂-; D=E=H;
Compound of Formula (III): R¹=OCH3, R²=H; A=B=H; D and E taken together is - CH₂-O-CH₂-;
Compound of Formula (III): R¹=OCH3, R²=H; A=D=E=H; B=-CH₂-CH₂-phenyl ;
Compound of Formula (III): R¹=OCH3, R²=H; A=D=E=H; B=-CH₂-CH₂-CH₂-phenyl ;
Compound of Formula (III): R¹=OCH3, R²=H; A=D=E=H; B=-CH₂-O-CH₂-phenyl ;
Compound of Formula (III): R¹=OCH3, R²=H; A=D=E=H; B=-CH₂-CH₂-(4-OCH₃-phenyl);
Compound of Formula (III): R¹=OCH3, R²=H; A=-CH₂-CH₂-phenyl; B=D=E=H ;
Compound of Formula (III): R¹=OCH3, R²=H; A=-CH₂-CH₂-CH₂-phenyl; B=D=E=H ;
Compound of Formula (III): R¹=OCH3, R²=H; A=CH₂-O-CH₂-phenyl ; B=D=E=H ;
Compound of Formula (III): R¹=OCH3, R²=H; B=D=E=H; A=-CH₂-CH₂-(4-OCH₃-phenyl);
Compound of Formula (III): R¹=OCH3, R²=H; A=B=D=H; E=-CH₂-CH₂-Ph;
Compound of Formula (III): R¹=OCH3, R²=H; A=B=E=H; D=-CH₂-CH₂-Ph;
Compound of Formula (III): R¹=OCH3, R²=H; A=B=D=H; E=-CH₂-CH₂-CH₂-Ph;
Compound of Formula (III): R¹=OCH3, R²=H; A=B=E=H; D=-CH₂-CH₂-CH₂-Ph;
Compound of Formula (III): R¹=OCH3, R²=H; A=- CH₂-CH₂-OPh; B=D=E=H;
Compound of Formula (III) R1=OCH3, R2=H; A=-CH₂-CH₂-NH₂; B=D=E=H;
Compound of Formula (III) R1=OCH3, R2=H; A=-CH₂-CH₂-OH; B=D=E=H;
Compound of Formula (III): R¹=OCH3, R²=H; A=- CH₂-CH₂-NH(4-Pyridyl-); B=D=E=H
Compound of Formula (III): R¹=OCH3, R²=H; A=B=D=H; E=-CH₂-OH;
Compound of Formula (III): R¹=OCH3, R²=H; A=B=E=H; D=-CH₂-OH;
Compound of Formula (III): R¹=OCH3, R²=H; A=B=E=H; D=-CH₂-NHBenzoyl;
Compound of Formula (III): R¹=OCH3, R²=H; A=B=E=H; D=-CH₂-NHBenzyl;
Compound of Formula (III): R¹=OCH3, R²=H; A=B=D=H; E=-CH₂-NHBenzoyl;
Compound of Formula (III): R¹=OCH3, R²=H; A=B=D=H; E=-CH₂-NHBenzyl;
Compound of Formula (III): R¹=OCH3, R²=H; B=D=H; A=E=-CH₂OCH₂(4-Cl-phenyl);
Compound of Formula (III): R¹=OCH3, R²=H; A=B=E=H; D=-CH₂-N(CH₃)-Benzyl;
Compound of Formula (III): R¹=OCH3, R²=H; A=B=D=H; E=-CH₂-N(CH₃)-Benzyl;
Compound of Formula (III): R¹=OCH3, R²=H; A=B=D=H: E=-CH₂-NH-phenyl;
or
Compound of Formula (III): R¹=OCH3, R²=H; A=B=E=H; D=-CH₂-NH-phenyl.

9. A compound according to Claim 1 which is:
Compound of Formula (IV); R¹=methoxy; R²=hydrogen; A=B=D=E=hydrogen;
Compound of Formula (III), A=B=E=H, D=benzyl, R¹=methoxy, R²=hydrogen;
Compound of Formula (III), A=B=D=H, E=benzyl, R¹=methaxy, R²=hydrogen;
Compound of Formula (III); A=benzyl, B=D=E=H, R1=methoxy, R2=hydrogen;
Compound of Formula (III); B=benzyl, A=D=E=H, R¹=methoxy, R²=hydrogen;
Compound of Formula (III); A=E=phenyl, B=D=H, R¹=methoxy, R²=hydrogen;
Compound of Formula (III); A=methyl, B=D=E=H, R¹=methoxy, R²=hydrogen;
Compound of Formula (III); B=methyl, A=D=E=H, R¹=methoxy, R²=hydrogen;
Compound of Formula (III); A=D=methyl; B=E=H, R¹=methoxy, R²=hydrogen;
Compound of Formula (III); A=E=methyl; B=D=H, R¹=methoxy, R²=hydrogen ;
Compound of Formula (III); B=D=H; A and E taken together is -CH₂CH₂CH₂-, R¹=methoxy, R²=hydrogen; or
Compound of Formula (III); B=E=H; A and D taken together is -CH₂CH₂CH₂CH₂-, R¹=methoxy, R²=hydrogen.

10. A pharmaceutical composition comprising a therapeutically effective amount of a compound according to any of Claims 1 through 9 in combination with a pharmaceutically acceptable carrier.

11. Use of a pharmaceutical compound according to any of Claims 1 through 9 for preparing a medicament for controlling a bacterial infection in a mammal.

12. A process for the preparation of tricyclic macrolide compounds having the Formulas: 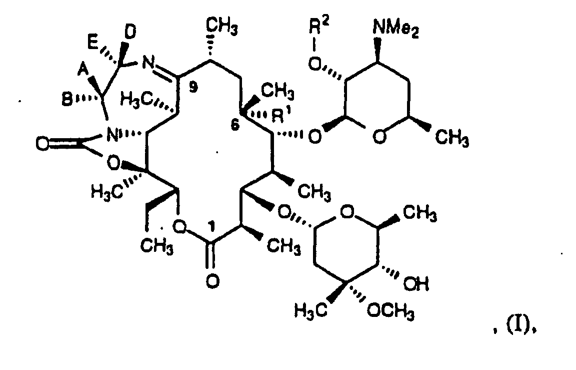 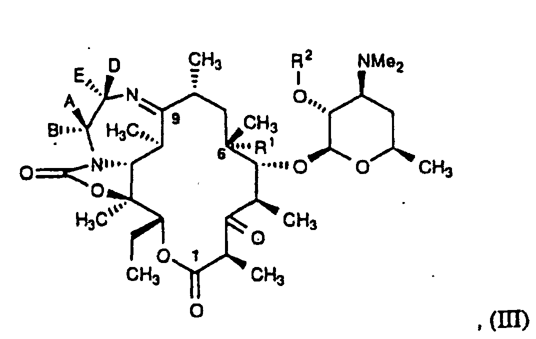 or 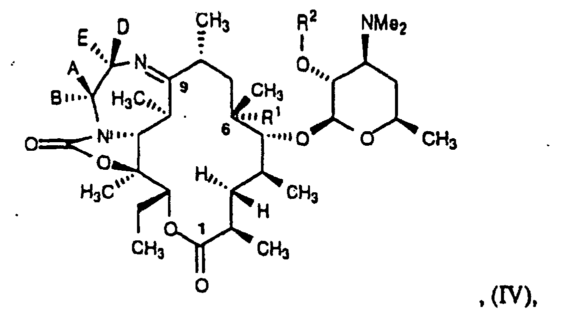 wherein
A, B, D and E are independently selected from the group consisting of:
(a) hydrogen;
(b) C₁-C₆-alkyl, unsubstituted or substituted with one substituent selected from the group consisting of:
(i) phenyl;
(ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
(iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
(v) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
(vi) hydroxy;
(vii) C₁-C₆ alkoxy;
(viii) halogen consisting of Br, Cl, F or I; and
(ix) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(phenyl-C₁-C₆-alkyl-)-, -N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
(c) C₃-C₇-cycloalkyl;
(d) phenyl;
(e) substituted-phenyl as defined above;
(f) heteroaryl as defined above;
(g) substituted-heteroaryl as defined above;
(h) heterocycloalkyl as defined above;
and
(i) a group selected from option (b) above in which the substituent is - M-R⁵, wherein M is selected from the group consisting of:
(aa) -C(O)-NH-;
(bb) -NH-C(O)-;
(cc) -NH-
(dd) -N(CH₃)-
(ee) -O-;
(ff) -S(O)ₙ-, wherein n is 0, 1 or 2; and
(gg) -C(=NH)-NH-;
and R⁵ is selected from the group consisting of:
(aaa) C₁-C₆-alkyl, optionally substituted with a substituent selected from the group consisting of:
(i) phenyl;
(ii) substituted-phenyl as defined above;
(iii) heteroaryl as defined above;
(iv) substituted-heteroaryl as defined above;
(bbb) phenyl;
(ccc) substituted-phenyl as defined above;
(ddd) heteroaryl as defined above;
(eee) substituted-heteroaryl as defined above; and
(fff) heterocycloalkyl as defined above;
or
any one pair of substituents, consisting of AB, AD, AE, BD, BE, or DE, is taken together with the atom or atoms to which they are attached to form a 3- to 7-membered ring optionally containing a hetero function consisting of
-O-,
-NH-,
-N(C₁-C₆-alkyl-)-,
- N(phenyl-C₁-C₆-alkyl-)-,
- N(substituted-phenyl-C₁-C₆-alkyl-)-
wherein substituted-phenyl is as defined above,
-N(heteroaryl-C₁-C₆-alkyl-)-
wherein heteroaryl is as defined above,
-N(substituted-heteroaryl-C₁-C₆-alkyl-)-
wherein substituted-heteroaryl is as defined above,
-S-, or -S(O)ₙ-,
wherein n is 1 or 2;
- C(O)-NH-;
- C(O)-NR⁵-,
wherein R⁵ is as defined above;
- NH-C(O)-;
-NR⁵-C(O)-,
wherein R⁵ is as defined above; and
- C(=NH)-NH-;
R¹ is -O-C₁-C₃-alkyl; and
R² is hydrogen;
with the provisos that when the compound is of Formulas (I) or (III) then A, B, D, and E may not all be hydrogen, D and E may not be C₁-C₃-alkyl when A and B are hydrogen, nor may one of D and E be hydrogen and the other be C₁-C₃-alkyl when A and B are hydrogen;
or a compound of Formula (III) in which R¹ = OCH₃, R² is H, B = E = H, and A and D taken together is either -CH₂-N(Cbz)-CH₂- or -CH₂-N(benzoyl)-CH₂-;
or a compound of Formula (III) in which B=E=H, R¹ is methoxy, R² is H; and A and D together is CH₂-N(R⁷)-CH₂-, wherein R⁷ is selected from the group consisting of:
(i) phenyl;
(ii) substituted-phenyl as defined above;
(iii) heteroaryl as defined above; and
(iv) substituted-heteroaryl as defined above;
or a compound of Formula (III) in which B=E=H, R¹ is methoxy, R² is H; and A and D together is CH₂-N(-(CH₂)ᵣ-M-(CH₂)ₛ-R⁷)-CH₂-, wherein r is 0, 1 or 2; s = 0, 1 or 2, M is as defined above; and R⁷ is selected from the group consisting of:
(i) C₁-C₃-alkyl;
(ii) phenyl;
(iii) substituted-phenyl as defined above;
(iv) heteroaryl as defined above; and
(v) substituted-heteroaryl as defined above, the method comprising:
(a) treating a compound having the formula: 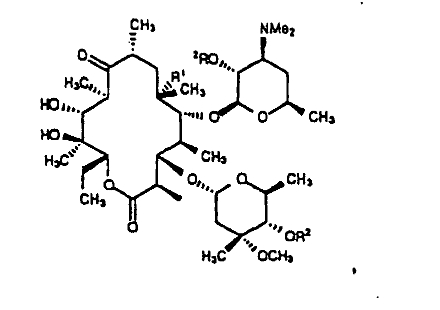 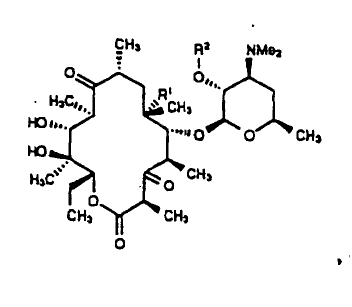 or 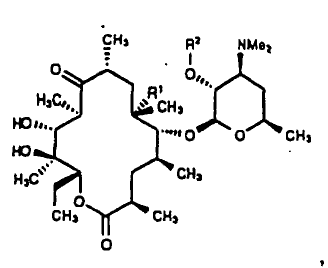 respectively, wherein R¹ is as described above and R² is a hydroxy-protecting group, with a base, and followed by reaction with carbonyldiimidazole, in an aprotic solvent, at a reaction temperature from -20°C to 70°C, for a period from 0.5 hours to 10 days, to prepare first intermediate compounds having the formulas: 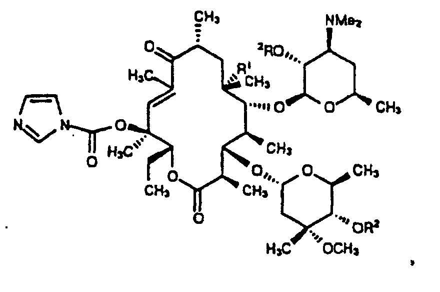 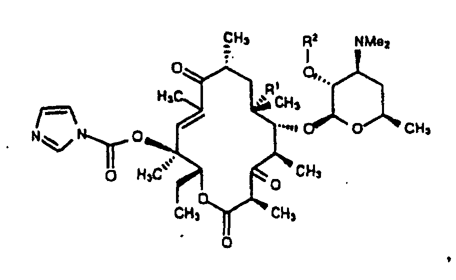 or 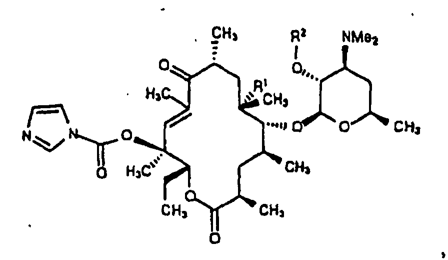 respectively; wherein R¹ and R² are as described above;
(b) reacting said first intermediate compounds with a compound having the formula: 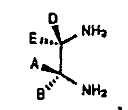 wherein A, B, D, and E are as described above, to give the bicyclic second intermediate compounds having the formulas: 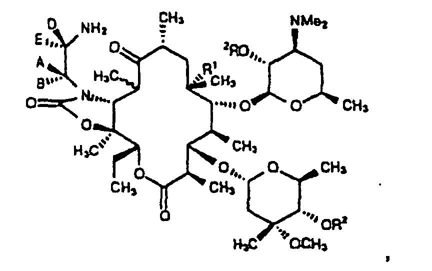 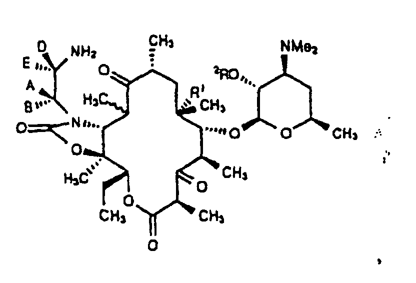 or 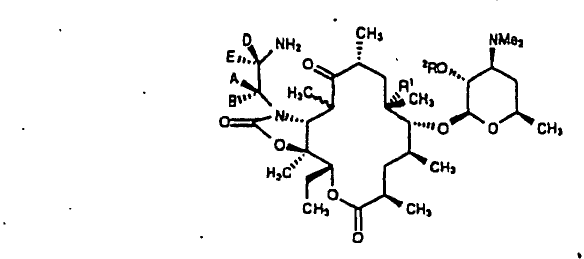 respectively; wherein R¹ and R² are as described above;
(c) deprotecting said second intermediate compounds by treatment with methanol or ethanol when OR² is an ester or with fluoride in THF or acetonitrile when R² is a trialkylsilyl group, for from 1 to 24 hours, to give the third intermediate compounds: 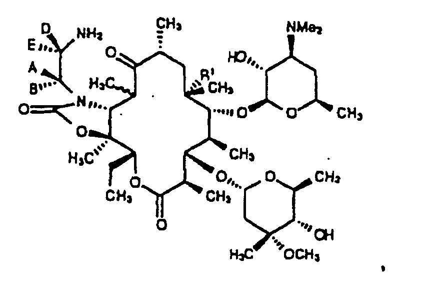 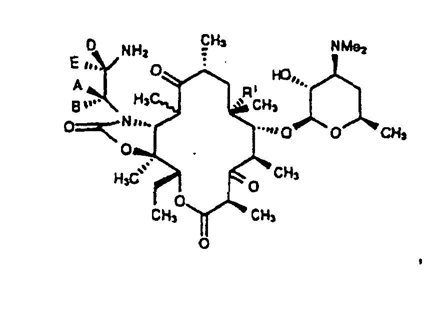 or 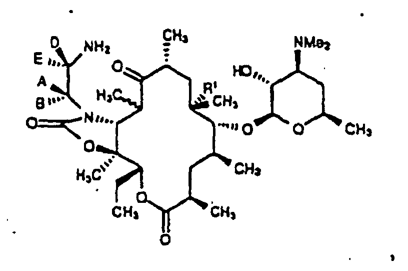 respectively; and
(d) cyclizing said third intermediate compounds by treatment with dilute acid, in an organic solvent, for a period of from 4 hours to 10 days to give the desired compounds (I), (III) or (IV) above.

13. A process according to Claim 12 wherein:
in step (a) the aprotic solvent is selected from the group consisting of dichloromethane, chloroform, DMF, tetrahydrofuran (THF), N-methyl pyrrolidinone or a mixture thereof, the reaction temperature is from 0°C to reaction temperature, and the period of reaction is 1-8 hours or up to 1-5 days;
in step (b) the reaction is run in aqueous acetonitrile, DMF or aqueous DMF;
in step (c) deprotecting said second intermediate compounds by treatment with methanol or ethanol when OR² is an ester or with fluoride in THF or acetonitrile when R² is a trialkylsilyl group, for from 1 to 24 hours; and
in step (d) cyclizing said third intermediate compounds by treatment with dilute acetic acid or HCl in ethanol or propanol for a period of from 4 hours to 10 days to give the desired compounds.

14. A process for the preparation of tricyclic macrolide derivatives having the Formulas: 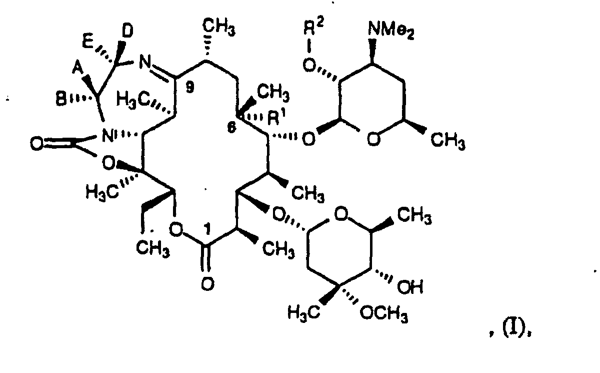 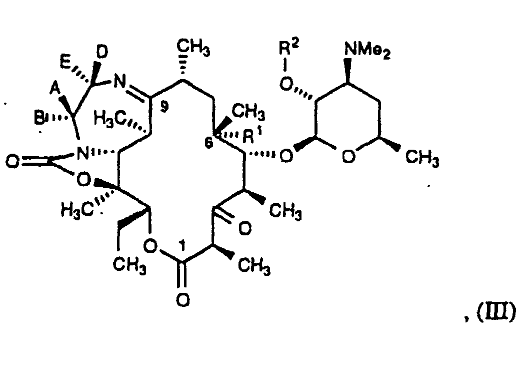 or 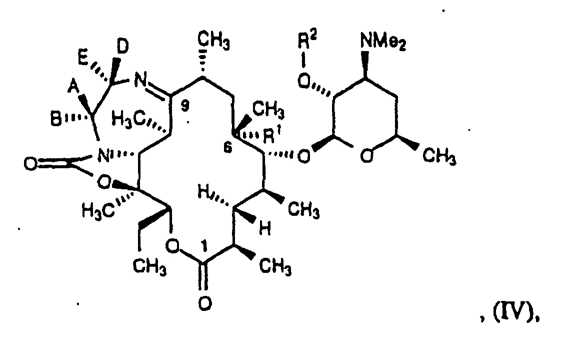 wherein
A, B, D and E are independently selected from the group consisting of:
(a) hydrogen;
(b) C₁-C₆-alkyl, unsubstituted or substituted with one substituent selected from the group consisting of:
(i) phenyl;
(ii) phenyl substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino;
(iii) heteroaryl selected from pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl and isoquinolinyl;
(iv) heteroaryl as defined above substituted with one, two or three groups independently selected from Cl, Br, F, I, OH, C₁-C₃-alkyl, C₁-C₆-alkoxy, methoxymethoxy, amino, C₁-C₃-alkyl-amino and di-(C₁-C₃-alkyl)-amino ;
(v) heterocycloalkyl selected from pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl and tetrahydrofuryl;
(vi) hydroxy;
(vii) C₁-C₆ alkoxy;
(viii) halogen consisting of Br, Cl, F or I; and
(ix) NR³R⁴, wherein R³ and R⁴ are independently selected from hydrogen and C₁-C₆-alkyl, or R³ and R⁴ are taken with the nitrogen atom to which they are connected to form a 3- to 7-membered ring optionally containing a hetero function consisting of -O-, -NH-, -N(C₁-C₆-alkyl-)-, -N(phenyl-C₁-C₆-alkyl-)-, - N(substituted-phenyl-C₁-C₆-alkyl-)- wherein substituted phenyl is as defined above, -N(heteroaryl-C₁-C₆-alkyl-)- wherein heteroaryl is as defined above, -N(substituted-heteroaryl-C₁-C₆-alkyl-)- wherein substituted heteroaryl is as defined above, -S- or -S(O)ₙ-, wherein n is 1 or 2;
(c) C₃-C₇-cycloalkyl;
(d) phenyl;
(e) substituted-phenyl as defined above;
(f) heteroaryl as defined above;
(g) substituted-heteroaryl as defined above;
(h) heterocycloalkyl as defined above;
and
(i) a group selected from option (b) above in which the substituent is - M-R⁵, wherein M is selected from the group consisting of:
(aa) -C(O)-NH-;
(bb) -NH-C(O)-;
(cc) -NH-
(dd) -N(CH₃) -
(ee) -O-;
(ff) -S(O)ₙ-, wherein n is 0, 1 or 2; and
(gg) -C(=NH)-NH-;
and R⁵ is selected from the group consisting of:
(aaa) C₁-C₆-alkyl, optionally substituted with a substituent selected from the group consisting of:
(i) phenyl;
(ii) substituted-phenyl as defined above;
(iii) heteroaryl as defined above;
(iv) substituted-heteroaryl as defined above;
(bbb) phenyl;
(ccc) substituted-phenyl as defined above;
(ddd) heteroaryl as defined above;
(eee) substituted-heteroaryl as defined above; and
(fff) heterocycloalkyl as defined above;
or
any one pair of substituents, consisting of AB, AD, AE, BD, BE, or DE, is taken together with the atom or atoms to which they are attached to form a 3- to 7-membered ring optionally containing a hetero function consisting of
-O-,
-NH-,
- N(C₁-C₆-alkyl-)-,
- N(phenyl-C₁-C₆-alkyl-)-,
- N(substituted-phenyl-C₁-C₆-alkyl-)-
wherein substituted-phenyl is as defined above,
- N(heteroaryl-C₁-C₆-alkyl-)-
wherein heteroaryl is as defined above,
- N(substituted-heteroaryl-C₁-C₆-alkyl-)-
wherein substituted-heteroaryl is as defined above,
- S-, or -S(O)ₙ-,
wherein n is 1 or 2;
- C(O)-NH-;
- C(O)-NR⁵-,
wherein R⁵ is as defined above;
-NH-C(O)-;
- NR⁵-C(O)-,
wherein R⁵ is as defined above; and
- C(=NH)-NH-;
R¹ is -O-C₁-C₃-alkyl; and
R² is hydrogen;
with the provisos that when the compound is of Formulas (I) or (III) then A, B, D, and E may not all be hydrogen, D and E may not be C₁-C₃-alkyl when A and B are hydrogen, nor may one of D and E be hydrogen and the other be C₁-C₃-alkyl when A and B are hydrogen;
or a compound of Formula (III) in which R¹ = OCH₃, R² is H, B = E = H, and A and D taken together is either -CH₂-N(Cbz)-CH₂- or -CH₂-N(benzoyl)-CH₂-;
or a compound of Formula (III) in which B=E=H, R¹ is methoxy, R² is H; and A and D together is CH₂-N(R⁷)-CH₂-, wherein R⁷ is selected from the group consisting of:
(i) phenyl;
(ii) substituted-phenyl as defined above;
(iii) heteroaryl as defined above; and
(iv) substituted-heteroaryl as defined above;
or a compound of Formula (III) in which B=E=H, R¹ is methoxy, R² is H; and A and D together is CH₂-N(-(CH₂)ᵣ-M-(CH₂)ₛ-R⁷)-CH₂-, wherein r is 0, 1 or 2; s = 0, 1 or 2, M is as defined above; and R⁷ is selected from the group consisting of:
(i) C₁-C₃-alkyl;
(ii) phenyl;
(iii) substituted-phenyl as defined above;
(iv) heteroaryl as defined above; and
(v) substituted-heteroaryl as defined above, the method comprising:
(a) treating a compound having the formula: 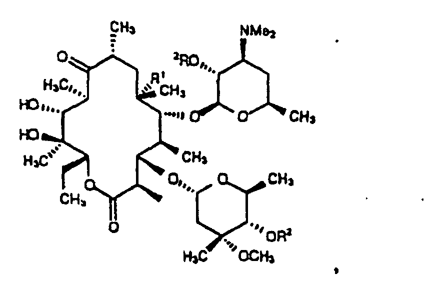 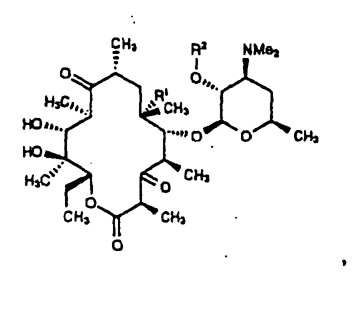 , or 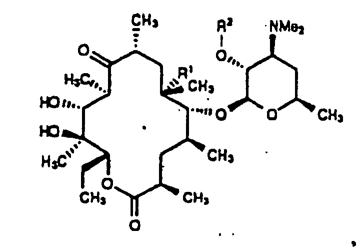 respectively, wherein R¹ is as described above and R² is a hydroxy-protecting group, with a base, and followed by reaction with carbonyldiimidazole, in an aprotic solvent, at a reaction temperature from -20°C to 70°C, for a period from 0.5 to 24 hours, to prepare first intermediate compounds having the formulas: 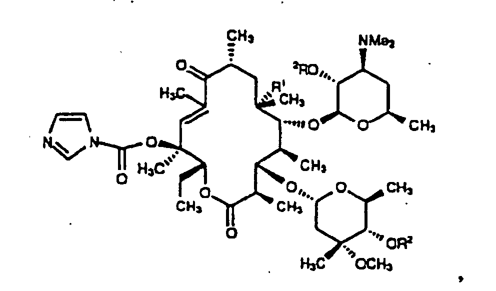 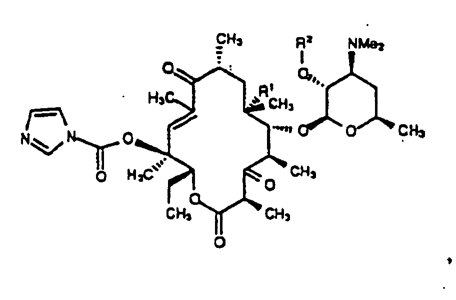 or 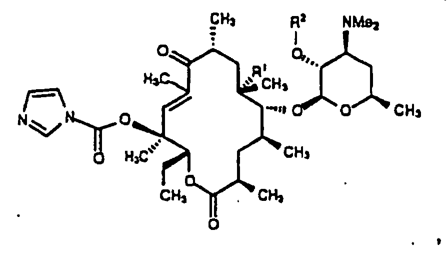 respectively; wherein R¹ and R² are as described above;
(b) reacting said first intermediate compounds with a compound having the formula: 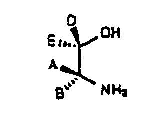 wherein A, B, D, and E are as described above to give the bicyclic second intermediate compounds: 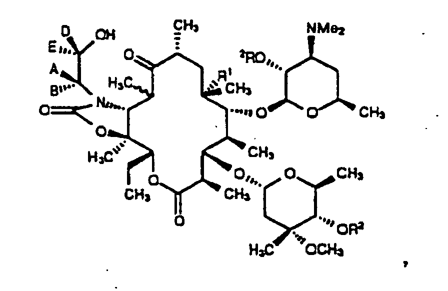 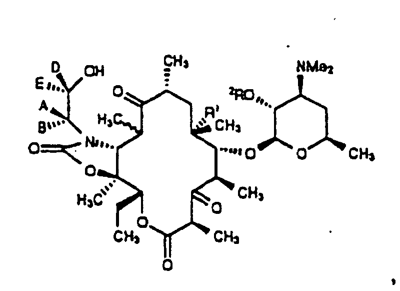 or 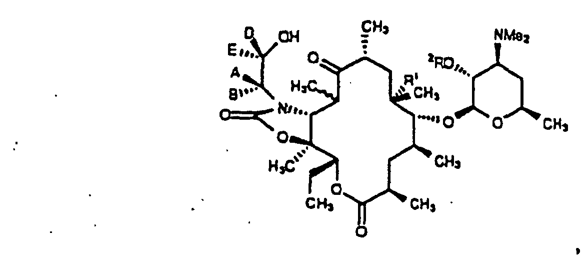 respectively;
(c) reacting the hydroxy group of the said bicyclic second intermediate compounds by treatment wi th triphenylphosphine and diphenylphosphoryl azide in tetrahydrofuran, under Mitsunobu reaction conditions, to prepare the third intermediate compounds: 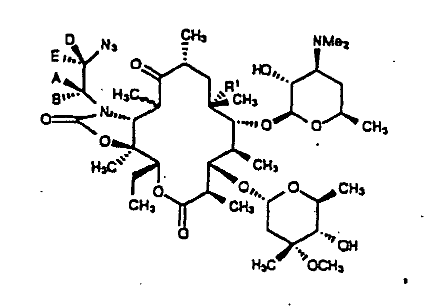 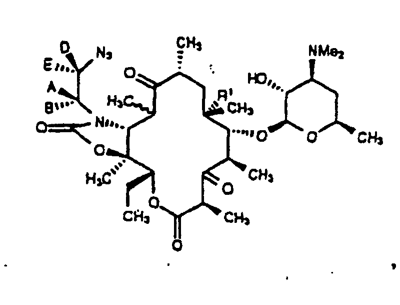 or 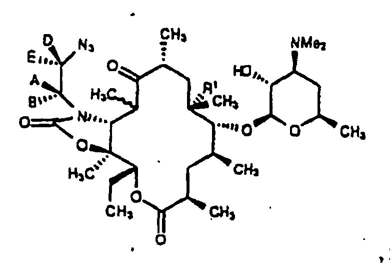 respectively;
(d) reducing the third intermediate compounds having an azido group, with reducing reagents to prepare the fourth intermediate compounds having the formulas: 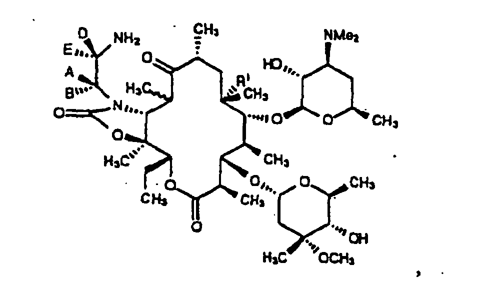 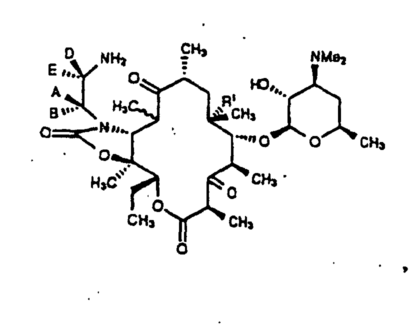 or 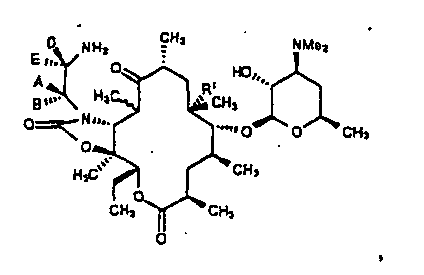 respectively; and
(e) cyclizing said fourth intermediate compounds by treatment with a dilute acid, in an organic solvent, for a period of from 4 hours to 10 days to give the desired compounds of Formulas (I), (III) or (IV).

15. The process as in Claim 14 wherein:
in step (a) the base is sodium hydride, lithium hydride, or potassium carbonate, the aprotic solvent is dichloromethane, chloroform, DMF, tetrahydrofuran (THF), N-methyl pyrrolidinone or a mixture thereof, the reaction temperature is from 0°C to reaction temperature, and the reaction period is from 1-8 hours;
in step (b) the solvent is aqueous acetonitrile, DMF or aqueous DMF;
in step (c) the reagents are triphenylphosphine and diphenylphosphoryl azide-DEAD in tetrahydrofuran, under Mitsunobu reaction conditions;
in step (d) the reducing reagent is triphenylphosphine-water, hydrogen with a catalyst, sodium borohydride, or dialkylaluminum hydride;
in step (e) cyclizing said third intermediate compounds by treatment with dilute acetic acid or HCl in ethanol or propanol for a period of from 4 hours to 10 days to give the desired compounds.

16. The process as in Claim 14 wherein:
step (c) is replaced with a two-step sequence which comprises (1) reacting the hydroxy group of the bicyclic second intermediate compounds thereof with an alkyl or aryl sulfonyl chloride, an alkyl or aryl sulfonic anhydride or trifluoromethanesulfonic anhydride in an aprotic solvent at -78°C to reaction temperature to give the corresponding sulfonate, and (2) reacting the said sulfonate with lithium azide or sodium azide in an aprotic solvent at 0°C to 100°C to give the third intermediate compound.

## Patentansprüche

1. Eine Verbindung gewählt aus der Gruppe mit folgenden Formeln: 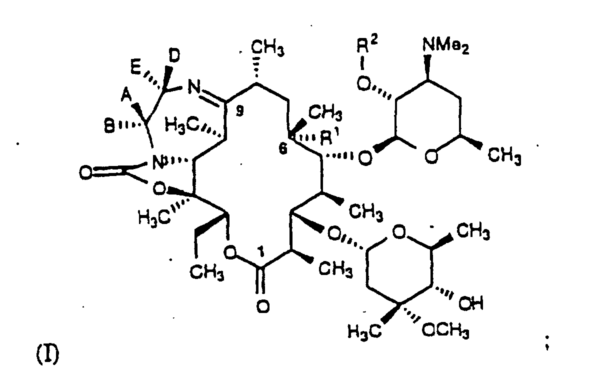 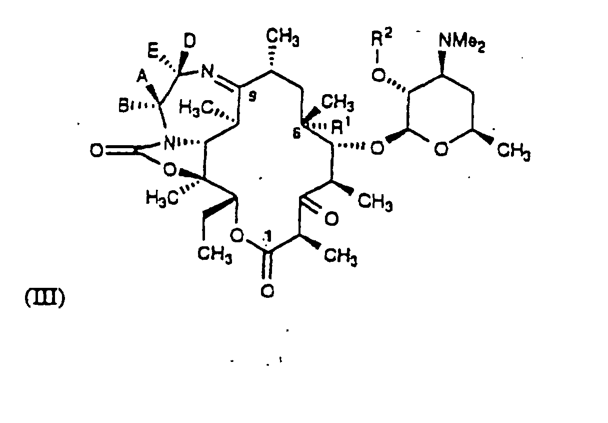 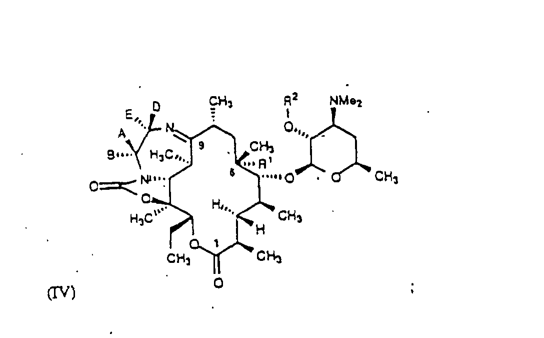 und ebenso wie pharmazeutisch verträgliche Salze und Ester davon, worin:
A, B, D und E unabhängig gewählt sind aus der Gruppe bestehend aus:
(a) Wasserstoff;
(b) C₁-C₆-Alkyl, unsubstituiert oder substituiert mit einem Substituenten gewählt aus der Gruppe bestehend aus:
(i) Phenyl;
(ii) Phenyl substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(iii) Heteroaryl gewählt aus Pyridyl, Pyrazinyl, Pyrimidinyl, Pyrrolyl, Pyrazolyl, Imidazolyl, Thiazolyl, Oxazolyl, Isooxazolyl, Thiadiazolyl, Oxadiazolyl, Thiophenyl, Furanyl, Chinolinyl und Isochinolinyl;
(iv) Heteroaryl, wie oben definiert, substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(v) Hetercycloalkyl gewählt aus Pyrrolidinyl, Pyrazolinyl, Pyrazolidinyl, Imidazolinyl, Imidazolidinyl, Piperidinyl, Piperazinyl, Oxazolidinyl, Isoxazolidinyl, Morpholinyl, Thiazolidinyl, Isothiazolidinyl und Tetrahydrofuryl;
(vi) Hydroxy;
(vii) C₁-C₆ Alkoxy;
(viii) Halogen bestehend aus Br, Cl, F oder I; und
(ix) NR³R⁴, worin R³ und R⁴ unabhängig gewählt sind aus Wasserstoff und C₁-C₆-Alkyl oder R³ und R⁴ werden zusammengenommen mit dem Stickstoffatom, an welches sie gebunden sind, um einen 3- bis 7-gliedrigen Ring zu bilden, der wahlweise eine Heterofunktion enthält, bestehend aus' -O-, -NH-, -N(C₁-C₆-Alkyl-)-,
-N(Phenyl-C₁-C₆-alkyl-)-,
-N(substituiertes-Phenyl-C₁-C₆-alkyl-)-, worin substituiertes Phenyl, wie oben definiert ist, -N(Heteroaryl-C₁-C₆-alkyl-)-worin Heteroaryl, wie oben definiert ist, -N(substituiertes-Heteroaryl-C₁-C₆-alkyl-)- worin substituiertes Heteroaryl wie oben definiert ist, -S- oder -S(O)ₙ-, worin n 1 oder 2 ist;
(c) C₃-C₇-Cycloalkyl;
(d) Phenyl;
(e) substituiertes Phenyl, wie oben definiert;
(f) Heteroaryl, wie oben definiert;
(g) substituiertes-Heteroaryl, wie oben definiert;
(h) Heterocycloalkyl, wie oben definiert;
und
(i) einer Gruppe gewählt aus Option (b) oben, in der der Substituent -M-R⁵ ist, worin M gewählt ist aus der Gruppe bestehend aus :
(aa) -C(O)-NH-;
(bb) -NH-C(O)-;
(cc) -NH-
(dd) -N(CH₃)-
(ee) -O-;
(ff) -S(O)ₙ-, worin n 0, 1 oder 2 ist; und
(gg) -C(=NH)-NH-;
und R₅ ist gewählt aus der Gruppe bestehend aus:
(aaa) C₁-C₆-Alkyl, wahlweise substituiert mit einem Substituenten gewählt aus der Gruppe bestehend aus:
(i) Phenyl;
(ii) substituiertes-Phenyl, wie oben definiert;
(iii) Heteroaryl, wie oben definiert;
(iv) substituiertes-Heteroaryl, wie oben definiert;
(bbb) Phenyl;
(ccc) substituiertes-Phenyl, wie oben definiert;
(ddd) Heteroaryl, wie oben definiert;
(eee) substituiertes-Heteroaryl, wie oben definiert; und
(fff) Heterocycloalkyl, wie oben definiert;
oder
irgendein Paar von Substituenten, bestehend aus AB, AD, AE, BD, BE oder DE wird zusammengenommen mit dem Atom oder den Atomen, an welche sie gebunden sind, um einen 3-bis 7-gliedrigen Ring zu bilden, der wahlweise eine Heterofunktion enthält, bestehend aus
-O-,
- NH-,
-N(C₁-C₆-Alkyl-)-,
-N(Phenyl-C₁-C₆-alkyl-)-,
-N(substituiertes-Phenyl-C₁-C₆-alkyl-)-,
worin substituiertes-Phenyl wie oben definiert ist,
-N(Heteroaryl-C₁-C₆-alkyl-)-,
worin Heteroaryl wie oben definiert ist,
-N(substituiertes-Heteroaryl-C₁-C₆-alkyl-)-,
worin substituiertes-Heteroaryl wie oben definiert ist,
-S- oder -S(O)ₙ-,
worin n 1 oder 2 ist;
-C(O)-NH-;
- C(O)-NR⁵-,
worin R⁵ wie oben definiert ist;
-NH-C(O)-;
-NR⁵-C(O)-,
worin R⁵ wie oben definiert ist; und
- C(=NH)-NH-;
R¹ ist -O-C₁-C₃-Alkyl; und
R² ist Wasserstoff;
unter den Voraussetzungen, daß, wenn die Verbindung die Formeln (I) oder (III) hat, dann können A, B, D und E nicht alle Wasserstoff sein, D und E können nicht C₁-C₃-Alkyl sein, wenn A und B Wasserstoff sind, noch kann eines von D und E Wasserstoff und das andere C₁-C₃-Alkyl sein, wenn A und B Wasserstoff sind;
oder eine Verbindung von Formel (III), in der R¹ = OCH₂ ist, R² ist H, B = E = H, und A und D zusammengenommen sind entweder -CH₂-N(Cbz)-CH₂- oder -CH₂-N(Benzoyl)-CH₂-;
oder eine Verbindung von Formel (III), in der B=E=H ist, R¹ ist Methoxy, R² ist H; und A und D zusammen sind CH₂-N (R₇) -CH₂-, worin R⁷ gewählt ist aus der Gruppe bestehend aus:
(i) Phenyl;
(ii) substituiertes-Phenyl wie oben definiert;
(iii) Heteroaryl wie oben definiert; und
(iv) substituiertes-Heteroaryl wie oben definiert;
oder eine Verbindung von Formel (III), in der B=E=H ist; R¹ ist Methoxy, R² ist H; und A und D zusammen sind CH₂-N(-(CH₂)ᵣ-M-(CH₂)ₛ-R⁷)-CH₂-, worin r 0, 1 oder 2 ist; s = 0, 1 oder 2, M ist wie oben definiert; und R⁷ ist gewählt aus der Gruppe bestehend aus:
(i) C₁-C₃-Alkyl;
(ii) Phenyl;
(iii) substituiertes-Phenyl, wie oben definiert;
(iv) Heteroaryl, wie oben definiert; und
(v) substituiertes-Heteroaryl, wie oben definiert.

2. Eine Verbindung gemäß Anspruch 1, mit der Formel: 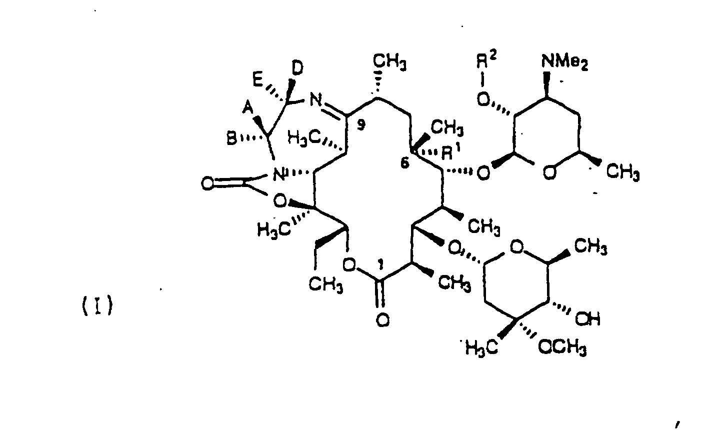 worin A, B, D, E und R¹-R⁵ wie darin beschrieben sind.

3. Eine Verbindung gemäß Anspruch 1, mit der Formel: 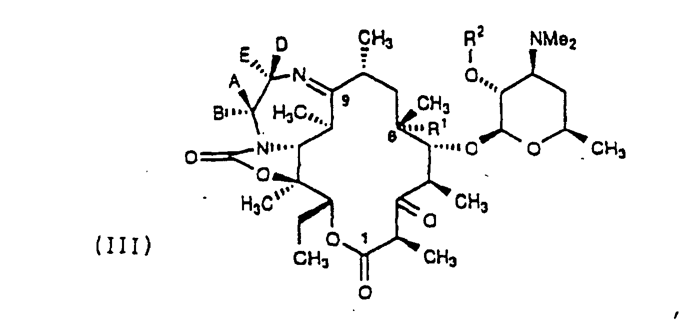 worin A, B, D, E und R¹-R⁵ wie darin beschrieben sind.

4. Eine Verbindung gemäß Anspruch 1 mit der Formel: 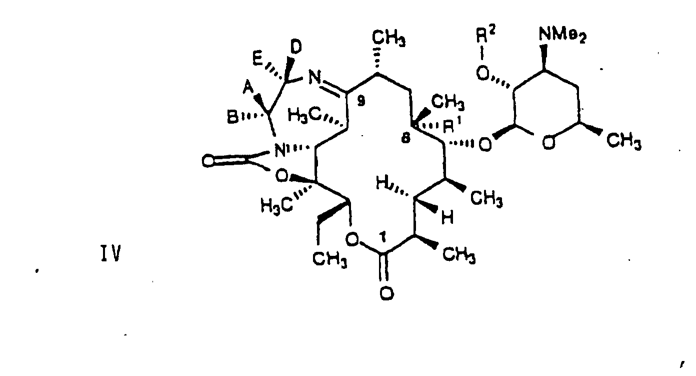 worin A, B, D, E und R¹-R⁵ wie darin beschrieben sind.

5. Eine Verbindung gemäß Anspruch 3, worin R¹ Methoxy ist, und A, B, D, E und R¹-R⁵ wie darin beschrieben sind.

6. Eine Verbindung gemäß Anspruch 5, worin irgendwelche drei von den A, B, D und E Gruppen Wasserstoff sind, und die andere Gruppe ist gewählt aus einer einzelnen substituierten C₁-C₆-Alkylgruppe, bestehend aus -(CH₂)ₘR⁶, worin m = 1, 2, 3 oder 4 ist, und R⁶ ist gewählt aus der Gruppe bestehend aus:
(a) Phenyl;
(b) Phenyl substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(c) Heteroaryl gewählt aus Pyridyl, Pyrazinyl, Pyrimidinyl, Pyrrolyl, Pyrazolyl, Imidazolyl, Thiazolyl, Oxazolyl, Isooxazolyl, Thiadiazolyl, Oxadiazolyl, Thiophenyl, Furanyl, Chinolinyl und Isochinolinyl;
(d) Heteroaryl wie oben definiert, substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(e) Heterocycloalkyl, gewählt aus Pyrrolidinyl, Pyrazolinyl, Pyrazolidinyl, Imidazolinyl, Imidazolidinyl, Piperidinyl, Piperazinyl, Oxazolidinyl, Isoxazolidinyl, Morpholinyl, Thiazolidinyl, Isothiazolidinyl und Tetrahydrofuryl;
(f) Hydroxy;
(g) C₁-C₆-Alkoxy;
(h) NR³R⁴, worin R³ und R⁴ unabhängig gewählt sind aus Wasserstoff und C₁-C₆-Alkyl, oder R³ und R⁴ werden zusammengenommen mit dem Stickstoffatom, an welches sie gebunden sind, um einen 3- bis 7-gliedrigen Ring zu bilden, der wahlweise eine Heterofunktion enthält, bestehend aus -O-, -NH-, -N(C₁-C₆-Alkyl-)-, -N(Phenyl-C₁-C₆-alkyl-)-, -N(substituiertes-Phenyl-C₁-C₆-alkyl-)-, worin substituiertes Phenyl wie oben definiert ist, -N(Heteroaryl-C₁-C₆-alkyl-)-, worin Heteroaryl wie oben definiert ist, -N(substituiertes-Heteroaryl-C₁-C₆-alkyl-)-, worin substituiertes Heteroaryl wie oben definiert ist, -S- oder -S(O)ₙ-, worin n 1 oder 2 ist;
(i) Halogen bestehend aus Br, Cl, F oder I,
(j) C₁-C₃-Alkyl; oder
(k) (CH₂)ᵣ-M-(CH₂)ₛ-R⁷, worin r = 0, 1 oder 2 ist, s = 0, 1 oder 2 und M ist gewählt aus der Gruppe bestehend aus:
(aa) -C(O)-NH-;
(bb) -NH-C(O)-;
(cc) -NH-
(dd) -N(CH₃)-
(ee) -O-;
(ff) -S(O)ₙ-, worin n 0, 1 oder 2 ist; und
(gg) -C (=NH) -NH-;
und R⁷ ist gewählt aus der Gruppe bestehend aus:
(aaa) C₁-C₃-Alkyl;
(bbb) Phenyl;
(ccc) substituiertes-Phenyl wie oben definiert;
(ddd) Heteroaryl wie oben definiert; und
(eee) substituiertes-Heteroaryl wie oben definiert.

7. Eine Verbindung gemäß Anspruch 5, worin B = E = H, und A und D zusammen sind gewählt aus der Gruppe bestehend aus:
(a) -CH₂-Z-CH₂-, worin Z gewählt ist aus der Gruppe bestehend aus:
(aa) -C(O)-NH-;
(bb) -C(O)-NR⁵-, worin R⁵ gewählt ist aus der Gruppe bestehend aus:
(aaa) C₁-C₆ Alkyl, unsubstituiert oder substituiert mit einem Substituenten gewählt aus der Gruppe bestehend aus:
(i) Phenyl;
(ii) Phenyl substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(iii) Heteroaryl gewählt aus Pyridyl, Pyrazinyl, Pyrimidinyl, Pyrrolyl, Pyrazolyl, Imidazolyl, Thiazolyl, Oxazolyl, Isooxazolyl, Thiadiazolyl, Oxadiazolyl, Thiophenyl, Furanyl, Chinolinyl und Isochinolinyl; und
(iv) Heteroaryl wie oben definiert, substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(bbb) Phenyl;
(ccc) substituiertes-Phenyl, wie oben definiert;
(ddd) Heteroaryl, wie oben definiert;
(eee) substituiertes-Heteroaryl, wie oben definiert; und
(fff) Heterocycloalkyl gewählt aus Pyrrolidinyl, Pyrazolinyl, Pyrazolidinyl, Imidazolinyl, Imidazolidinyl, Piperidinyl, Piperazinyl, Oxazolidinyl, Isoxazolidinyl, Morpholinyl, Thiazolidinyl, Isothiazolidinyl und Tetrahydrofuryl;
(cc) -NH-C(O)-;
(dd) -NR⁵-C(O)-, worin R⁵ wie oben definiert ist
(ee) -NH-;
(ff) -N(CH₃)-;
(gg) -O-;
(hh) -S(O)ₙ-, worin n 0, 1 oder 2 ist; und
(ii) -C(=NH)-NH-;
(b) -CH₂-N(-(CH₂)ₛ-R⁷)-CH₂-, worin s = 0, 1 oder 2 ist und R⁷ ist gewählt aus der Gruppe bestehend aus:
(aa) C₁-C₃-Alkyl;
(bb) Phenyl;
(cc) substituiertes-Phenyl, wie oben definiert;
(dd) Heteroaryl, wie oben definiert; und
(ee) substituiertes-Heteroaryl, wie oben definiert;
(c) -CH₂-N(-(CH₂)ᵣ-M-(CH₂)ₛ-R⁷)-CH₂-, worin r 0, 1 oder 2 ist; s = 0,1 oder 2, R⁷ ist wie oben definiert und M ist gewählt aus der Gruppe bestehend aus:
(i) -C(O)-NH-;
(ii) -NH-C(O)-;
(iii) -NH-
(iv) -N(CH₃)-
(v) -O-;
(vi) -S(O)ₙ-, worin n 0, 1 oder 2 ist; und
(vii) -C(=NH)-NH-; und
(d) -CH₂-(CH₂)ᵣ-CH₂-, worin r = 0, 1 oder 2 ist.

8. Eine Verbindung gemäß Anspruch 1, die folgende ist:
Verbindung von Formel (IV); R¹=Methoxy; R²= Wasserstoff; A=B=D=E=Wasserstoff;
Verbindung von Formel (III); A=B=E=H, D=Benzyl, R¹=Methoxy; R²= Wasserstoff;
Verbindung von Formel (III); A=B=E=H, E=Benzyl, R¹=Methoxy; R²= Wasserstoff;
Verbindung von Formel (III); A=Benzyl, B=D=E=H, R¹=Methoxy; R²= Wasserstoff;
Verbindung von Formel (III); B=Benzyl, A=D=E=H, R¹=Methoxy; R²= Wasserstoff;
Verbindung von Formel (III); A=E=Phenyl, B=D=H, R¹=Methoxy, R²= Wasserstoff;
Verbindung von Formel (III); A=Methyl, B=D=E=H, R¹=Methoxy, R²= Wasserstoff;
Verbindung von Formel (III); B=Methyl, A=D=E=H, R¹=Methoxy, R²= Wasserstoff;
Verbindung von Formel (III); A=D=Methyl, B=E=H, R¹=Methoxy, R²= Wasserstoff;
Verbindung von Formel (III) ; A=E=Methyl, B=D=H, R¹=Methoxy, R²= Wasserstoff;
Verbindung von Formel (III); B=D=H; A und E zusammengenommen sind -CH₂CH₂CH₂-, R¹=Methoxy, R²=Wasserstoff;
Verbindung von Formel (III); B=E=H, A und D zusammengenommen sind -CH₂CH₂CH₂CH₂-, R¹=Methoxy, R²= Wasserstoff;
Verbindung von Formel (III); R¹=OCH3, R²=H; A=B=D=H; E=-CH₂NH₂;
Verbindung von Formel (III); R¹=OCH3, R²=H; A=B=E=H; D=-CH₂NH₂;
Verbindung von Formel (III); R¹=OCH3, R²=H; B=E=H; A und D zusammengenommen sind -CH₂CH₂CH₂-;
Verbindung von Formel (III); R¹=OCH3, R²=H; B=E=H; A und D zusammengenommen sind -CH₂OCH₂-;
Verbindung von Formel (III); R¹=OCH3, R²=H; B=E=H; A und D zusammengenommen sind -CH₂NH-CH₂-;
Verbindung von Formel (III); R¹=OCH3, R²=H; B=E=H; A und D zusammengenommen sind -CH₂-N(Cbz)CH₂-;
Verbindung von Formel (III); R¹=OCH3, R²=H; B=E=H; A und D zusammengenommen sind -CH₂-N(Benzyl)-CH₂-;
Verbindung von Formel (III); R¹=OCH3, R²=H; B=E=H; A und D zusammengenommen sind -CH₂-N(Benzoyl)-CH₂-;
Verbindung von Formel (III); R¹=OCH3, R²=H; B=E=H; A und D zusammengenommen sind -CH₂-N(Phenyl-CH₂-CH₂-)-CH₂-;
Verbindung von Formel (III); R¹=OCH3, R²=H; B=E=H; A und D zusammengenommen sind -CH₂-N(4-Cl-phenyl-CH₂-)-CH₂-;
Verbindung von Formel (III); R¹=OCH3, R²=H; B=E=H; A und D zusammengenommen sind -CH₂-N(4-Pyridyl-CH₂-)-CH₂-;
Verbindung von Formel (III); R¹=OCH3, R²=H; B=E=H; A und D zusammengenommen sind -CH₂-N(2-Pyridyl-CH₂-)-CH₂-;
Verbindung von Formel (III); R¹=OCH3, R²=H; B=E=H; A und D zusammengenommen sind -CH₂-N(3-Pyridyl-CH₂-)-CH₂-;
Verbindung von Formel (III); R¹=OCH3, R²=H; B=E=H; A und D zusammengenommen sind -CH₂-N(4-Chinolyl-CH₂-)-CH₂-;
Verbindung von Formel (III); R¹=OCH3, R²=H; B=E=H; A=D=-CH₂-O-CH₂-Phenyl;
Verbindung von Formel (III); R¹=OCH3, R²=H; B=E=H; A=D=-CH₂-OH;
Verbindung von Formel (III); R¹=OCH3, R²=H; B=E=H; A=D=-CH₂-O-Phenyl;
Verbindung von Formel (III); R¹=OCH3, R²=H; A=B=H, D und E zusammengenommen sind -CH₂-CH₂-CH₂-CH₂-;
Verbindung von Formel (III) ; R¹=OCH3, R²=H; A und B zusammengenommen sind -CH₂-CH₂-CH₂-CH₂-; D=E=H;
Verbindung von Formel (III); R¹=OCH3, R²=H; A=B=H; D und E zusammengenommen sind -CH₂-O-CH₂-;
Verbindung von Formel (III); R¹=OCH3, R²=H; A=D=E=H; B=-CH₂-CH₂-Phenyl;
Verbindung von Formel (III) ; R¹=OCH3, R²=H; A=D=E=H; B=-CH₂-CH₂-CH₂-Phenyl;
Verbindung von Formel (III) ; R¹=OCH3, R²=H; A=D=E=H; B=-CH₂-O-CH₂-Phenyl;
Verbindung von Formel (III) ; R¹=OCH3, R²=H; A=D=E=H; B=-CH₂-CH₂-(4-OCH₃-Phenyl) ;
Verbindung von Formel (III) ; R¹=OCH3, R²=H; A=-CH₂-CH₂-Phenyl; B=D=E=H;
Verbindung von Formel (III) ; R¹=OCH3, R²=H; A=-CH₂-CH₂-CH₂-Phenyl; B=D=E=H;
Verbindung von Formel (III) ; R¹=OCH3, R²=H; A=-CH₂-O-CH₂-Phenyl; B=D=E=H;
Verbindung von Formel (III); R¹=OCH3, R²=H; B=D=E=H; A=-CH₂-CH₂-(4-OCH₃-Phenyl);
Verbindung von Formel (III) ; R¹=OCH3, R²=H; A=B=D=H; E=-CH₂-CH₂-Ph;
Verbindung von Formel (III) ; R¹=OCH3, R²=H; A=B=E=H; D=-CH₂-CH₂-Ph;
Verbindung von Formel (III) ; R¹=OCH3, R²=H; A=B=D=H; E=-CH₂-CH₂-CH₂-Ph;
Verbindung von Formel (III) ; R¹=OCH3, R²=H; A=B=E=H; D=-CH₂-CH₂-CH₂-Ph;
Verbindung von Formel (III); R¹=OCH3, R²=H; A=-CH₂-CH₂-OPh; B=D=E=H;
Verbindung von Formel (III); R¹=OCH3, R²=H; A=-CH₂-CH₂-NH2; B=D=E=H;
Verbindung von Formel (III); R¹=OCH3, R²=H; A=-CH₂-CH₂-OH; B=D=E=H;
Verbindung von Formel (III); R¹=OCH3, R²=H; A=-CH₂-CH₂-NH(4-Pyridyl-); B=D=E=H
Verbindung von Formel (III) ; R¹=OCH3, R²=H; A=B=D=H; E=-CH₂-OH;
Verbindung von Formel (III) ; R¹=OCH3, R²=H; A=B=E=H; D=-CH₂-OH;
Verbindung von Formel (III); R¹=OCH3, R²=H; A=B=E=H; D=-CH₂-NHBenzoyl;
Verbindung von Formel (III); R¹=OCH3, R²=H; A=B=E=H; D=-CH₂-NHBenzyl;
Verbindung von Formel (III); R¹=OCH3, R²=H; A=B=D=H; E=-CH₂-NHBenzoyl;
Verbindung von Formel (III) ; R¹=OCH3, R²=H; A=B=D=H; E=-CH₂-NHBenzyl;
Verbindung von Formel (III) ; R¹=OCH3, R²=H; B=D=H; A=E=-CH₂OCH₂(4-Cl-Phenyl);
Verbindung von Formel (III); R¹=OCH3, R²=H; A=B=E=H; D=-CH₂-N(CH₃)-Benzyl;
Verbindung von Formel (III); R¹=OCH3, R²=H; A=B=D=H; E=-CH₂-N(CH₃)-Benzyl;
Verbindung von Formel (III) ; R¹=OCH3, R²=H; A=B=D=H; E=-CH₂-NH-Phenyl; oder
Verbindung von Formel (III) ; R¹=OCH3, R²=H; A=B=E=H; D=-CH₂-NH-Phenyl.

9. Eine Verbindung gemäß Anspruch 1, die folgende ist:
Verbindung von Formel (IV); R¹=Methoxy; R²=Wasserstoff; A=B=D=E=Wasserstoff;
Verbindung von Formel (III); A=B=E=H, D=Benzyl, R¹=Methoxy; R²=Wasserstoff;
Verbindung von Formel (III); A=B=D=H, E=Benzyl, R¹=Methoxy; R²=Wasserstoff;
Verbindung von Formel (III); A=Benzyl, B=D=E=H, R¹=Methoxy; R²=Wasserstoff;
Verbindung von Formel (III); B=Benzyl, A=D=E=H, R¹=Methoxy; R²=Wasserstoff;
Verbindung von Formel (III); A=E=Phenyl, B=D=H, R¹=Methoxy; R²=Wasserstoff;
Verbindung von Formel (III); A=Methyl, B=D=E=H, R¹=Methoxy; R²=Wasserstoff;
Verbindung von Formel (III); B=Methyl, A=D=E=H, R¹=Methoxy; R²=Wasserstoff;
Verbindung von Formel (III); A=D=Methyl; B=E=H, R¹=Methoxy; R²=Wasserstoff;
Verbindung von Formel (III); A=E=Methyl; B=D=H, R¹=Methoxy; R²=Wasserstoff;
Verbindung von Formel (III); B=D=H; A und E zusammengenommen sind -CH₂CH₂CH₂-, R¹=Methoxy; R²=Wasserstoff; oder
Verbindung von Formel (III); B=E=H; A und D zusammengenommen sind -CH₂CH₂CH₂CH₂-, R¹=Methoxy; R²=Wasserstoff.

10. Eine pharmazeutische Zusammensetzung, die eine therapeutisch wirksame Menge einer Verbindung gemäß irgendeinem der Ansprüche 1 bis 9 in Kombination mit einem pharmazeutisch verträglichen Träger umfaßt.

11. Verwendung einer pharmazeutischen Verbindung gemäß irgendeinem der Ansprüche 1 bis 9 zur Herstellung eines Medikaments zur Kontrolle einer bakteriellen Infektion in einem Säugetier.

12. Ein Verfahren für die Herstellung von trizyklischen Makrolidverbindungen mit folgenden Formeln: 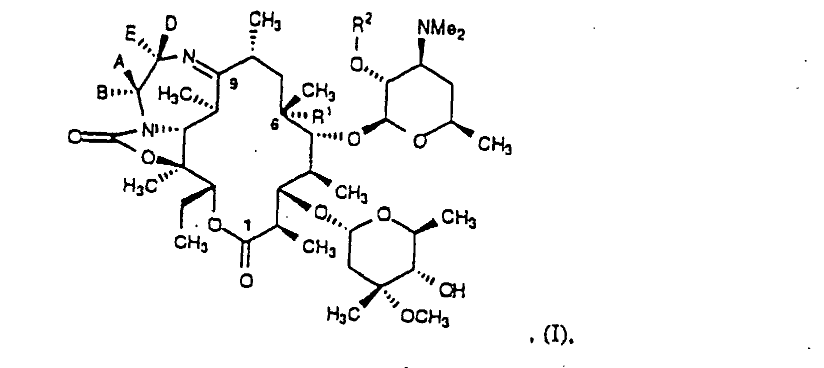 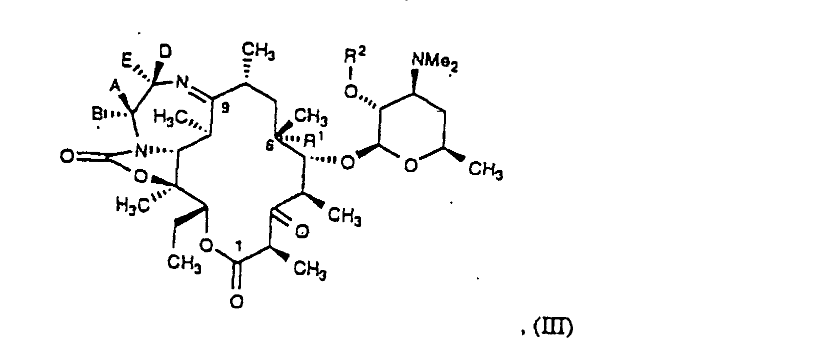 oder 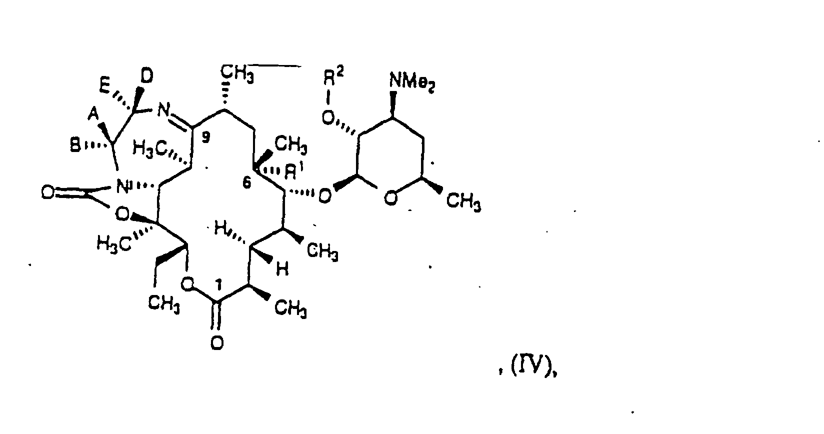 worin
A, B, D und E unabhängig gewählt sind aus der Gruppe bestehend aus:
(a) Wasserstoff;
(b) C₁-C₆-Alkyl, unsubstituiert oder substituiert mit einem Substituenten gewählt aus der Gruppe bestehend aus:
(i) Phenyl;
(ii) Phenyl substituiert mit ein, zwei oder drei Gruppen, unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(iii) Heteroaryl . gewählt aus Pyridyl, Pyrazinyl, Pyrimidinyl, Pyrrolyl, Pyrazolyl, Imidazolyl, Thiazolyl, Oxazolyl, Isooxazolyl, Thiadiazolyl, Oxadiazolyl, Thiophenyl, Furanyl, Chinolinyl und Isochinolinyl;
(iv) Heteroaryl wie oben definiert, substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkylamino und di-(C₁-C₃-Alkyl)-amino;
(v) Heterocycloalkyl gewählt aus Pyrrolidinyl, Pyrazolinyl,- Pyrazolidinyl, Imidazolinyl, Imidazolidinyl, Piperidinyl, Piperazinyl, Oxazolidinyl, Isoxazolidinyl, Morpholinyl, Thiazolidinyl, Isothiazolidinyl und Tetrahydrofuryl;
(vi) Hydroxy;
(vii) C₁-C₆ Alkoxy;
(viii) Halogen bestehend aus Br, Cl, F oder I; und
(ix) NR³R⁴, worin R³ und R⁴ unabhängig gewählt sind aus Wasserstoff und C₁-C₆-Alkyl, oder R³ und R⁴ werden mit dem Stickstoffatom, an das sie gebunden sind, genommen, um einen 3-bis 7-gliedrigen Ring zu bilden, der wahlweise eine Heterofunktion enthält, bestehend aus -O-, -NH-, -N(C₁-C₆-Alkyl-)-, -N(Phenyl-C₁-C₆-alkyl-)-, -N(substituiertes-Phenyl-C₁-C₆-alkyl-)-, worin substituiertes Phenyl wie oben definiert ist, -N(Heteroaryl-C₁-C₆-alkyl-)-, worin Heteroaryl wie oben definiert ist, -N(substituiertes-Heteroaryl-C₁-C₆-alkyl-)-, worin substituiertes Heteroaryl wie oben definiert ist, -S- oder -S(O)ₙ-, worin n 1 oder 2 ist;
(c) C₃-C₇-Cycloalkyl;
(d) Phenyl;
(e) substituiertes Phenyl, wie oben definiert;
(f) Heteroaryl, wie oben definiert;
(g) substituiertes-Heteroaryl, wie oben definiert;
(h) Heterocycloalkyl, wie oben definiert;
und
(i) eine Gruppe gewählt aus Option (b) oben, in der der Substituent -M-R⁵ ist, worin M gewählt ist aus der Gruppe bestehend aus:
(aa) -C(O)-NH-;
(bb) -NH-C(O)-;
(cc) -NH-
(dd) -N(CH₃) -
(ee) -O-;
(ff) -S(O)ₙ-, worin n 0, 1 oder 2 ist; und
(gg) -C (=NH) -NH-;
und R⁵ ist gewählt aus der Gruppe bestehend aus:
(aaa) C₁-C₆-Alkyl, wahlweise substituiert mit einem Substituenten, gewählt aus der Gruppe bestehend aus:
(i) Phenyl;
(ii) substituiertes-Phenyl, wie oben definiert;
(iii) Heteroaryl, wie oben definiert;
(iv) substituiertes-Heteroaryl, wie oben definiert;
(bbb) Phenyl;
(ccc) substituiertes-Phenyl, wie oben definiert;
(ddd) Heteroaryl, wie oben definiert;
(eee) substituiertes-Heteroaryl, wie oben definiert; und
(fff) Heterocycloalkyl, wie oben definiert;
oder
irgendein Paar von Substituenten, bestehend aus AB, AD, AE, BD, BE oder DE, wird zusammengenommen mit dem Atom oder den Atomen, an welche sie gebunden sind, um einen 3- bis 7-gliedrigen Ring zu bilden, der wahlweise eine Heterofunktion enthält, bestehend aus
-O-,
- NH-,
- N(C₁-C₆-Alkyl-)-,
-N(Phenyl-C₁-C₆-alkyl-)-,
- N(substituiertes-Phenyl-C₁-C₆-alkyl-)-,
worin substituiertes-Phenyl wie oben definiert ist;
-N(Heteroaryl-C₁-C₆-alkyl-)-,
worin Heteroaryl wie oben definiert ist,
- N(substituiertes-Heteroaryl-C₁-C₆-alkyl-)-,
worin substituierts-Heteroaryl wie oben definiert ist;
- S- oder -S(O)ₙ-,
worin n 1 oder 2 ist;
-C(O)-NH-;
-C(O)-NR⁵,
worin R⁵ wie oben definiert ist;
- NH-C(O)-;
- NR⁵-C(O)-,
worin R⁵ wie oben definiert ist; und
- C(=NH)-NH-;
R¹ ist -O-C₁-C₃-Alkyl; und
R² ist Wasserstoff;
unter den Voraussetzungen, daß, wenn die Verbindung die Formeln (I) oder (III) hat, dann können A, B, D und E nicht alle Wasserstoff sein, D und E können nicht C₁-C₃ Alkyl sein, wenn A und B Wasserstoff sind, noch können D und E Wasserstoff und das andere C₁-C₃-Alkyl sein, wenn A und B Wasserstoff sind;
oder eine Verbindung von Formel (III), in der R¹ = OCH₃ ist, R² ist H, B = E = H, und A und D zusammengenommen sind jeweils -CH₂-N(Cbz)-CH₂- oder -CH₂-N(Benzoyl)-CH₂-;
oder eine Verbindung von Formel (III), in der B=E=H ist, R¹ ist Methoxy, R² ist H; und A und D zusammen sind CH₂-N(R⁷)-CH₂-, worin R⁷ gewählt ist aus der Gruppe bestehend aus:
(i) Phenyl;
(ii) substituiertes-Phenyl, wie oben definiert;
(iii) Heteroaryl, wie oben definiert; und
(iv) substituiertes-Heteroaryl, wie oben definiert;
oder eine Verbindung von Formel (III), in der B=E=H ist, R¹ ist Methoxy, R² ist H; und A und D zusammen sind CH₂-N(-(CH₂)ᵣ-M-(CH₂)ₛ-R⁷)-CH₂-, worin r 0, 1 oder 2 ist, S = O, 1 oder 2, M ist wie oben definiert; und R⁷ ist gewählt aus der Gruppe bestehend aus:
(i) C₁-C₃-Alkyl;
(ii) Phenyl;
(iii) substituiertes-Phenyl wie oben definiert;
(iv) Heteroaryl wie oben definiert; und
(v) substituiertes-Heteroaryl, wie oben definiert;
wobei das Verfahren folgendes umfaßt:
(a) Behandeln einer Verbindung mit der Formel: 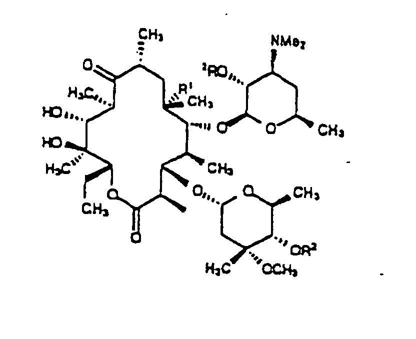 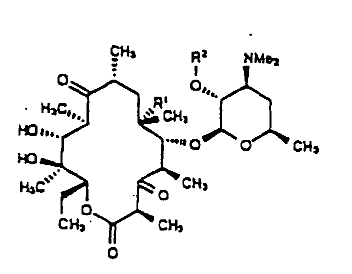 bzw. 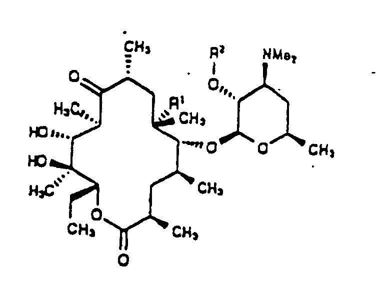 worin R¹ wie oben beschrieben ist und R² ist eine Hydroxyschutzgruppe, mit einer Base, und gefolgt von der Reaktion mit Carbonyldiimidazol in einem aprotischen Lösungsmittel, bei einer Reaktionstemperatur von -20°C bis 70°C, für einen Zeitraum von 0,5 Stunden bis 10 Tage, um erste Intermediatverbindungen herzustellen, die folgende Formeln haben: 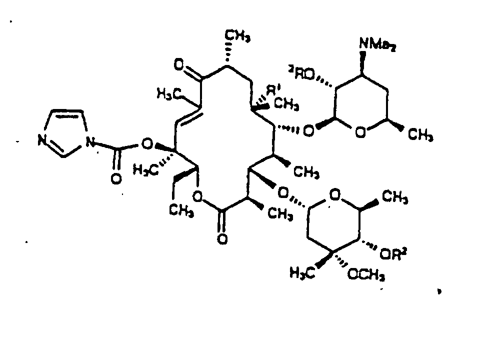 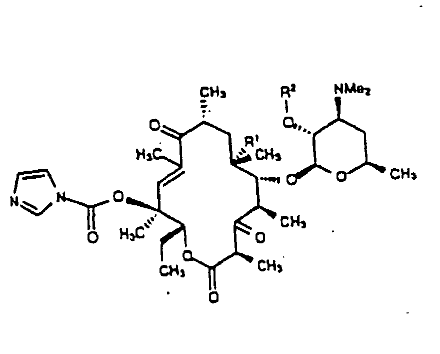 bzw. 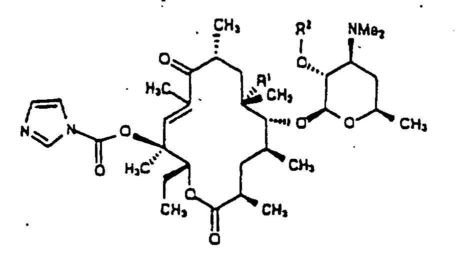 worin R¹ und R² wie oben beschrieben sind;
(b) Reagieren der ersten Intermediatverbindungen mit einer Verbindung mit der Formel: 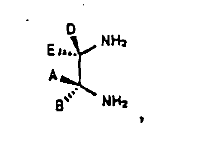 worin A, B, D und E wie oben beschrieben sind, um die bizyklischen zweiten Intermediatverbindungen zu ergeben, die die folgenden Formeln haben: 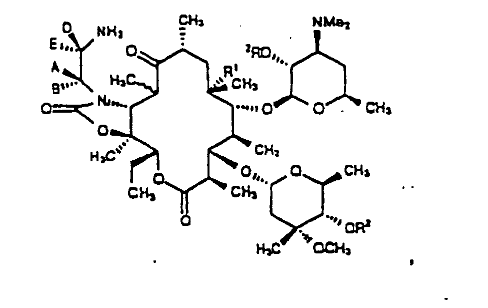 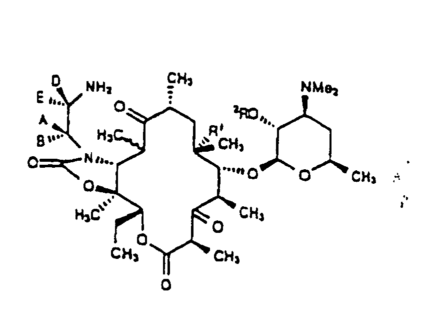 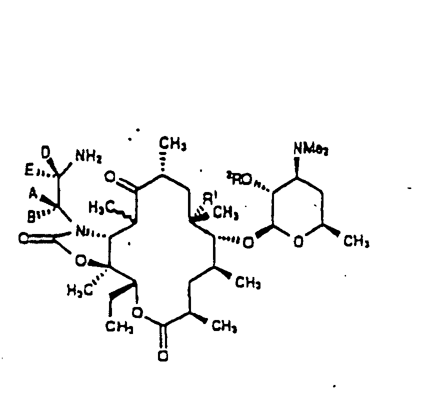 bzw. worin R¹ und R² wie oben beschrieben sind;
(c) Deprotektieren der zweiten Intermediatverbindungen durch Behandlung mit Methanol oder Ethanol, wenn OR² ein Ester ist, oder mit Fluorid in THF oder Acetonitril, wenn R² eine Trialkylsilylgruppe ist, für von 1 bis 24 Stunden, um die dritten Intermediatverbindungen zu ergeben: 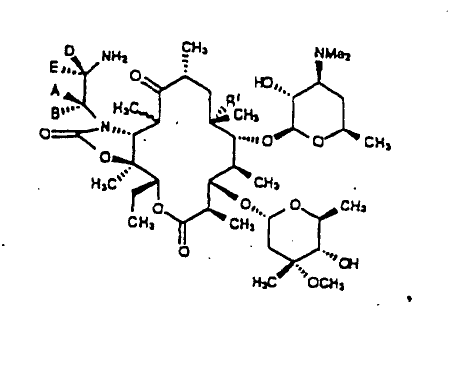 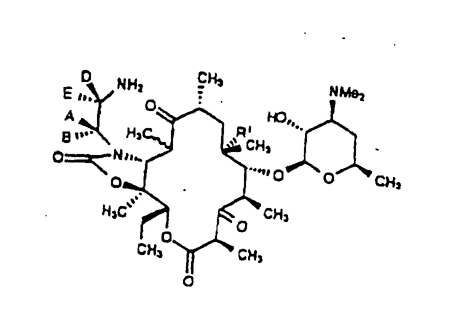 bzw. 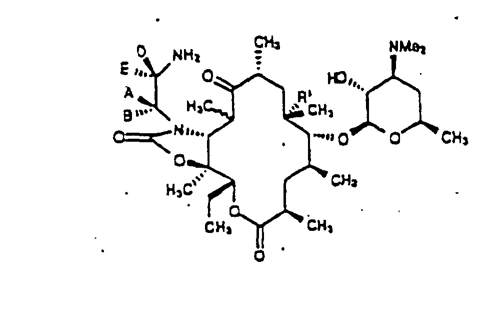
(d) Cyclisieren der dritten Intermediatverbindungen durch Behandlung mit verdünnter Säure, in einem organischen Lösungsmittel für einen Zeitraum von 4 Stunden bis 10 Tagen, um die gewüschten Verbindungen (I), (III) oder (IV) oben zu ergeben.

13. Ein Verfahren gemäß Anspruch 12, worin:
in Schritt (a) das aprotische Lösungsmittel gewählt ist aus der Gruppe bestehend aus Dichlormethan, Chloroform, DMF, Tetrahydrofuran (THF), N-Methylpyrrolidinon oder einer Mischung davon, wobei die Reaktionstemperatur von 0°C bis Reaktionstemperatur ist, und der Zeitraum der Reaktion ist 1-8 Stunden oder bis zu 1-5 Tagen;
in Schritt (b) wird die Reaktion in wässerigem Acetonitril, DMF oder wässerigem DMF durchgeführt;
in Schritt (c) Deprotektieren der zweiten Intermediatverbindungen durch Behandlung mit Methanol oder Ethanol, wenn OR² ein Ester ist, oder mit Fluorid in THF oder Acetonitril, wenn R² eine Trialkylsilylgruppe ist, für von 1 bis 24 Stunden; und
in Schritt (d) Cyclisieren der dritten Intermediatverbindungen durch Behandlung mit verdünnter Essigsäure oder HCl in Ethanol oder Propanol für einen Zeitraum von 4 Stunden bis 10 Tagen, um die gewünschten Verbindungen zu ergeben.

14. Ein Verfahren zur Herstellung von trizyklischen Makrolidderivaten, die folgende Formeln haben: 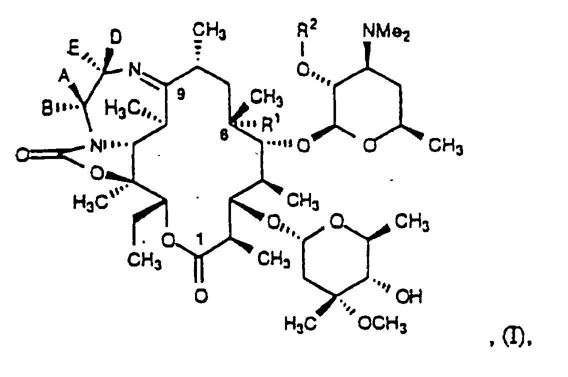 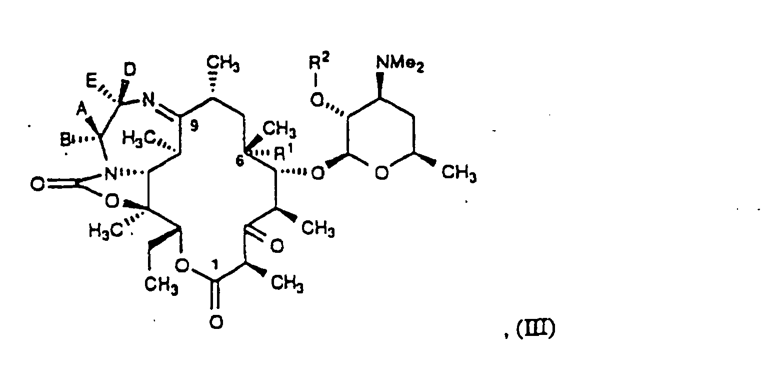 oder 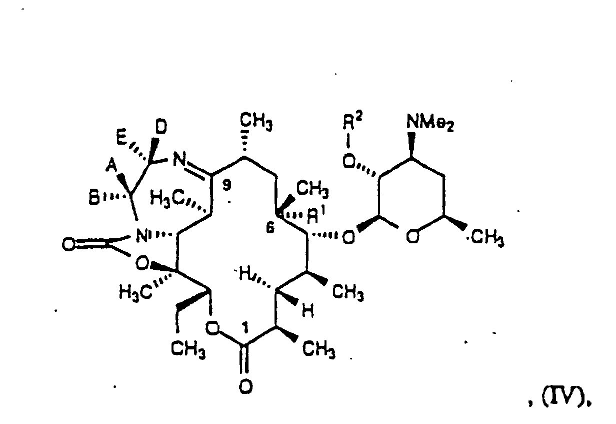 worin
A, B, D und E unabhängig gewählt sind aus der Gruppe bestehend aus:
(a) Wasserstoff;
(b) C₁-C₆-Alkyl, unsubstituiert oder substituiert mit einem Substituenten gewählt aus der Gruppe bestehend aus:
(i) Phenyl;
(ii) Phenyl substituiert mit ein, zwei oder drei Gruppen unabhängig gewählt aus Cl, Br, F, I, OH, C₁-C₃-Alkyl, C₁-C₆-Alkoxy, Methoxymethoxy, Amino, C₁-C₃-Alkyl-amino und di-(C₁-C₃-Alkyl)-amino;
(iii) Heteroaryl gewählt aus Pyridyl, Pyrazinyl, Pyrimidinyl, Pyrrolyl, Pyrazolyl, Imidazolyl, Thiazolyl, Oxazolyl, Isooxazolyl, Thiadiazolyl, Oxadiazolyl, Thiophenyl, Furanyl, Chinolinyl und Isochinolinyl;
(iv) Heteroaryl, wie oben definiert, substituiert mit ein, zwei oder drei Gruppen, unabhängig gewählt aus Cl, Br, F, I, OH, C₁-C₃-Alkyl, C₁-C₆-Alkoxy, Methoxymethoxy, Amino, C₁-C₃-Alkylamino und di-(C₁-C₃-Alkyl)-amino;
(v) Heterocycloalkyl, gewählt aus Pyrrolidinyl, Pyrazolinyl, Pyrazolidinyl, Imidazolinyl, Imidazolidinyl, Piperidinyl, Piperazinyl, Oxazolidinyl, Isoxazolidinyl, Morpholinyl, Thiazolidinyl, Isothiazolidinyl und Tetrahydrofuryl;
(vi) Hydroxy;
(vii) C₁-C₆ Alkoxy;
(viii) Halogen bestehend aus Br, Cl, F oder I; und
(ix) NR³R⁴, worin R³ und R⁴ unmabhängig gewählt sind aus Wasserstoff und C₁-C₆-Alkyl, oder R³ und R⁴ werden mit dem Stickstoffatom genommen, an welches sie gebunden sind, um einen 3- bis 7-gliedrigen Ring zu bilden, der wahlweise eine Heterofunktion enthält, bestehend aus -O-, -NH-, -N(C₁-C₆-Alkyl-)-, -N(Phenyl-C₁-C₆-alkyl-)-, -N(substituiertes-Phenyl-C₁-C₆-alkyl-)-, worin substituiertes Phenyl wie oben definiert ist, -N(Heteroaryl-C₁-C₆-alkyl-)-, worin Heteroaryl wie oben definiert ist; -N(substituiertes-Heteroaryl-C₁-C₆-alkyl-)-, worin substituiertes Heteroaryl wie oben definiert ist, -S- oder -S(O)ₙ-, worin n 1 oder 2 ist;
(c) C₃-C₇-Cycloalkyl;
(d) Phenyl;
(e) substituiertes-Phenyl, wie oben definiert;
(f) Heteroaryl, wie oben definiert;
(g) substituiertes-Heteroaryl, wie oben definiert;
(h) Heterocycloalkyl, wie oben definiert;
und
(i) eine Gruppe gewählt aus Option (b) oben, in der der Substituent -M-R⁵ ist, worin M gewählt ist aus der Gruppe bestehend aus:
(aa) -C(O)-NH-;
(bb) -NH-C(O)-;
(cc) -NH-
(dd) -N(CH₃)-
(ee) -O-;
(ff) -S(O)ₙ-, worin n 0, 1 oder 2 ist; und
(gg) -C(=NH)-NH-;
und R⁵ ist gewählt aus der Gruppe bestehend aus:
(aaa) C₁-C₆-Alkyl, wahlweise substituiert mit einem Substituenten gewählt aus der Gruppe bestehend aus:
(i) Phenyl;
(ii) substituiertes-Phenyl, wie oben definiert;
(iii) Heteroaryl, wie oben definiert;
(iv) substituiertes-Heteroaryl, wie oben definiert;
(bbb) Phenyl;
(ccc) substituiertes-Phenyl, wie oben definiert;
(ddd) Heteroaryl, wie oben definiert;
(eee) substituiertes-Heteroaryl, wie oben definiert; und
(fff) Heterocycloalkyl, wie oben definiert;
oder
irgendein Paar von Substituenten, bestehend aus AB, AD, AE, BD, BE oder DE, wird zusammengenommen mit dem Atom oder den Atomen, an welche sie gebunden sind, um einen 3- bis 7-gliedrigen Ring zu bilden, der wahlweise eine Heterofunktion enthält, bestehend aus
-O-,
- NH-,
-N(C₁-C₆-Alkyl-)-,
-N(Phenyl-C₁-C₆-alkyl-)-,
-N(substituiertes-Phenyl-C₁-C₆-alkyl-)-,
worin substituiertes-Phenyl, wie oben definiert ist,
-N(Heteroaryl-C₁-C₆-alkyl-)-,
worin Heteroaryl wie oben definiert ist,
- N(substituiertes-Heteroaryl-C₁-C₆-alkyl-)-,
worin substituiertes-Heteroaryl wie oben definert ist,
-S- oder -S(O)ₙ-,
worin n 1 oder 2 ist;
-C(O)-NH-;
-C(O)-NR⁵,
worin R⁵ wie oben definiert ist;
-NH-C(O)-;
-NR⁵-C(O)-,
worin R⁵ wie oben definiert ist; und
-C(=NH)-NH-;
R¹ ist -O-C₁-C₃-Alkyl; und
R² ist Wasserstoff;
unter den Voraussetzungen, daß, wenn die Verbindung die Formeln (I) oder (III) hat, dann können A, B, D und E nicht alle Wasserstoff sein, D und E können nicht C₁-C₃-Alkyl sein, wenn A und B Wasserstoff sind, noch kann eines von D und E Wasserstoff und das andere C₁-C₃-Alkyl sein, wenn A und B Wasserstoff sind;
oder eine Verbindung von Formel (III), in der R¹ = OCH₃ ist, R² ist H, B = E = H, und A und D zusammengenommen sind entweder -CH₂-N(Cbz)-CH₂- oder -CH₂-N(Benzoyl)-CH₂-;
oder eine Verbindung von Formel (III), in der B=E=H ist, R¹ ist Methoxy, R² ist H; und A und D zusammen sind CH₂-N(R⁷)-CH₂-, worin R⁷ gewählt ist aus der Gruppe bestehend aus:
(i) Phenyl;
(ii) substituiertes-Phenyl, wie oben definiert;
(iii) Heteroaryl, wie oben definiert; und
(iv) substituiertes-Heteroaryl, wie oben definiert;
oder eine Verbindung von Formel (III), in der B=E=H ist, R¹ ist Methoxy, R² ist H; und A und D zusammen sind CH₂-N(-(CH₂)ᵣ-M-(CH₂)ₛ-R⁷)-CH₂-, worin r 0, 1 oder 2 ist, s = O, 1 oder 2, M ist wie oben definiert; und R⁷ ist gewählt aus der Gruppe bestehend aus:
(i) C₁-C₃-Alkyl;
(ii) Phenyl;
(iii) substituiertes-Phenyl, wie oben definiert;
(iv) Heteroaryl, wie oben definiert; und
(v) substituiertes-Heteroaryl, wie oben definiert;
wobei das Verfahren folgendes umfaßt:
(a) Behandeln einer Verbindung mit der Formel: 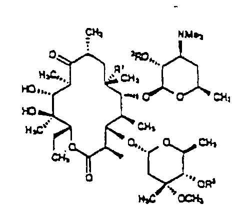 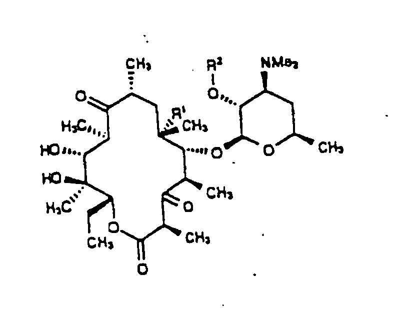 bzw. 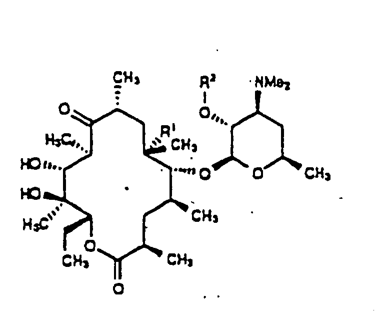 worin R¹ wie oben beschrieben ist, und R² ist eine Hydroxyschutzgruppe, mit einer Base, und gefolgt von der Reaktion mit Carbonyldiimidazol, in einem aprotischen Lösungsmittel, bei einer Reaktionstemperatur von -20°C bis 70°C, für einen Zeitraum von 0,5 bis 24 Stunden, um erste Intermediatverbindungen herzustellen, die folgende Formeln haben: 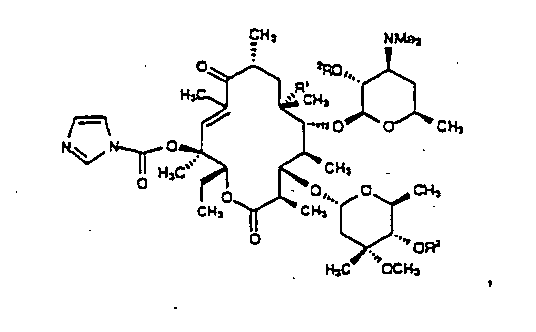 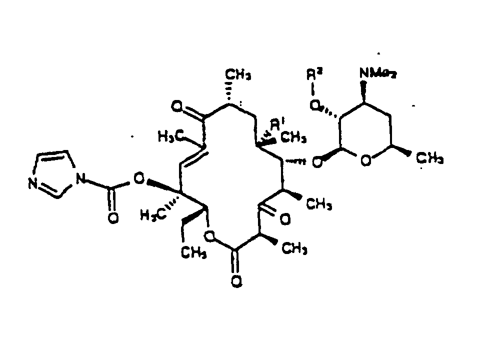 bzw. 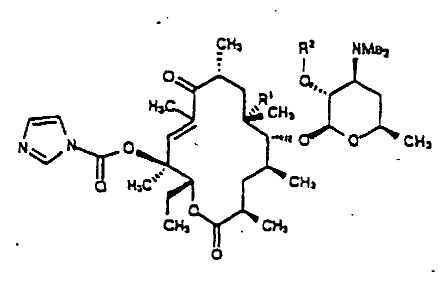 worin R¹ und R² wie oben beschrieben sind;
(b) Reagieren der ersten Intermediatverbindungen mit einer Verbindung mit der Formel: 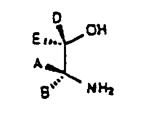 worin A, B, D und E wie oben beschrieben sind, um die bizyklischen zweiten Intermediatverbindungen zu ergeben: 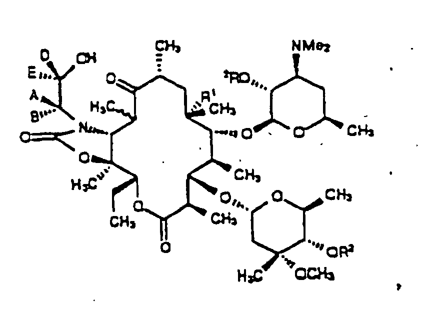 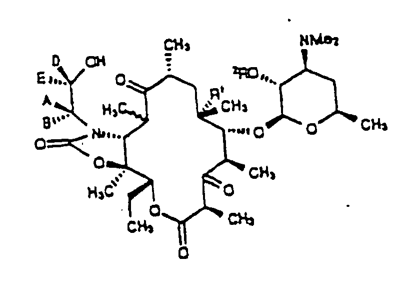 bzw. 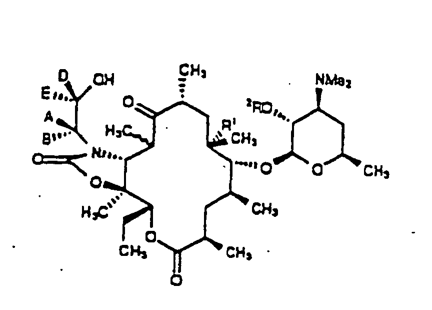
(c) Reagieren der Hydroxygruppe der bizyklischen zweiten Intermediatverbindungen durch Behandlung mit Triphenylphosphin und Diphenylphosphorylazid in Tetrahydrofuran, unter Mitsunobu-Reaktionsbedingungen, um die dritten Intermediatverbindungen herzustellen: 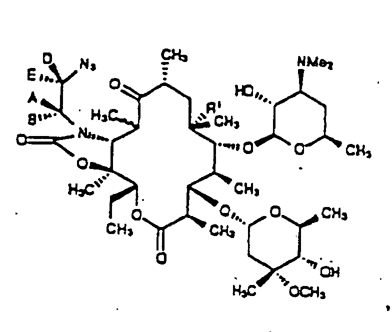 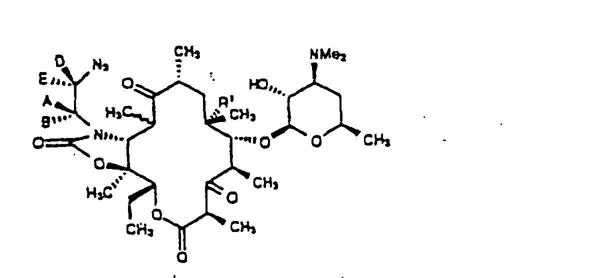 bzw. 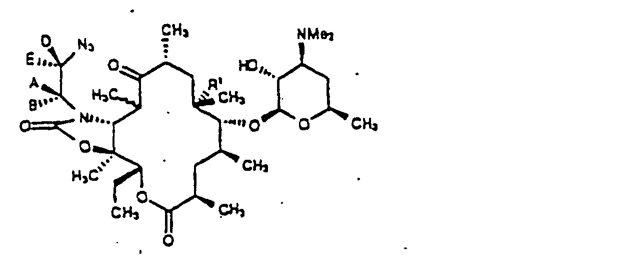
(d) Reduzieren der dritten Intermediatverbindungen, die eine Azidogruppe haben, mit Reduktionsmitteln, um die vierten Intermediatverbindungen herzustellen, die die folgenden Formeln haben: 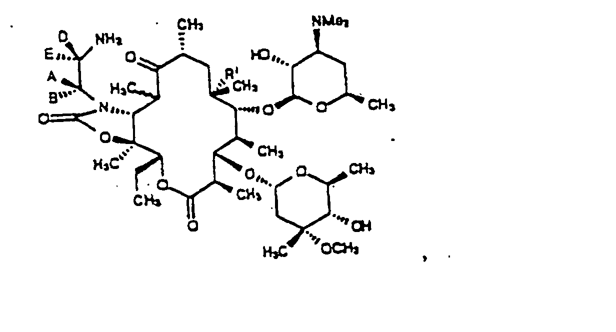 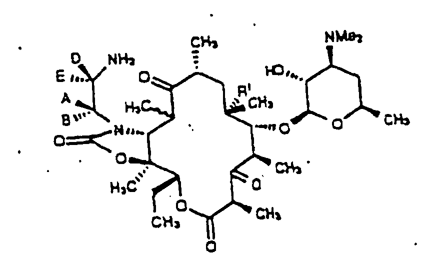 bzw. 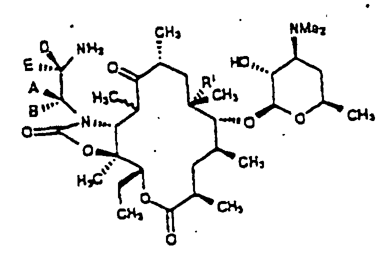 und
(e) Cyclisieren der vierten Intermediatverbindungen durch Behandlung mit einer verdünnten Säure, in einem organischen Lösungsmittel, für einen Zeitraum von 4 Stunden bis 10 Tagen, um die gewünschten Verbindungen der Formeln (I), (III) oder (IV) zu ergeben.

15. Das Verfahren wie in Anspruch 14, worin:
in Schritt (a) die Base Natriumhydrid, Lithiumhydrid oder Kaliumcarbonat ist, das aprotische Lösungsmittel Dichlormethan, Chloroform, DMF, Tetrahydrofuran (THF), N-Methylpyrrolidinon oder eine Mischung davon ist, die Reaktionstemperatur von 0°C bis Reaktionstemperatur ist und der Reaktionszeitraum von 1-8 Stunden ist;
in Schritt (b) ist das Lösungsmittel wässeriges Acetonitril, DMF oder wässeriges DMF;
in Schritt (c) sind die Reagenzien Triphenylphosphin und Diphenylphosphorylazid-DEAD in Tetrahydrofuran, unter Mitsunobu-Reaktionsbedingungen;
in Schritt (d) ist das Reduktionsmittel Triphenylphosphin-Wasser, Wasserstoff mit einem Katalysator, Natriumborhydrid oder Dialkylaluminiumhydrid;
in Schritt (e) Cyclisieren der dritten Intermediatverbindungen durch Behandlung mit verdünnter Essigsäure oder HCl in Ethanol oder Propanol für einen Zeitraum von 4 Stunden bis 10 Tagen, um die gewünschten Verbindungen zu ergeben.

16. Das Verfahren wie in Anspruch 14, worin:
Schritt (c) ersetzt wird durch eine Zwei-Schritt-Sequenz, die folgendes umfaßt: (1) Reagieren der Hydroxygruppe der bizyklischen zweiten Intermediatverbindungen davon mit einem Alkyl oder Arylsulfonylchlorid, einem Alkyl- oder Arylsulfonsäureanhydrid oder Trifluormethansulfonsäureanhydrid in einem aprotischen Lösungsmittel bei -78°C bis Reaktionstemperatur, um das entsprechende Sulfonat zu ergeben, und (2) Reagieren des Sulfonats mit Lithiumazid oder Natriumazid in einem aprotischen Lösungsmittel bei 0°C bis 100°C, um die dritte Intermediatverbindung zu ergeben.

## Revendications

1. Composé choisi dans le groupe répondant aux formules : 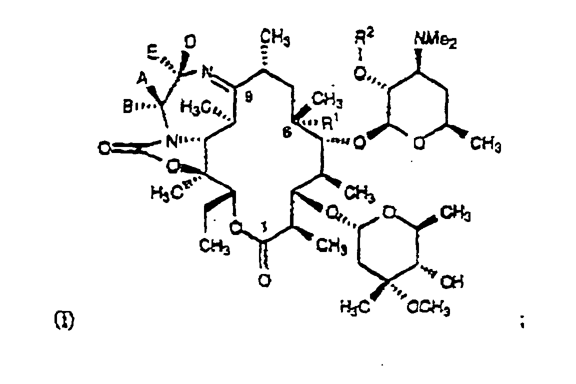 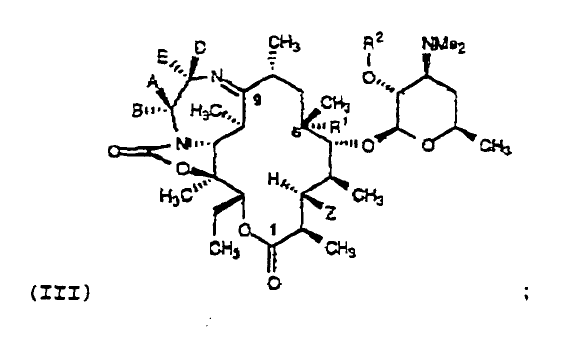 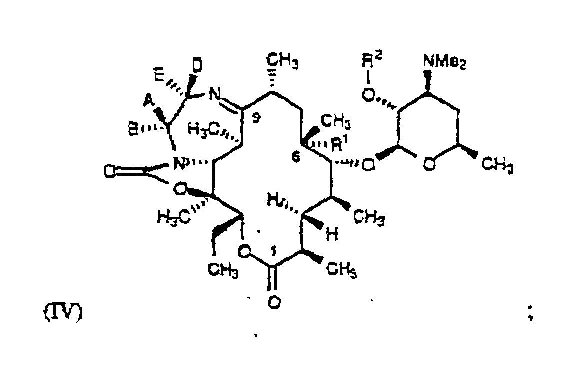 de même que ses sels et esters pharmaceutiquement acceptables dans lesquels :
A, B, D et E sont indépendamment choisis dans le groupe constitué par :
(a) un atome d'hydrogène ;
(b) un alkyle en C₁ à C₆, non substitué ou substitué par un substituant choisi dans le groupe constitué par :
(i) le phényle ;
(ii) un phényle substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di-(alkyl en C₁ à C₃)-amino ;
(iii) un hétéroaryle choisi parmi le pyridyle, le pyrazinyle, le pyrimidinyle, le pyrrolyle, le pyrazolyle, l'imidazolyle, le thiazolyle, l'oxazolyle, l'isooxasolyle, le thiadiazolyle, l'oxadiazolyle, le thiophényle, le furanyle, le quinolinyle et l'isoquinolinyle ;
(iv) un hétéroaryle tel que défini ci-dessus substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyle en C₁ à C₃)-amino et un di- (alkyl en C₁ à C₃)-amino ;
(v) un hétérecycloalkyle choisi parmi le pyrrolidinyle, le pyrazolinyle, le pyrazolidinyle, l'imidazolinyle, l'imidazolidinyle, le pipéridinyle, le pipérazinyle, l'oxazolidinyle, l'isoxazolidinyle, le morpholinyle, le thiazolidinyle, l'isothiazolidinyle et le tétrahydrofuryle ;
(vi) l'hydroxy ;
(vii) un alcoxy en C₁ à C₆
(viii)un atome d'halogène constitué par Br, Cl, F ou I ; et
(ix) NR³R⁴, dans lequel R³ et R⁴ sont indépendamment choisis parmi un atome d'hydrogène et un alkyle en C₁ à C₆, ou R³ et R⁴ sont pris avec l'atome d'azote auquel ils sont reliés pour former un cycle à 3 à 7 chaînons contenant facultativement une fonction hétéro constituée par -O-, -NH-, -N(alkyle en C₁ à C₆)-, - N(phényl-alkyle en C₁ à C₆)-, -N(phényl substitué-alkyle en C₁ à C₆)- dans lequel le phényle substitué est tel que défini ci-dessus, -N(hétéroaryl-alkyle en C₁ à C₆)- dans lequel l'hétéroaryle est tel que défini ci-dessus, N-(hétéroaryl substitué-alkyle en C₁ à C₆)- dans lequel l'hétéroaryle substitué est tel que défini ci-dessus, -S- ou -S(O)ₙ-, dans lequel n vaut 1 ou 2 ;
(c) un cycloalkyle en C₃ à C₇ ;
(d) le phényle ;
(e) un phényle substitué tel que défini ci-dessus ;
(f) un hétéroaryle tel que défini ci-dessus ;
(g) un hétéroaryle substitué tel que défini ci-dessus ;
(h) un hétérocycloalkyle tel que défini ci-dessus ; et
(i) un groupe choisi parmi l'option (b) ci-dessus dans lequel le substituant est
-M-R⁵, dans lequel M est choisi dans le groupe constitué par :
(aa) -C(O)-NH- ;
(bb) -NH-C(O)- ;
(cc) -NH-
(dd) -N(CH₃)-
(ee) -O- ;
(ff) -S(O)ₙ-, dans lequel n vaut 0, 1 ou 2 ; et
(gg) -C(=NH)-NH- ;
et R⁵ est choisi dans le groupe constitué par :
(aaa) un alkyle en C₁ à C₆, facultativement substitué par un substituant choisi dans le groupe constitué par :
(i) le phényle ;
(ii) un phényle substitué tel que défini ci-dessus ;
(iii) un hétéroaryle tel que défini ci-dessus ;
(iv) un hétéroaryle substitué tel que défini ci-dessus ;
(bbb) le phényle ;
(ccc) un phényle substitué tel que défini ci-dessus ;
(ddd) un hétéroaryle tel que défini ci-dessus ;
(eee) un hétéroaryle substitué tel que défini ci-dessus ; et
(fff) un hétérocycloalkyle tel que défini ci-dessus ; ou
toute paire de substituants, constituée par AB, AD, AE, BD, BE ou DE, est prise conjointement avec l'atome ou les atomes auxquels elles sont attachées pour former un cycle à 3 à 7 chaînons contenant facultativement une fonction hétéro constituée par
-O-,
-NH-,
-N(alkyl en C₁ à C₆),
-N(phényl-alkyl en C₁ à C₆),
-N(phényl substitué-alkyle en C₁ à C₆)-
dans lequel le phényle substitué est tel que défini ci-dessus,
- N (hétéroaryl-alkyle en C₁ à C₆)
dans lequel l'hétéroaryle est tel que défini ci-dessus,
- N(hétéroaryl substitué-alkyle en C₁ à C₆)-
dans lequel l'hétéroaryle substitué est tel que défini ci-dessus,
- S- ou S(O)ₙ-,
dans lequel n vaut 1 ou 2 ;
-C(O)-NH- ;
- C(O)-NR⁵-,
dans lequel R⁵ est tel que défini ci-dessus ;
-NH-C(O)- ;
-NR⁵-C(O)-,
dans lequel R⁵ tel que défini ci-dessus ; et
-C(=NH)-NH- ;
R¹ représente -O-alkyle en C₁ à C₃ ; et
R² représente un atome d'hydrogène ;
à condition que lorsque le composé est de Formule (I) ou (III), alors A, B, D et E ne peuvent pas tous représenter un atome d'hydrogène, D et E ne peuvent pas représenter un alkyle en C₁ à C₃ lorsque A et B représentent un atome d'hydrogène, de même l'un de D et E ne peut pas représenter un atome d'hydrogène et l'autre représenter un alkyle en C₁ à C₃ lorsque A et B représentent un atome d'hydrogène ;
ou un composé de Formule (III) dans lequel R¹=OCH₃, R² représente H, B=E=H, et A et D pris ensemble représentent soit -CH₂-N(Cbz)-CH₂- soit -CH₂-N(benzoyl) -CH₂- ;
ou un composé de Formule (III) dans lequel B=E=H, R¹ représente le méthoxy, R² représente H, et A et D ensemble représentent CH₂-N(R⁷)-CH₂-, dans lequel R⁷ est choisi dans le groupe constitué par :
(i) le phényle ;
(ii) un phényle substitué tel que défini ci-dessus ;
(iii) un hétéroaryle tel que défini ci-dessus ; et
(iv) un hétéroaryle substitué tel que défini ci-dessus ;
ou un composé de Formule (III) dans lequel B=E=H, R¹ représente le méthoxy, R² représente H ; et A et D ensemble représentent CH₂-N(-(CH₂)ᵣ-M-(CH₂)ₛ-R⁷)-CH₂-, dans lequel r vaut 0, 1 ou 2 ; s = 0, 1 ou 2, M est tel que défini ci-dessus ; et R⁷ est choisi dans le groupe constitué par :
(i) un alkyle en C₁ à C₃ ;
(ii) le phényle ;
(iii) un phényle substitué tel que défini ci-dessus ;
(iv) un hétéroaryle tel que défini ci-dessus ; et
(v) un hétéroaryle substitué tel que défini ci-dessus.

2. Composé selon la revendication 1, répondant à la formule : 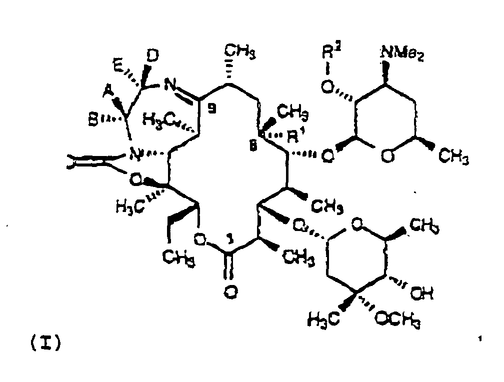 dans laquelle A, B, D, E
et R¹ à R⁵ sont tels que décrits ici.

3. Composé selon la revendication 1, répondant à la formule : 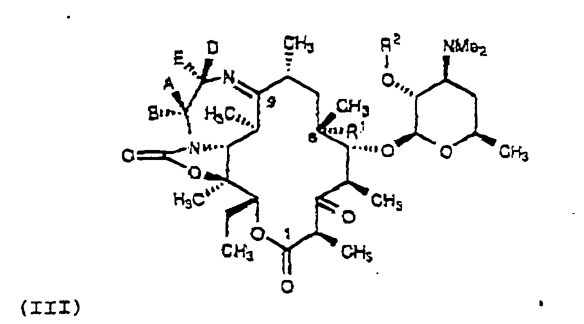 dans laquelle A, B, D, E et R¹ à R⁵ sont tels que décrits ici.

4. Composé selon la revendication 1, répondant à la formule : 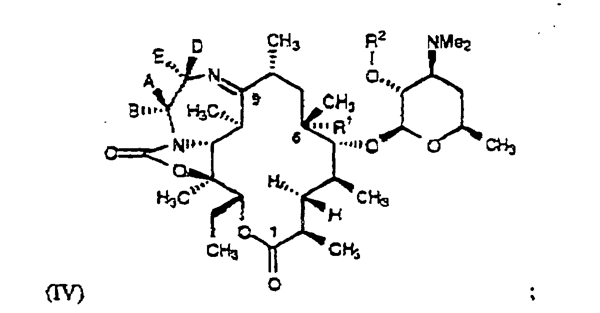 dans laquelle A, B, D, E et R¹ à R⁵ sont tels que décrits ici.

5. Composé selon la revendication 3, dans lequel R¹ ,représente le méthoxy, et A, B, D, E et R¹ à R⁵ sont tels que décrits ici.

6. Composé selon la revendication 5, dans lequel trois quelconques des groupes A, B, D et E représente un atome d'hydrogène et l'autre groupe est choisi parmi un groupe alkyle en C₁ à C₆ une seule fois substitué comprenant -(CH₂)ₘR⁶ où m = 1, 2, 3 ou et R⁶ est choisi dans le groupe constitué par :
(a) le phényle ;
(b) un phényle substitué par un, deux ou trois groupes indépendamment choisis parmi Cl, Br, F, I, OH, un alkyle (en C₁ à C₃, un alcoxy en C₁ à C₆, le méthoxyméthoxy, un amino, un (alkyl en C₁ à C₃) -amino et un di- (alkyl en C₁ à C₃)-amino ;
(c) un hétéroaryle choisi parmi le pyridyle, le pyrazinyle, le pyrimidinyle, le pyrrolyle, le pyrazolyle, l'imidazolyle, le thiazolyle, l'oxazolyle, l'isooxazolyle, le thiadiazolyle, l'oxadiazolyle, le thiophényle, le furanyle, le quinolinyle et l'isoquinolinyle ;
(d) un hétéroaryle tel que défini ci-dessus substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di- (alkyl en C₁ à C₃)-amino ;
(e) un hétérocycloalkyle choisi parmi le pyrrolidinyle, le pyrazolinyle, le pyrazolidinyle, l'imidazolinyle, l'imidazolidinyle, le pipéridinyle, le pipérazinyle, l'oxazolidinyle, l'isoxazolidinyle, le morpholinyle, le thiazolidinyle, l'isothiazolidinyle et le tétrahydrofuryle ;
(f) un hydroxy ;
(g) un alcoxy en C₁ à C₆ ;
(h) NR³R⁴, dans lequel R³ et R⁴ sont indépendamment choisis parmi un atome d'hydrogène, et un alkyle en C₁ à C₆, ou R³ et R⁴ sont pris avec l'atome d'azote auquel ils sont reliés pour former un cycle à 3 à 7 chaînons contenant facultativement une fonction hétéro constituée par -O-, -NH-, -N(alkyl en C₁ à C₆)-, -N(phényl-alkyl en C₁ à C₆)-, -N(phényl substitué-alkyl en C₁ à C₆)- dans lequel le phényle substitué est tel que défini ci-dessus, -N(hétéroaryl-alkyle en C₁ à C₆)- dans lequel l'hétéroaryle est tel que défini ci-dessus, (hétéroaryl substitué-alkyle en C₁ à C₆) - dans lequel l'hétéroaryle substitué est tel que défini ci-dessus, -S-ou -S(O)ₙ-, dans lequel n vaut 1 ou 2 ;
(i) un atome d'halogène constitué par Br, Cl, F ou I ;
(j) un alkyle en C₁ à C₃ ; ou
(k) (CH₂)ᵣ-M-(CH₂)ₛ-R⁷ dans lequel r = 0, 1 ou 2 ; s= 0, 1 ou 2 et M est choisi dans le groupe constitué par :
(aa) -C(O)-NH- ;
(bb) -NH-C(O)- ;
(cc) -NH-
(dd) -N(CH₃)-
(ee) -O- ;
(ff) -S(O)ₙ-, dans lequel n vaut 0, 1 ou 2 ; et
(gg) -C(=NH)-NH- ;
et R⁷ est choisi dans le groupe constitué par :
(aaa) un alkyle en C₁ à C₃ ;
(bbb) le phényle ;
(ccc) un phényle substitué tel que défini ci-dessus ;
(ddd) un hétéroaryle tel que défini ci-dessus ; et
(eee) un hétéroaryle substitué tel que défini ci-dessus.

7. Composé selon la revendication 5, dans lequel B=E=H, et A et D ensemble sont choisis dans le groupe constitué par :
(a) -CH₂-Z-CH₂-, dans lequel Z est choisi dans le groupe constitué par :
(aa) -C(O)-NH ;
(bb) -C(O)-NR⁵-, dans lequel R⁵ est choisi dans le groupe constitué par :
(aaa) un alkyle en C₁ à C₆, non substitué ou substitué par un substituant choisi dans le groupe constitué par :
(i) le phényle ;
(ii) un phényle substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di- (alkyl en C₁ à C₃)-amino ;
(iii) un hétéroaryle choisi parmi le pyridyle, le pyrazinyle, le pyrimidinyle, le pyrrolyle, le pyrazolyle, l'imidazolyle, le thiazolyle, l'oxazolyle, l'isooxazolyle, le thiadiazolyle, l'oxadiazolyle, le thiophényle, le furanyle, le quinolinyle et l'isoquinolinyle ; et
(iv) un hétéroaryle tel que défini ci-dessus substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di-(alkyl en C₁ à C₃)-amino ;
(bbb) le phényle
(cccc) un phényle substitué tel que défini ci-dessus ;
(ddd) un hétéroaryle tel que défini ci-dessus ;
(eee) un hétéroaryle substitué tel que défini ci-dessus ; et
(fff) un hétérocycloalkyle choisi parmi le pyrrolidinyle, le pyrazolinyle, le pyrazolidinyle, l'imidazolinyle, l'imidazolidinyle, le pipéridinyle, le pipérazinyle, l'oxazolidinyle, l'isoxazolidinyle, le morpholinyle, le thiazolidinyle, l'isothiazolidinyle et le tétrahydrofuryle ;
(cc) -NH-C(O)- ;
(dd) -NR⁵-C(O)-, dans lequel R⁵ est tel que défini ci-dessus
(ee) -NH- ;
(ff) -N(CH₃)- ;
(gg) -O- ;
(hh) -S(O)ₙ-, lequel n vaut 0, 1 ou 2 ; et
(ii) -C(=NH)-NH- ;
(b) -CH₂-N(-(CH₂)ₛ-R⁷)-CH₂-, dans lequel s = 0, 1 ou 2, et R⁷ est choisi dans le groupe constitué par :
(aa) un alkyle en C₁ à C₃ ;
(bb) le phényle ;
(cc) un phényle substitué tel que défini ci-dessus ;
(dd) un hétéroaryle tel que défini ci-dessus ; et
(ce) un hétéroaryle substitué tel que défini ci-dessus ;
(c) -CH₂-N(-(CH₂)ᵣ-M-(CH₂)₃-R⁷)-CH₂-, dans lequel r vaut 0, 1 ou 2 ; s = 0, 1 ou 2, R⁷ est tel que défini ci-dessus et M est choisi dans le groupe constitué par :
(i) -C(O)-NH- ;
(ii) -NH-C(O)- ;
(iii) -NH-
(iv) -N(CH₃)-
(v) -O- ;
(vi) -S(O)ₙ-, dans lequel n vaut 0,1 ou 2 ; et
(d) -CH₂-(CH₂)ᵣ-CH₂-, dans lequel r vaut 0, 1 ou 2.

8. Composé selon la revendication 1, qui est :
le composé de formule (IV) ; R¹=méthoxy ; R²=hydrogène ; A=B=D=E=hydrogène ;
le composé de formule (III), A=B=E=H, D=benzyle, R¹=méthoxy, R²=hydrogène ;
le composé de formule (III), A=B=D=H, E=benzyle, R¹=méthoxy, R²=hydrogène ;
le composé de formule (III) ; A=benzyle, B=D=E=H, R¹=méthoxy, R²=hydrogène ;
le composé de formule (III) ; B=benzyle, A=D=E=H, R¹=méthoxy, R²=hydrogène ;
le composé de formule (III) ; A=E=phényle, B=D=H, R¹=méthoxy, R²=hydrogène ;
le composé de formule (III) ; A=méthyle, B=D=E=H, R¹=méthoxy, R²=hydrogène ;
le composé de formule (III) ; B=méthyle, A=D=E=H, R¹=méthoxy, R²=hydrogène ;
le composé de formule (III) ; A=D=méthyle ; B=E=H, R¹=méthoxy, R²=hydrogène ;
le composé de formule (III) ; A=E=méthyle ; B=D=H, R¹=méthoxy, R²=hydrogène ;
le composé de formule (III) ; B=D=H ; A et E pris ensemble représentent -CH₂CR₂CH₂-, R¹=méthoxy, R²=hydrogène ;
le composé de formule (III) ; B=E=H ; A et D pris ensemble représentent -CH₂CH₂CH₂CH₂-, R¹=méthoxy, R²=hydrogène ;
le composé de formule (III) : R¹=OCH₃, R²=H ; A=B=D=H ; E=-CH₂NH₂ ;
le composé de formule (III) : R¹=OCH₃, R²=H, A=B=E=H ; D=-CH₂NH₂ ;
le composé de formule (III) : R¹=OCH₃, R²=H ; B=E=H ; A et D pris ensemble sont -CH₂CH₂CH₂- ;
le composé de formule (III) : R¹=OCH₃, R²=H ; B=E=H ; A et D pris ensemble sont -CH₂OCH₂- ;
le composé de formule (III) : R¹=OCH₃, R²=H ; B=E=H ; A et D pris ensemble sont -CH₂-NH-CH₂- ;
le composé de formule (III) : R¹=OCH₃, R²=H ; B=E=H ; A et D pris ensemble représentent -CH₂-N(Cbz)-CH₂- ;
le composé de formule (III) : R¹=OCH₃, R²=H ; B=E=H ; A et D pris ensemble représentent -CH₂-N(benzyl)-CH₂- ;
le composé de formule (III) : R¹=OCH₃, R²=H ; B=E=H ; A et D pris ensemble représentent -CH₂-N(benzoyl)-CH₂- ;
le composé de formule (III) : R¹=OCH₃, R²=H ; B=E=H ; A et D pris ensemble représentent -CH₂-N(phényl-CH₂-CH₂-)-CH₂- ;
le composé de formule (III) : R¹=OCH₃, R²=H ; B=E=H ; A et D pris ensemble représentent -CH₂-N(4-Cl-phényl-CH₂-)-CH₂- ;
le composé de formule (III) : R¹=OCH₃, R²=H ; B=E=H ; A et D pris ensemble représentent -CH₂-N(4-pyridyl-CH₂-)-CH₂- ;
le composé de formule (III) : R¹=OCH₃, R²=H ; B=E=H ; A et D pris ensemble représentent -CH₂-N(2-pyridyl-CH₂-)-CH₂- ;
le composé de formule (III) : R¹=OCH₃ , R²=H ; B=E=H ; A et D pris ensemble représentent -CH₂-N(₃-pyridyl-CH₂-)-CH₂- ;
le composé de formule (III) : R¹=OCH₃, R²=H ; B=E=H ; A et D pris ensemble représentent -CH₂-N(4-quinolyl-CH₂)-)CH₂- ;
le composé de formule (III) : R¹=OCH₃, R²=H ; B=E=H ; A=D=-CH₂-O-CH₂-phényle ;
le composé de formule (III) : R²=OCH₃, R²=H ; B=E=H ; A=D=-CH₂-OH ;
le composé de formule (III) : R¹=OCH₃, R²=H ; B=E=H ; A=D=-CH₂-O-phényle ;
le composé de formule (U) : R¹=OCH₃, R²=H ; A=B=H ; D et E pris ensemble représentent -CH₂-CH₂-CH₂-CH₂- ;
le composé de formule (III) : R¹=OCH₃, R²=H ; A et B pris ensemble représentent -CH₂-CH₂-CH₂-CH₂- ; D=E=H ;
le composé de formule (III) : R¹=OCH₃, R²=H ; A=B=H ; D et E pris ensemble représentent -CH₂-O-CH₂- ;
le composé de formule (III) : R¹=OCH₃, R²=H ; A=D=E=H ; B=-CH₂-CH₂-phényle ;
le composé de formule (III) : R¹=OCH₃, R²=H ; A=D=E=H ; B=CH₂-CH₂-CH₂-phényle ;
le composé de formule (III) : R¹=OCH₃, R²=H ; A=D=E=H ; B=-CH₂-O-CH₂-phényle ;
le composé de formule (III) : R¹=OCH₃, R²=H ; A=D=E=H ; B=-CH₂-CH₂-(4-OCH₃-phényle) ;
le composé de formule (III) : R¹=OCH₃, R²=H ; A=-CH₂-CH₂-phényle ; B=D=E=H ;
le composé de formule (III) : R¹=OCH₃, R²=H ; A=-CH₂-CH₂-CH₂-phényle ; B=D=E=H ;
le composé de formule (III) : R¹=OCH₃, R²=H ; A=-CH₂-O-CH₂-phényle, B=D=E=H ;
le composé de formule (III) : R¹=OCH₃, R²=H ; B=D=E=H ; A=-CH₂-CH₂-(4-OCH₃-phényle) ;
le composé de formule (III) : R²=OCH₃, R²=H ; A=B=D=H ; E=-CH₂-CH₂-Ph ;
le composé de formule (III) : R¹=OCH₃, R²=H ; A=B=E=H ; D=-CH₂-CH₂-Ph ;
le composé de formule (III) : R¹=OCH₃, R²=H ; A=B=D=H ; E=-CH₂-CH₂-CH₂-Ph ;
le composé de formule (III) : R¹=OCH₃, R²=H ; A=B=E=H ; D=-CH₂-CH₂-CH₂-Ph ;
le composé de formule (III) : R¹=OCH₃, R²=H ; A= -CH₂-CH₂-OPh ; B=D=E=H ;
le composé de formule (III) : R¹=OCH₃, R² =H ; A= -CH₂-CH₂-NH₂ ; B=D=E=H ;
le composé de formule (III) : R¹=OCH₃, R² =H ; A= -CH₂-CH₂-OH ; B=D=E=H ;
le composé de formule (III) : R¹=OCH₃, R²=H ; A=-CH₂-CH₂-NH(4-pyridyle-) ; B=D=E=H
le composé de formule (III) : R¹=OCH₃, R²=H ; A=B=D=H ; E=-CH₂-OH ;
le composé de formule (III) : R¹=OCH₃, R²=H ; A=B=E=H ; D=-CH₂-OH ;
le composé de formule (III) : R¹=OCH₃, R²=H ; A=B=E=H ; D=-CH₂-NHbenzoyle ;
le composé de formule (III) : R¹=OCH₃, R²=H ; A=B=E=H ; D=-CH₂-NHbenzoyle ;
le composé de formule (III) : R¹=OCH₃, R²=H ; A=B=D=H ; E=-CH₂-NHbenzoyle ;
le composé de formule (III) : R¹=OCH₃, R²=H ; A=B=D=H ; E=-CH₂-NHbenzoyle ;
le composé de formule (III) : R¹=OCH₃, R²=H ; B=D=H ; A=E=-CH₂OCH₂(4-Cl-phényle) ;
le composé de formule (III) : R¹=OCH₃, R²=H ; A=B=E=H ; D=-CH₂-N(CH₃)-benzyle ;
le composé de formule (III) : R¹=OCH₃, R²=H ; A=B=D=H ; E=-CH₂-N(CH₃)-benzyle ;
le composé de formule (III) : R¹=OCH₃, R²=H ; A=B=D=H ; E=-CH₂-NH-phényle ;
ou
le composé de formule (III) : R¹=OCH₃, R²=H ; A=B=E=H ; D=-CH₂-NH-phényle.

9. Composé selon la revendication 1, qui est :
le composé de formule (IV) ; R¹=méthoxy ; R²=hydrogène ; A=B=D=E=hydrogène ;
le composé de formule (III), A=B=E=H, D=benzyle, R¹=méthoxy, R²=hydrogène ;
le composé de formule (III), A=B=D=H, E=benzyle, R¹=méthoxy, R²=hydrogène ;
le composé de formule (III) ; A=benzyle, B=D=E=H, R¹=méthoxy, R²=hydrogène ;
le composé de formule (III) ; B=benzyle, A=D=E=H, R¹=méthoxy, R²=hydrogène ;
le composé de formule (III) ; A=E=phényle, B=D=H, R¹=méthoxy, R²=hydrogène ;
le composé de formule (III) ; A=méthyle, B=D=E=H, R¹=méthoxy, R²=hydrogène ;
le composé de formule (III) ; B=méthyle, A=D=E=H, R¹=méthoxy, R²=hydrogène ;
le composé de formule (III) ; A=D=méthyle ; B=E=H, R¹=méthoxy, R²=hydrogène ;
le composé de formule (III) ; A=E=méthyle ; B=D=H, R¹=méthoxy, R²=hydrogène ;
le composé de formule (III) ; B=D=H ; A et E pris ensemble représentent -CH₂CH₂CH₂- R¹ =méthoxy, R²=hydrogène ; ou
le composé de formule (III) ; B=E=H ; A et D pris ensemble représentent -CH₂CH₂CH₂CH₂- R¹=méthoxy, R²=hydrogène.

10. Composition pharmaceutique comprenant une quantité thérapeutiquement efficace d'un composé selon l'une quelconque des revendications 1 à 9 en combinaison avec un véhicule pharmaceutiquement acceptable.

11. Utilisation d'un composé pharmaceutique selon l'une quelconque des revendications 1 à 9, pour préparer un médicament destiné à maîtriser une infection bactérienne chez un mammifère.

12. Procédé pour la préparation de composés macrolides tricycliques répondant aux Formules : 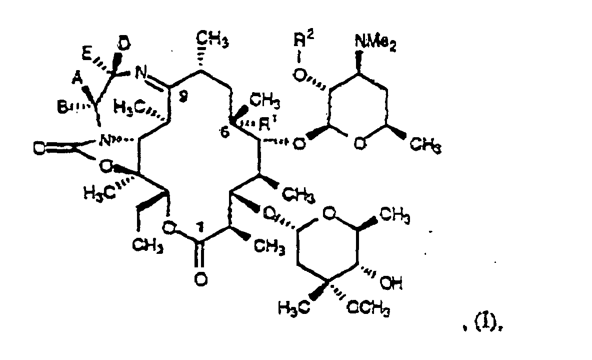 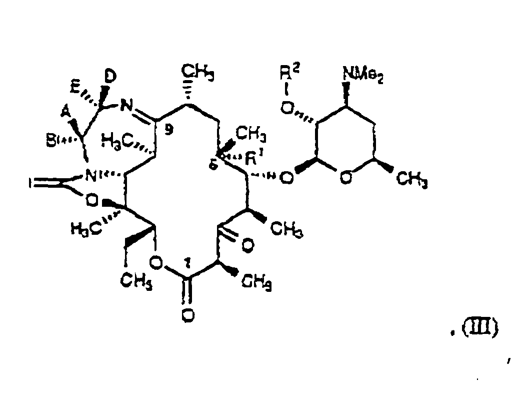 ou 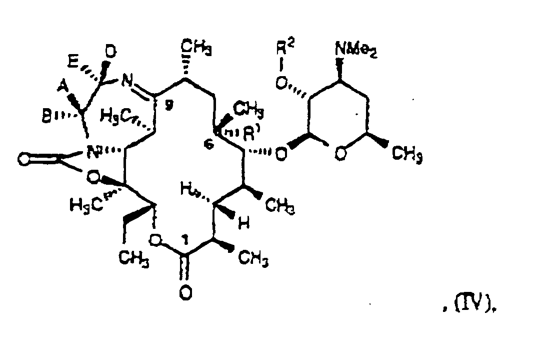 dans lesquelles
A, B, D et E sont indépendamment choisis dans le groupe constitué par :
(a) un atome d'hydrogène ;
(b) un alkyle en C₁ à C₆, non substitué ou substitué par un substituant choisi dans le groupe constitué par :
(i) le phényle ;
(ii) un phényle substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di-(alkyl en C₁ à C₃)-amino ;
(iii) un hétéroaryle choisi parmi le pyridyle, le pyrazinyle, le pyrimidinyle, le pyrrolyle, le pyrazolyle, l'imidazolyle, le thiazolyle, l'oxazolyle, l'isooxazolyle, le thiadiazolyle, l'oxadiazolyle, le thiophényle, le furanyle, le quinolinyle et l'isoquinolinyle ;
(iv) un hétéroaryle tel que défini ci-dessus substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di-(alkyl en C₁ à C₃)-amino ;
(v) un hétérocycloalkyle choisi parmi le pyrrolidinyle, le pyrazolinyle, le pyrazolidinyle, l'imidazolinyle, l'imidazolidinyle, le pipéridinyle, le pipérazinyle, l'oxazolidinyle, l'isoxazolidinyle, le morpholinyle, le thiazolidinyle, l'isothiazolidinyle et le tétrahydrofuryle ;
(vi) l'hydroxy ;
(vii) un alcoxy en C₁ à C₆
(viii)un atome d'halogène constitué par Br, Cl, F ou I ; et
(ix) NR³R⁴, dans lequel R³ et R⁴ sont indépendamment choisis parmi un atome d'hydrogène, et un alkyle en C₁ à C₆, ou R³ et R⁴ sont pris avec l'atome d'azote auquel ils sont reliés pour former un cycle à 3 à 7 chaînons contenant facultativement une fonction hétéro constituée par -O-, -NH-, -N(alkyle en C₁ à C₆)-, -N(phényl-alkyle en C₁ à C₆)-, -N(phényl substitué-alkyle en C₁ à C₆)- dans lequel le phényle substitué est tel que défini ci-dessus, -N(hétéroaryl-alkyle en C₁ à C₆)- dans lequel l'hétéroaryle est tel que défini ci-dessus, N-(hétéroaryl substitué-alkyle en C₁ à C₆) - dans lequel l'hétéroaryle substitué est tel que défini ci-dessus, -S-ou -S(O)ₙ-, dans lequel n vaut 1 ou 2 ;
(c) un cycloalkyle en C₃ à C₇ ;
(d) le phényle ;
(e) un phényle substitué tel que défini ci-dessus ;
(f) un hêtéroaryle tel que défini ci-dessus ;
(g) un hétéroaryle substitué tel que défini ci-dessus ;
(h) un hétérocycloalkyle tel que défini ci-dessus ; et
(i) un groupe choisi parmi l'option (b) ci-dessus dans lequel le substituant est -M-^{R5}, dans lequel M est choisi dans le groupe constitué par :
(aa) -C(O)-NH- ;
(bb) -NH-C(O)- ;
(cc) -NH-
(dd) -N(CH₃)-
(ee) -O- ;
(ff) -S(O)ₙ-, dans lequel n vaut 0, 1 ou 2 ; et
(gg) -C(=NH)-NH- ;
et R⁵ est choisi dans le groupe constitué par :
(aaa) un alkyle en C₁ à C₆, facultativement substitué par un substituant choisi dans le groupe constitué par :
(i) le phényle,
(ii) un phényle substitué tel que défini ci-dessus ;
(iii) un hétéroaryle tel que défini ci-dessus ;
(iv) un hétéroaryle substitué tel que défini ci-dessus ;
(bbb) le phényle ;
(ccc) un phényle substitué tel que défini ci-dessus ;
(ddd) un hétéroaryle tel que défini ci-dessus ;
(eee) un hétéroaryle substitué tel que défini ci-dessus ; et
(fff) un hétérocycloalkyle tel que défini ci-dessus ; ou
toute paire de substituants, constituée par AB, AD, AE, BD, BE ou DE, est prise conjointement avec l'atome ou les atomes auxquels elles sont attachées pour former un cycle à 3 à 7 chaînons contenant facultativement une fonction hétéro constitué par
-O-,
- NH-,
- N (alkyle en C₁ à C₆),
-N(phényl-alkyle en (C₁ à C₆)-,
-N(phényl substitué-alkyle en C₁ à C₆)-
dans lequel le phényle substitué est tel que défini ci-dessus,
-N(hétéroaryl-alkyle en C₁ à C₆)-
dans lequel l'hétéroaryle est tel que défini ci-dessus,
- N(hétéroaryl substitué-alkyle en C₁ à C₆)-
dans lequel l'hétéroaryle substitué est tel que défini ci-dessus,
- S- ou S(O)ₙ-,
dans lequel n vaut 1 ou 2 ;
-C(O)-NH- ;
- C(O)-NR⁵,
dans lequel R⁵ est tel que défini ci-dessus ;
-NH-C(O)- ;
- NR⁵-C(O)-,
dans lequel R⁵ tel que défini ci-dessus ; et
-C(=NH)-NH- ;
R¹ représente -O-alkyle en C₁ à C₃ ; et
R² représente un atome d'hydrogène ;
à condition que lorsque le composé est de Formule (I) ou (III) alors A, B, D et E ne peuvent pas tous représenter un atome d'hydrogène, D et E ne peuvent pas représenter un alkyle en C₁ à C₃ lorsque A et B représentent un atome d'hydrogène, de,même l'un de D et E ne peut pas représenter un atome d'hydrogène et l'autre représenter un alkyle en C₁ à C₃ lorsque A et B représentent un atome d'hydrogène ;
ou un composé de Formule (III) dans lequel R¹=OCH₃, R² représente H, B=E=H, et A et D pris ensemble représentent soit -CH₂-N(Cbz)-CH₂- soit -CH₂-N(benzoyl) -CH₂- ;
ou un composé de Formule (III) dans lequel B=E=H, R¹ représente le méthoxy, R² représente H, et A et D ensemble représentent CH₂-N(R⁷)-CH₂-, dans lequel R⁷ est choisi dans le groupe constitué par :
(i) le phényle ;
(ii) un phényle substitué tel que défini ci-dessus ;
(iii) un hétéroaryle tel que défini ci-dessus ; et
(iv) un hétéroaryle substitué tel que défini ci-dessus ;
ou un composé de Formule (III) dans lequel B=E=H, R¹ représente le méthoxy, R² représente H ; et A et D ensemble représentent CH₂-N(-(CH₂)ᵣ-M-(CH₂)ₛ-R⁷) -CH₂-, dans lequel r vaut 0, 1 ou 2 ; s = 0, 1 ou 2, M est tel que défini ci-dessus ; et R⁷ est choisi dans le groupe constitué par :
(i) un alkyle en C₁ à C₃ ;
(ii) le phényle ;
(iii) un phényle substitué tel que défini ci-dessus ;
(iv) un hétéroaryle tel que défini ci-dessus ; et
(v) un hétéroaryle substitué tel que défini ci-dessus, le procédé consistant à :
(a) traiter un composé répondant à la formule : 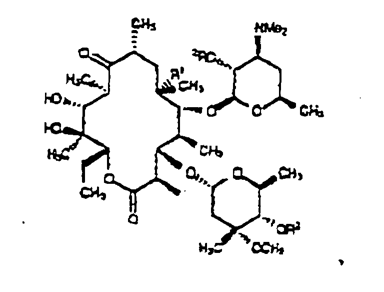 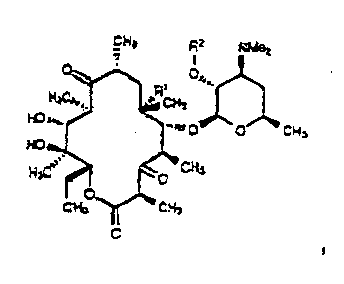 ou 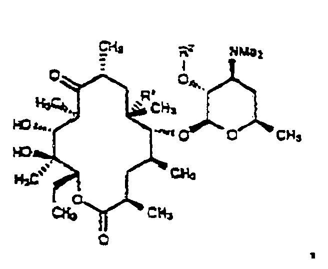 respectivement,
dans lesquelles R¹ est tel que décrit ci-dessus et R² est un groupe protecteur d'hydroxy, avec une base, et suivi par la réaction avec du carbonyldiimidazole, dans un solvant aprotique, à une température de réaction de - 20°C à 70°C, pendant une période de 0,5 heure à 10 jours, pour préparer des premiers composés intermédiaires répondant aux formules : 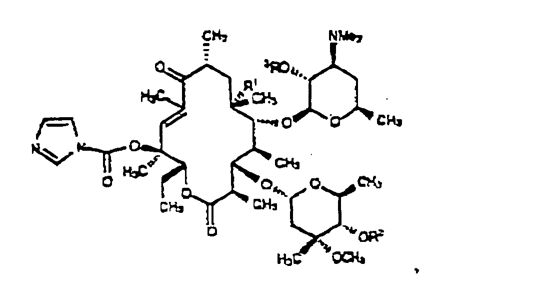 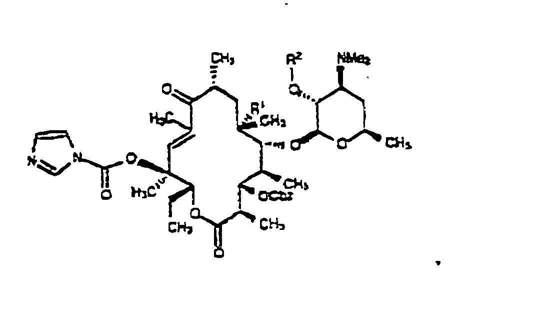 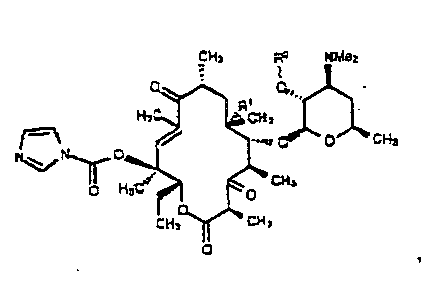
ou 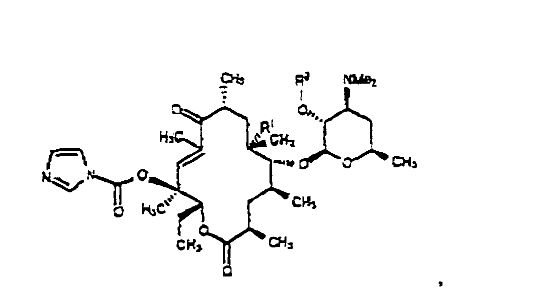 , respectivement ;
dans lesquelles R¹ et R² sont tels que décrit ci-dessus ;
(b) faire réagir lesdits premiers composés intermédiaires avec un composé répondant à la formule : 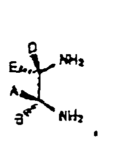 dans A, B, D et E sont tels que décrits ci-dessus, pour donner les deuxièmes composés intermédiaires bicycliques répondant aux formules : 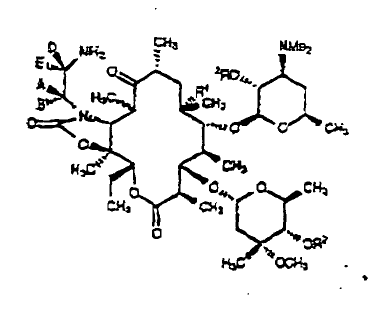 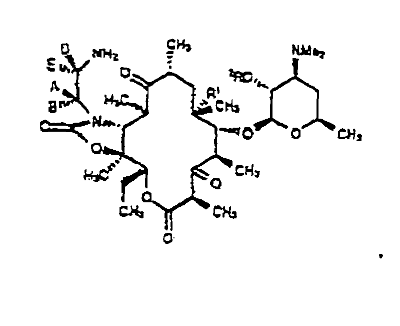 ou 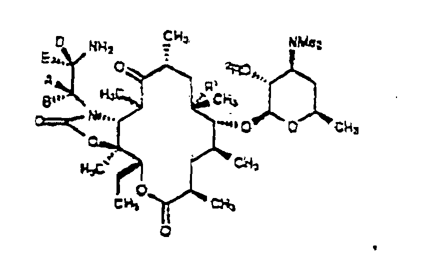 , respectivement ;
dans lesquelles R¹ et R² sont tels décrits ci-dessus ;
(c) déprotéger lesdits deuxièmes composés intermédiaires par traitement avec du méthanol ou de l'éthanol lorsque OR² est un ester ou avec du fluorure dans du THF ou de l'acétonitrile lorsque R² est un groupe trialkylsilyle, pendant 1 à 24 heures, pour donner les troisièmes composés intermédiaires : 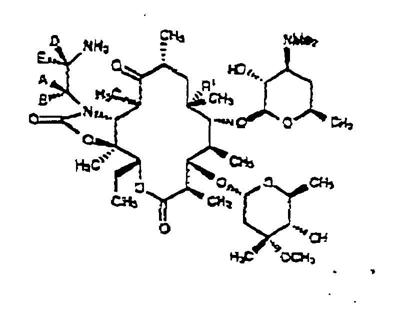 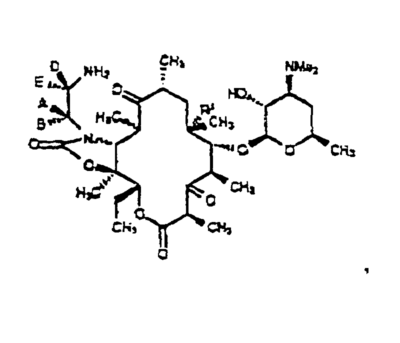 ou 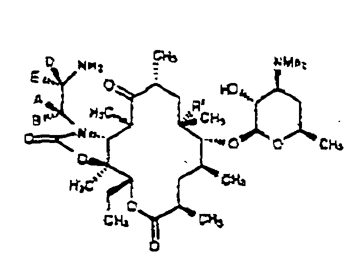 .respectivement ; et
(d) cycliser lesdits troisièmes composés intermédiaires par traitement avec un acide dilué, dans un solvant organique, pendant une période de 4 heures à 10 jours pour donner les composés souhaités (I), (III) ou (IV) ci-dessus.

13. Procédé selon la revendication 12, dans lequel :
dans l'étape (a) le solvant aprotique est choisi dans le groupe constitué par le dichlorométhane, le chloroforme, le DMF, le tétrahydrofurane (THF), la N-méthylpyrrolidinone ou un mélange de ceux-ci, la température de réaction est comprise entre 0°C et la température de réaction, et la période de réaction est comprise entre 1 et 8 heures ou jusqu'à 1 à 5 jours ;
dans l'étape (b) la réaction est mise en oeuvre dans de l'acétonitrile aqueux, du DMF ou du DMF aqueux ;
dans l'étape (c) on déprotège lesdits deuxièmes composés intermédiaires par traitement avec du méthanol ou de l'éthanol lorsque OR² est un ester ou avec du fluorure dans du THF ou de l'acétonitrile lorsque R² est un groupe trialkylsilyle, pendant 1 à 24 heures ; et
dans l'étape (d) on cyclise lesdits troisièmes composés intermédiaires par traitement avec de l'acide acétique ou du HCl dilués dans de l'éthanol du propanol pendant une période de 4 heures à 10 jours pour donner les composés souhaités.

14. Procédé pour la préparation de dérivés macrolides tricycliques répondant aux Formules : 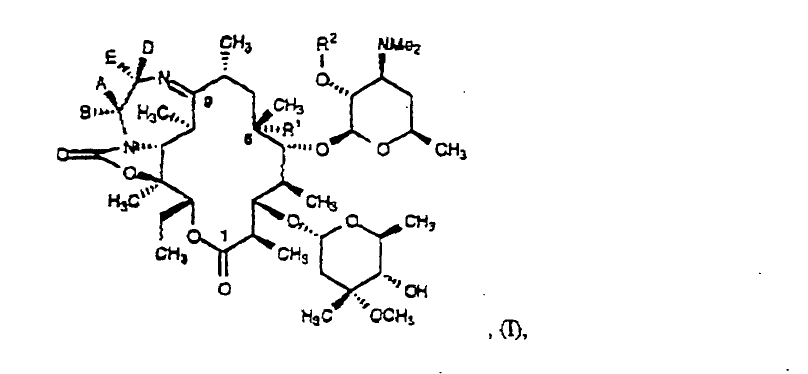 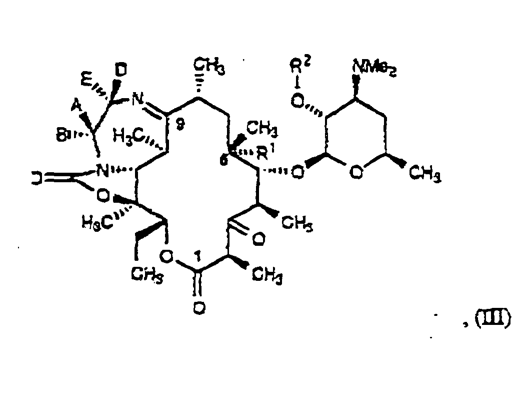 ou 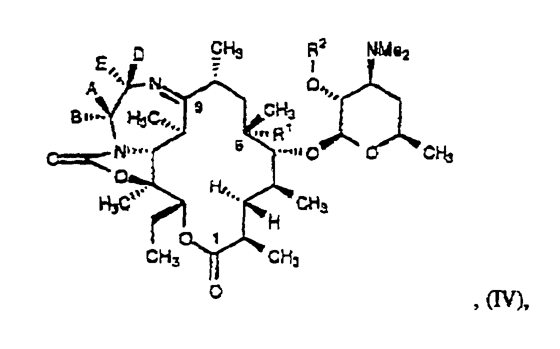 dans lesquelles
A, B, D et E sont indépendamment choisis dans le groupe constitué par :
(a) un atome d'hydrogène ;
(b) un alkyle en C₁ à C₆, non substitué ou substitué par un substituant choisi dans le groupe constitué par :
(i) le phényle ;
(ii) un phényle substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di-(alkyl en C₁ à C₃)-amino ;
(iii) un hétéroaryle choisi parmi le pyridyle, le pyrazinyle, le pyrimidinyle, le pyrrolyle, le pyrazolyle, l'imidazolyle, le thiazolyle, l'oxazolyle, l'isooxazolyle, le thiadiazolyle, l'oxadiazolyle, le thiophényle, le furanyle, le quinolinyle et l'isoquinolinyle ;
(iv) un hétéroaryle tel que défini ci-dessus substitué par un, deux ou trois groupes indépendamment choisis parmi Cl, Br, F, I, OH, un alkyle en C₁ à C₃, un alcoxy en C₁ à C₆, le méthoxyméthoxy, un amino, un (alkyl en C₁ à C₃)-amino et un di-(alkyl en C₁ à C₃)-amino ;
(v) un hétérocycloalkyle choisi parmi le pyrrolidinyle, le pyrazolinyle, le pyrazolidinyle, l'imidazolinyle, l'imidazolidinyle, le pipéridinyle, le pipérazinyle, l'oxazolidinyle, l'isoxazolidinyle, le morpholinyle, le thiazolidinyle, l'isothiazolidinyle et le tétrahydrofuryle ;
(vi) l'hydroxy ;
(vii) un alcoxy en C₁ à C₆
(viii)un atome d'halogène constitué par Br, Cl, F ou I ; et
(ix) NR³R⁴, dans lequel R³ et R⁴ sont indépendamment choisis parmi un atome d'hydrogène et un alkyle en C₁ à C₆, ou R³ et R⁴ sont pris avec l'atome d'azote auquel ils sont reliés pour former un cycle à 3 à 7 chaînons contenant facultativement une fonction hétéro constituée par -O-, -NH-, -N(alkyle en C₁ à C₆)-, -N(phényl-alkyle en C₁ à C₆)-, -N(phényl substitué-alkyle en C₁ à C₆)- dans lequel le phényle substitué est tel que défini ci-dessus, -N(hétéroaryl-alkyle en C₁ à C₆)- dans lequel l'hétéroaryle est tel que défini ci-dessus, N-(hétéroaryl substitué-alkyle en C₁ à C₆)- dans lequel l'hétéroaryle substitué est tel que défini ci-dessus, -S- ou -S(O)ₙ-, dans lequel n vaut 1 ou 2 ;
(c) un cycloalkyle en C₃ à C₇ ;
(d) le phényle ;
(e) un phényle substitué tel que défini ci-dessus ;
(f) un hétéroaryle tel que défini ci-dessus ;
(g) un hétéroaryle substitué tel que défini ci-dessus ;
(h) un hétérocycloalkyle tel que défini ci-dessus ; et
(i) un groupe choisi parmi l'option (b) ci-dessus dans lequel le substituant est -M-^{R5}, dans lequel M est choisi dans le groupe constitué par :
(aa) -C(O)-NH- ;
(bb) -NH-C(O)- ;
(cc) -NH-
(dd) -N(CH₃)-
(ee) -O- ;
(ff) -S(O)ₙ-, dans lequel n vaut 0, 1 ou 2 ; et
(gg) -C(=NH)-NH- ;
et R⁵ est choisi dans le groupe constitué par :
(aaa) un alkyle en C₁ à C₆, facultativement substitué par un substituant choisi dans le groupe constitué par :
(i) le phényle,
(ii) un phényle substitué tel que défini ci-dessus ;
(iii) un hétéroaryle tel que défini ci-dessus ;
(iv) un hétéroaryle substitué tel que défini ci-dessus ;
(bbb) le phényle ;
(ccc) un phényle substitué tel que défini ci-dessus ;
(ddd) un hétéroaryle tel que défini ci-dessus ;
(eee) un hétéroaryle substitué tel que défini ci-dessus ; et
(fff) un hétérocycloalkyle tel que défini ci-dessus ; ou
toute paire de substituants, constituée par AB, AD, AE, BD, BE ou DE, est prise conjointement avec l'atome ou les atomes auxquels elles sont attachées pour former un cycle à 3 à 7 chaînons contenant facultativement une fonction hétéro constitué par
-O-,
- NH-,
-N(alkyle en C₁ à C₆),
-N(phényl-alkyle en C₁ à C₆),
-N(phényl substitué-alkyle en C₁ à C₆)-
dans lequel le phényle substitué est tel que défini ci-dessus,
-N(hétéroaryl-alkyle en C₁ à C₆)
dans lequel l'hétéroaryle est tel que défini ci-dessus,
-N(hétéroaryl substitué-alkyle en C₁ à C₆)-
dans lequel l'hétéroaryle substitué est tel que défini ci-dessus,
-S- ou S(O)ₙ-,
dans lequel n vaut 1 ou 2 ;
-C(O)-NH- ;
-C(O)-NR⁵-,
dans lequel R⁵ est tel que défini ci-dessus ;
-NH-C(O)- ;
-NR⁵-C(O)-,
dans lequel R⁵ tel que défini ci-dessus ; et
-C(=NH)-NH- ;
R¹ représente -O-alkyle en C₁ à C₃ ; et
R² représente un atome d'hydrogène ;
à condition que lorsque le composé est de Formule (I) ou (III) alors A, B, D et E ne peuvent pas tous représenter un atome d'hydrogène, D et E ne peuvent pas représenter un alkyle en C₁ à C₃ lorsque A et B représentent un atome d'hydrogène, de même l'un de D et E ne peut pas représenter un atome d'hydrogène et l'autre représenter un alkyle en C₁ à C₃ lorsque A et B représentent un atome d'hydrogène ;
ou un composé de Formule (III) dans lequel R¹=OCH₃, R² représente H, B=E=H et A et D pris ensemble représentent CH₂-N(Cbz)-CH₂- ou -CH₂-N(benzoyl)-CH₂-,
ou un composé de Formule (III) dans lequel B=E=H, R¹ représente un méthoxy, R² représente H ; et A et D pris ensemble représentent CH₂-N(R⁷)-CH₂-, dans lequel R⁷ est choisi parmi un groupe constitué par :
(i) le phényle ;
(ii) un phényle substitué tel que défini ci-dessus ;
(iii) un hétéroaryle tel que défini ci-dessus ; et
(iv) un hétéroaryle substitué tel que défini ci-dessus ;
ou un composé de Formule (III) dans lequel B=E=H, R¹ représente le méthoxy, R² représente H ; et A et D ensemble représentent CH₂-N(-(CH₂)ᵣ-M-(CH₂)ₛ-R⁷)-CH₂-, dans lequel r vaut 0, 1 ou 2 ; s = 0, 1 ou 2, M est tel que défini ci-dessus ; et R⁷ est choisi dans le groupe constitué par :
(i) un alkyle en C₁ à C₃ ;
(ii) le phényle ;
(iii) un phényle substitué tel que défini ci-dessus ;
(iv) un hétéroaryle tel que défini ci-dessus ; et
(v) un hétéroaryle substitué tel que défini ci-dessus, le procédé consistant à :
(a) traiter un composé répondant à la formule : 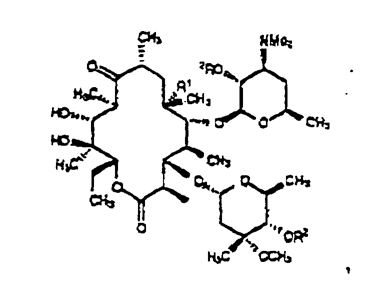 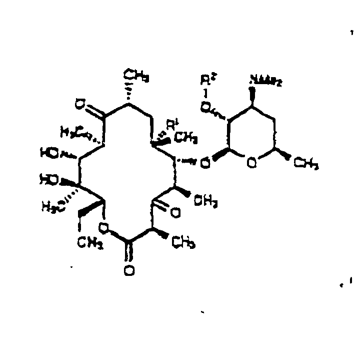 ou 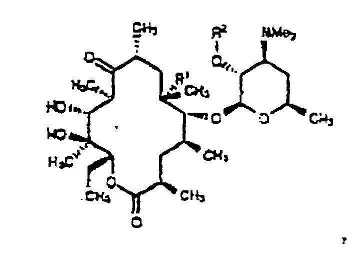 respectivement, dans lesquelles R¹ est tel que décrit ci-dessus, et R² est un groupe protecteur d'hydroxy, avec une base, et suivi par la réaction avec du carbonyldiimidazole, dans un solvant aprotique, à une température de réaction comprise entre - 20°C et 70°C, pendant une période comprise entre 0,5 et 24 heures, pour préparer des premiers composés intermédiaires répondant aux formules : 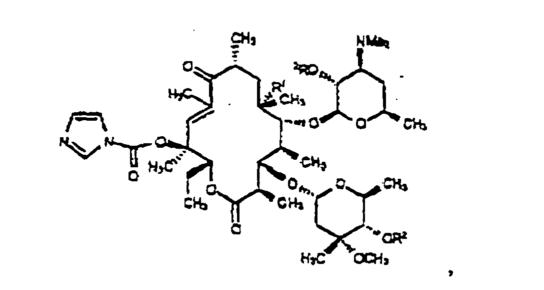 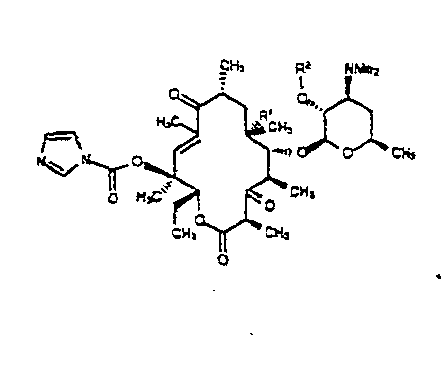 ou 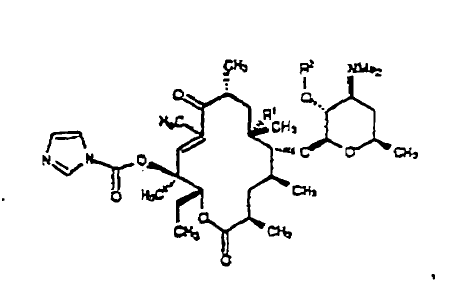 respectivement ;
dans lesquelles R¹ et R² sont tels que décrits ci-dessus ;
(b) faire réagir lesdits premiers composés intermédiaires avec un composé répondant à la formule : 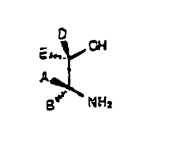 dans laquelle A, B, D et E sont tels que décrits ci-dessus pour donner les deuxièmes composés intermédiaires bicycliques : 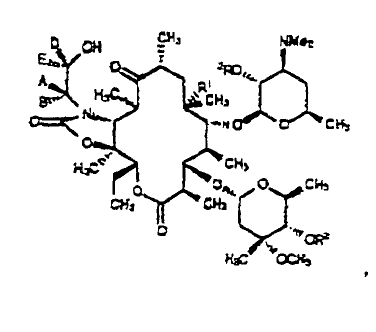 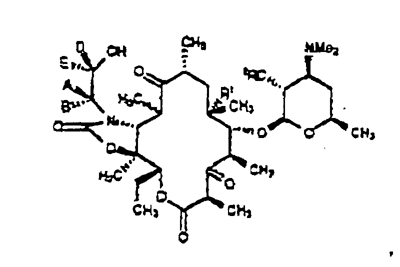 ou 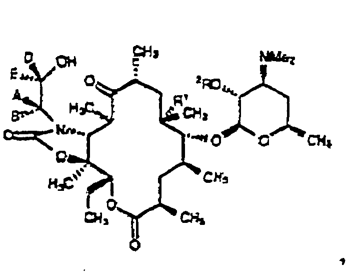 respectivement ;
(c) faire réagir le groupe hydroxy desdits deuxièmes composés intermédiaires bicycliques par traitement avec de la triphénylphosphine et de l'azoture de diphénylphosphoryle dans du tétrahydrofurane, dans des conditions de réaction de Mitsunobu, pour préparer les troisièmes composés intermédiaires : 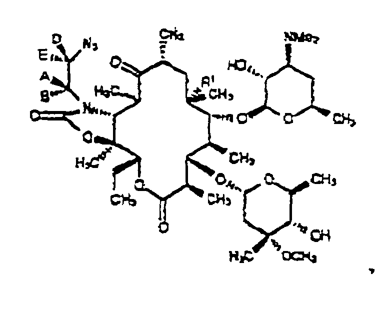 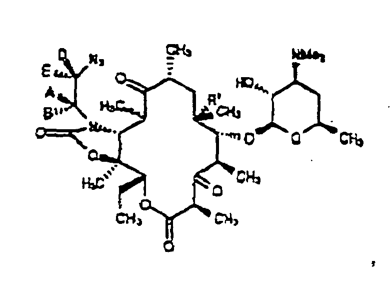 ou 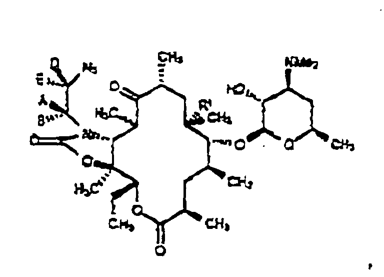 respectivement ;
(d) réduire les troisièmes composés intermédiaires comportant un groupe azido, avec des réactifs réducteurs pour préparer les quatrièmes composés intermédiaires répondant aux formules : 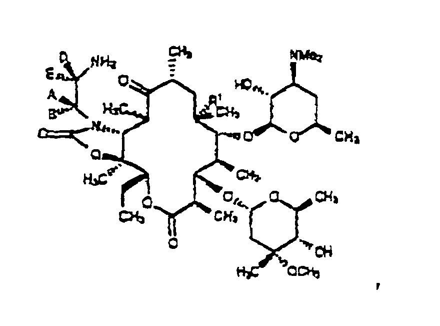 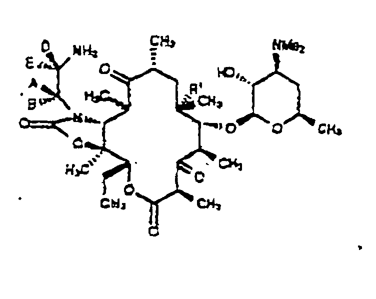 ou 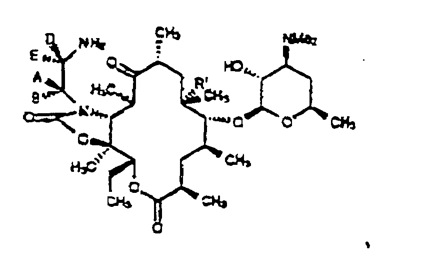 , respectivement ; et
(e) cycliser lesdits quatrièmes composés intermédiaires par traitement avec un acide dilué, dans un solvant organique, pendant une période comprise entre 4 heures et 10 jours pour donner les composés souhaités de Formule (I), (III) ou (IV).

15. Procédé selon la revendication 14, dans lequel :
dans l'étape (a) la base est de l'hydrure de sodium, de l'hydrure de lithium ou du carbonate de potassium, le solvant aprotique est le dichlorométhane, le chloroforme, le DMF, le tétrahydrofurane (THF), la N-méthylpyrrolidinone ou un mélange de ceux-ci, la température de réaction est comprise entre 0°C et la température de réaction et la période de réaction est comprise entre 1 et 8 heures ;
dans l'étape (b) le solvant est de l'acétonitrile aqueux, du DMF ou du DMF aqueux ;
dans l'étape (c) les réactifs sont la triphénylphosphine et l'azoture de diphénylphosphoryle-DEAD dans du tétrahydrofurane, dans des conditions de réaction de Mitsunobu ;
dans l'étape (d) le réactif réducteur est de la triphénylphosphine-eau, de l'hydrogène avec un catalyseur, du borohydrure de sodium ou un hydrure de dialkylaluminium ;
dans l'étape (e) on cyclise lesdits troisièmes composés intermédiaires par traitement avec de l'acide acétique ou du HCl dilué dans de l'éthanol ou du propanol pendant une période comprise entre 4 heures et 10 jours pour donner les composés souhaités.

16. Procédé selon la revendication 14, dans lequel :
l'étape (c) est remplacée par une séquence à deux étapes qui comprend (1) la mise à réagir du groupe hydroxy des deuxièmes composés intermédiaires bicycliques de celle-ci avec un chlorure d'alkyl- ou d'aryl-sulfonyle, un anhydride d'alkyl- ou aryl-sulfonique ou l'anhydride trifluorométhanesulfonique dans un solvant aprotique à -78°C jusqu'à la température de réaction pour donner le sulfonate correspondant, et (2) la mise à réaction dudit sulfonate avec de l'azoture de lithium ou de l'azoture de sodium dans un solvant aprotique à 0°C jusqu'à 100°C pour donner le troisième composé intermédiaire.
